# EUROPEAN PATENT SPECIFICATION

(11) **EP 2 970 242 B1**
(45) Date of publication and mention of the grant of the patent: **06.09.2017**
(21) Application number: 14716934.6
(22) Date of filing: 13.03.2014
(51) Int. Cl.: C07D 413/14, A61K 31/506, C07D 413/04, C07D 417/14, A61P 35/00

(54) **3-PYRIMIDIN-4-YL-OXAZOLIDIN-2-ONES AS INHIBITORS OF MUTANT IDH**
3-PYRIMIDIN-4-YL-OXAZOLIDIN-2-ONE ALS INHIBITOREN VON IDH-MUTATIONEN
3-PYRIMIDIN-4-YL-OXAZOLIDIN-2-ONES COMME INHIBITEURS D'IDH MUTANTE

(30) Priority: 14.03.2013 US 201361782211 P
(43) Date of publication of application: 20.01.2016
(73) Proprietor: Novartis AG, 4056 Basel (CH)
(72) Inventor: CHO, Young Shin, Cambridge, Massachusetts 02139 (US); LEVELL, Julian Roy, Cambridge, Massachusetts 02139 (US); LIU, Gang, Cambridge, Massachusetts 02139 (US); SHULTZ, Michael David, Cambridge, Massachusetts 02139 (US); VAN DER PLAS, Simon Cornelis, Cambridge, Massachusetts 02139 (US)
(74) Representative: Dyer, James
(86) International application number: PCT/IB2014/059758
(87) International publication number: WO 2014/141153

(56) References cited:
- EP-A1- 2 394 999
- WO-A1-01/60816
- WO-A1-2008/080937

## Description

### FIELD OF THE INVENTION

The present invention is directed to novel 3-pyrimidinyl-4-yl-oxazolidin-2-one compounds, compositions containing these compounds, the use of such compounds in the inhibition of mutant IDH proteins having a neomorphic activity and in the treatment of diseases or disorders associated with such mutant IDH proteins including, but not limited to, cell-proliferation disorders, such as cancer.

### BACKGROUND OF THE INVENTION

Isocitrate dehydrogenase (IDH) is a key family of enzymes found in cellular metabolism. They are NADP⁺ / NAD⁺ and metal dependent oxidoreductases of the enzyme class EC 1.1.1.42. The wild type proteins catalyze the oxidative decarboxylation of isocitrate to alpha-ketoglutarate generating carbon dioxide and NADPH / NADH in the process. They are also known to convert oxalosuccinate into alpha-ketoglutarate. Mutations in IDH1 (cytosolic) and IDH2 (mitochondrial) have been identified in multiple cancer types including, but not limited to, glioma, glioblastoma multiforme, paraganglioma, supratentorial primordial neuroectodermal tumors, acute myeloid leukemia (AML), prostate cancer, thyroid cancer, colon cancer, chondrosarcoma, cholangiocarcinoma, peripheral T-cell lymphoma, and melanoma. (See L. Deng et al., Trends Mol. Med., 2010, 16, 387; T. Shibata et al., Am. J. Pathol., 2011, 178(3), 1395; Gaal et al., J. Clin. Endocrinol. Metab. 2010; Hayden et al., Cell Cycle, 2009; Balss et al., Acta Neuropathol., 2008). The mutations have been found at or near key residues in the active site: G97D, R100, R132, H133Q, and A134D for IDH1, and R140 and R172 for IDH2. (See L. Deng et al., Nature, 2009, 462, 739; L. Sellner et al., Eur. J. Haematol., 2011, 85, 457).

These mutant forms of IDH are shown to have a neomorphic activity (also known as a gain of function activity), reducing alpha-ketoglutarate to 2-hydroxyglutarate (2-HG). (See P.S. Ward et al., Cancer Cell, 2010, 17, 225) In general, production of 2-HG is enantiospecific, resulting in generation of the D-enantiomer (also known as R enantiomer or R-2-HG). Normal cells have low native levels of 2-HG, whereas cells harboring these mutations in IDH1 or IDH2 show significantly elevated levels of 2-HG. High levels of 2-HG have been detected in tumors harboring the mutations. For example, high levels of 2-HG have been detected in the plasma of patients with mutant IDH containing AML. (See S. Gross et al., J. Exp. Med., 2010, 207(2), 339). High levels of 2-HG are highly associated with tumorigenesis.

Mutant IDH2 is also associated with the rare neurometabolic disorder D-2-hydroxyglutaric aciduria type II (D-2-HGA type II). Germline mutations were found at R140 in IDH2 in 15 pateints having D-2-HGA type II. Patients having this disorder also have consistently increased levels of D-2-HG in their urine, plasma and cerebrospinal fluid. (See Kranendijk, M. et al., Science, 2010, 330, 336). Finally, patients with Ollier Disease and Mafucci Syndrome (two rare disorders that predispose to cartilaginous tumors) have been shown to be somatically mosaic for IDH1 and 2 mutations and exhibit high levels of D-2-HG. (See Amary et al., Nature Genetics , 2011 and Pansuriya et al., Nature Genetics, 2011). WO 2008/080937 A1 discloses pyrimidine derivatives for the treatment of cancer. Thus, there is a need for small molecule inhibitors of mutant IDH proteins having a neomorphic activity for the treatment of diseases and disorders associated with these proteins.

### SUMMARY OF THE INVENTION

In one aspect, this invention provides for a compound of formula (I): 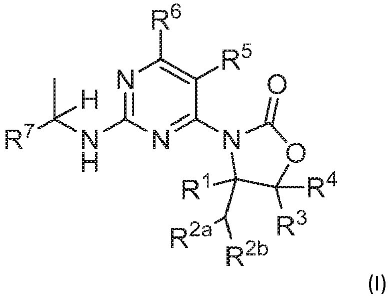 or a pharmaceutically acceptable salt thereof wherein R¹, R^{2a}, R^{2b} and R³-R⁷ are defined below.

In a second aspect, this invention provides for a pharmaceutical composition comprising a compound of formula (I), or a pharmaceutically acceptable salt thereof, and a pharmaceutically acceptable carrier or excipient.

In a third aspect, this invention provides for the use of a compound of formula (I), or a pharmaceutically acceptable salt thereof, as an inhibitor of a mutant IDH protein having a neomorphic activity such as reducing alpha-ketoglutarate to 2-hydroxyglutarate (2-HG neomorphic activity). Suitably, this invention provides for the use of a compound of formula (I), or a pharmaceutically acceptable salt thereof, as an inhibitor of mutant IDH1 having a neomorphic activity, such as 2-HG neomorphic activity, and/or mutant IDH2 having a neomorphic activity, such as 2-HG neomorphic activity. This invention further provides for the use of a compound of formula (I), or a pharmaceutically acceptable salt thereof, as an inhibitor of IDH1 having a mutation at residue 97, 100 or 132, for example G97D, R100Q, R132H, R132C, R132S, R132G, R132L, and R132V; and/or an inhibitor of IDH2 having a mutation at residue 140 or 172, for example R172K, R172M, R172S, R172G, and R172W.

In a fourth aspect, this invention provides for compounds for use in a method of treating a disease or disorder associated with a mutant IDH protein having a neomorphic activity comprising administration of an effective amount of a compound according to formula (I), or a pharmaceutically acceptable salt thereof, to a subject in need thereof. In one embodiment, the disease or disorder is a cell proliferation disorder, such as cancer. In another embodiment, the cancer is brain cancer, such as glioma, glioblastoma multiforme, paraganglioma, and supratentorial primordial neuroectodermal tumors (pNET); leukemia, such as acute myeloid leukemia (AML), myelodysplastic syndrome, and chronic myelogenous leukemia (CML); skin cancer, including melanoma; prostate cancer; thyroid cancer; colon cancer; lung cancer; sarcoma, including central chondrosarcoma, central and periosteal chondroma; and fibrosarcoma. In another embodiment the disease or disorder is D-2-hydroxyglutaric aciduria.

In a fifth aspect the invention provides for a compound of formula (I), or a pharmaceutically acceptable salt thereof, in combination with another therapeutic agent.

These and other aspects of the present invention are described further in the following detailed description of the invention.

### DETAILED DESCRIPTION OF THE INVENTION

The present invention is directed to a compound of formula (I) 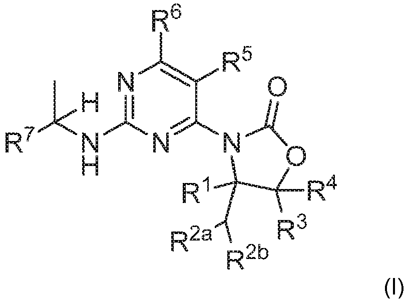 wherein:
R¹ is hydrogen, methyl or ethyl;
R^{2a} and R^{2b} are joined together forming a cyclopropyl ring;
R³ and R⁴ are each independently hydrogen, methyl or ethyl or R³ and R⁴ are joined together forming cyclopropyl, cyclobutyl or oxetanyl;
R⁵ and R⁶ are each independently hydrogen, deuterium, halo, -C(O)OCH₃, C₁₋₃ alkyl or C₁₋₃ haloalkyl;
R⁷ is

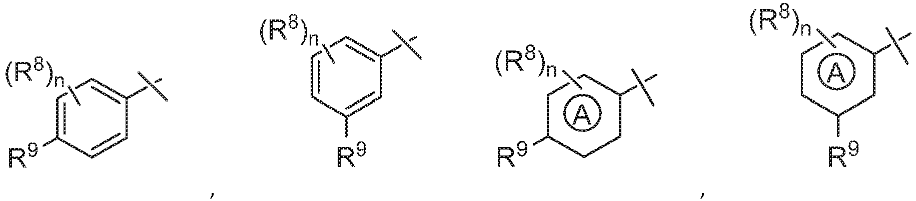

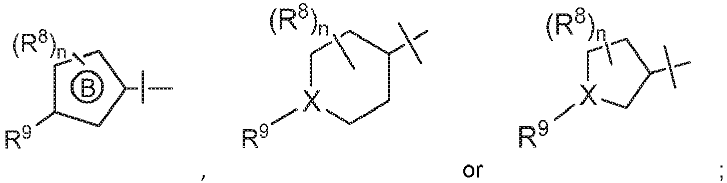

ring A is a 6 membered heteroaryl ring having one to three nitrogen atoms;
ring B is a 5 membered heteroaryl ring having one to four heteroatoms each independently selected from the group consisting of N, O and S;
X is N or CH;
each R⁸ is independently hydrogen, halo, C₁₋₃ alkyl, C₁₋₃ haloalkyl, C₁₋₃ alkoxy or C₁₋₃ haloalkoxy;
n is 1 or 2;
R⁹ is hydrogen, halo, C₁₋₃ haloalkyl, optionally substituted C₁₋₆ alkyl, optionally substituted C₃₋₆ cycloalkyl, optionally substituted 5 or 6 membered heterocyclic, optionally substituted aryl, optionally substituted heteroaryl, -OR^{9a}, -SO₂R^{9a}, -C(O)NHR^{9a}, CH₂R^{9b} or CHCH₃R^{9b} provided that when X is N, R⁹ is hydrogen, C₁₋₃ haloalkyl, optionally substituted C₁₋₆ alkyl, optionally substituted C₃₋₆ cycloalkyl, optionally substituted aryl, optionally substituted heteroaryl, -SO₂R^{9a} or -C(O)NHR^{9a}, wherein:
   said C₁₋₆ alkyl is optionally substituted with one to three substituents each independently selected from the group consisting of: OH and phenoxy, and said C₃₋₆ cycloalkyl, 5 or 6 membered heterocyclic, aryl and heteroaryl are each optionally substituted with one to three substituents each independently selected from the group consisting of: halo, hydroxyl, cyano, -NRR, C₁₋₆ alkyl, C₁₋₆ haloalkyl, C₁₋₃ alkoxy and C₁₋₃ haloalkoxy;
R^{9a} is optionally substituted C₁₋₆ alkyl, C₁₋₆ haloalkyl, optionally substituted C₃₋₆ cycloalkyl, optionally substituted phenyl or optionally substituted heterocyclic, wherein:
   said C₁₋₆ alkyl is optionally substituted with one C₃₋₆ cycloalkyl,
   said C₃₋₆ cycloalkyl and heterocyclic are optionally substituted with one to three substituents each independently selected from the group consisting of: hydroxyl, CH₂OH, -NRR, cyano, C₁₋₃ alkyl, C₁₋₃ haloalkyl and C₁₋₃ alkoxy, and
   said phenyl is optionally substituted with one to three substituents each independently selected from the group consisting of: halo, hydroxyl, cyano, -NRR, C₁₋₆ alkyl, C₁₋₆ haloalkyl, C₁₋₃ alkoxy and C₁₋₃ haloalkoxy;
R^{9b} is optionally substituted C₃₋₆ cycloalkyl, optionally substituted phenyl, or optionally substituted heterocyclic, wherein
   said C₃₋₆ cycloalkyl and heterocyclic are optionally substituted with one to four substituents each independently selected from the group consisting of: hydroxyl, CH₂OH, -NRR, -NRC(O)CH₃, 4 to 6 membered heterocyclic, cyano, halo, C₁₋₃ alkyl, C₁₋₃ haloalkyl and C₁₋₃ alkoxy, and
   said phenyl is optionally substituted with one to three substituents each independently selected from the group consisting of: halo, hydroxyl, cyano, C₁₋₆ alkyl, C₁₋₆ haloalkyl, C₁₋₃ alkoxy and C₁₋₃ haloalkoxy; and
each R is independently selected from the group consisting of H, C₁₋₃ alkyl and C₃₋₆ cycloalkyl.

"Alkyl" refers to a monovalent saturated hydrocarbon chain having the specified number of carbon atoms. For example, C₁₋₆ alkyl refers to an alkyl group having from 1 to 6 carbon atoms. Alkyl groups may be optionally substituted with one or more substituents as defined in formula (I). Alkyl groups may be straight or branched. Representative branched alkyl groups have one, two, or three branches. Examples of alkyl groups include, but are not limited to, methyl, ethyl, propyl (n-propyl and isopropyl), butyl (n-butyl, isobutyl, sec-butyl, and t-butyl), pentyl (n-pentyl, isopentyl, and neopentyl), and hexyl.

"Alkoxy" refers to any alkyl moiety attached through an oxygen bridge (i.e. a -O-C₁₋₃ alkyl group wherein C₁₋₃ alkyl is as defined herein). Examples of such groups include, but are not limited to, methoxy, ethoxy, and propoxy.

"Aryl" refers to a hydrocarbon ring system having an aromatic ring. Aryl groups are monocyclic ring systems or bicyclic ring systems. Monocyclic aryl ring refers to phenyl. Bicyclic aryl rings refer to naphthyl and to rings wherein phenyl is fused to a C₅₋₇ cycloalkyl or C₅₋₇ cycloalkenyl ring as defined herein. Aryl groups may be optionally substituted with one or more substituents as defined in formula (I).

"Cycloalkyl" refers to a saturated hydrocarbon ring system having the specified number of carbon atoms. Cycloalkyl groups are monocyclic or bicyclic ring systems. For example, C₃₋₆ cycloalkyl refers to a cycloalkyl group having from 3 to 6 carbon atoms. Cycloalkyl groups may be optionally substituted with one or more substituents as defined in formula (I). Examples of cycloalkyl groups include, but are not limited to, cyclopropyl, cyclobutyl, cyclopentyl and cyclohexyl.

"Cycloalkenyl" refers to an unsaturated hydrocarbon ring system having the specified number of carbon atoms and having a carbon-carbon double bond within the ring. For example, C₅₋₇ cycloalkenyl refers to a cycloalkenyl group having from 5 to 7 carbon atoms. In certain embodiments, cycloalkenyl groups have one carbon-carbon double bond within the ring. In other embodiments, cycloalkeneyl groups have more than one carbon-carbon double bond within the ring. Cycloalkenyl rings are not aromatic. Cycloalkenyl groups may be optionally substituted with one or more substituents as defined in formula (I).

"Halo" refers to the halogen radicals fluoro, chloro, bromo, and iodo.

"Haloalkyl" refers to an alkyl group wherein at least one hydrogen atom attached to a carbon atom within the alkyl group is replaced with halo. The number of halo substituents includes, but is not limited to, 1, 2, 3, 4, 5, or 6 substituents. Haloalkyl includes, but is not limited to, monofluoromethyl, difluoroethyl, and trifluoromethyl.

"Haloalkoxy" refers to a haloalkyl moiety attached through an oxygen bridge (i.e. a -O-C₁₋₃ haloalkyl group wherein C₁₋₃ haloalkyl is as defined herein). An example of a haloalkoxy group is trifluoromethoxy.

"Heteroaryl" refers to an aromatic ring system containing from 1 to 5 heteroatoms. Heteroaryl groups containing more than one heteroatom may contain different heteroatoms. Heteroaryl groups may be optionally substituted with one or more substituents as defined in formula (I). Heteroaryl groups are monocyclic ring systems or are fused bicyclic ring systems. Monocyclic heteroaryl rings have from 5 to 6 ring atoms. Bicyclic heteroaryl rings have from 8 to 10 member atoms. Bicyclic heteroaryl rings include those ring systems wherein a heteroaryl ring is fused to a phenyl ring. Heteroaryl includes, but is not limited to, pyrrolyl, pyrazolyl, imidazolyl, oxazolyl, isoxazolyl, oxadiazolyl (including 1,3,4-oxadiazolyl and 1,2,4-oxadiazolyl), thiazolyl, isothiazolyl, thiadiazolyl, furanyl, furanzanyl, thienyl, triazolyl, pyridinyl (including 2-, 3-, and 4-pyridinyl), pyrimidinyl, pyridazinyl, pyrazinyl, trazinyl, tetrazinyl, tetrzolyl, indonyl, isoindolyl, indolizinyl, indazolyl, purinyl, quinolinyl, isoquinolinyl, quinoxalinyl, quinazolinyl, benzimidazolyl, benzopyranyl, benzopyranyl, benzoxazolyl, benzoisoxazolyl, benzofuranyl, benzothiazolyl, benzothienyl, naphthyridinyl, 1H-pyrrolo[2,3-b]pyridinyl, tetrazolo[1,5-a]pyridinyl, imidazo[2,1-b][1,3,4]thiadiazolyl and the like.

"Heteroatom" refers to a nitrogen, oxygen, or sulfur atom.

"Heterocyclic" refers to a 3 to 11 membered saturated or unsaturated monocyclic or bicyclic ring containing from 1 to 4 heteroatoms. Heterocyclic ring systems are not aromatic. Heterocyclic groups containing more than one heteroatom may contain different heteroatoms. Heterocyclic includes ring systems wherein a sulfur atom is oxidized to form SO or SO₂. Heterocyclic groups may be optionally substituted with one or more substituents as defined in formula (I). Heterocyclic groups are monocyclic, spiro, or fused or bridged bicyclic ring systems. Monocyclic heterocyclic rings have 3 to 7 ring atoms. Examples of monocyclic heterocyclic groups include oxtanyl, tetrahydrofuranyl, dihydrofuranyl, 1,4-dioxanyl, morpholinyl, 1,4-dithianyl, piperazinyl, piperidinyl, 1,3-dioxolanyl, imidazolidinyl, imidazolinyl, pyrrolinyl, pyrrolidinyl, tetrahydropyranyl, dihydropyranyl, oxathiolanyl, dithiolanyl, 1,3-dioxanyl, 1,3-dithianyl, oxathianyl, thiomorpholinyl, tetrahydro-thiopyran1,1-dioxide, 1,4-diazepanyl, and the like. Fused heterocyclic ring systems have from 8 to 11 ring atoms and include groups wherein a heterocyclic ring is fused to a phenyl ring, a heteroaryl ring or another heterocyclic ring. Examples of fused heterocyclic rings include 2,3-dihydrobenzo[b][1,4]dioxinyl, octahydro-pyrrolo[1,2-a]pyrazinyl, octahydro-pyrido[1,2-a]pyrazinyl, octahydro-pyrrolo[3,4-c]pyrrolyl, 5,6,7,8-tetrahydro-[1,2,4]triazolo[4,3-a]pyrazinyl, 5,6,7,8-tetrahydro-imidazo[1,2-a]pyrazinyl and the like. Examples of bridged heterocyclic groups include 3,8-diaza-bicyclo[3.2.1]octanyl, 3,8-diaza-bicyclo[4.2.0]octanyl and the like. Examples of spiro heterocyclic groups include 4,7-diaza-spiro[2.5]octanyl and the like.

"4-6 membered heterocyclic" refers to a heterocyclic group as defined above, having from 4 to 6 ring atoms and containing from 1 to 4 heteroatoms.

"5-6 membered heterocylic" refers to a heterocyclic group as defined above, having 5 or 6 ring atoms and containing from 1 to 4 heteroatoms.

"Optionally substituted" indicates that a group, such as an alkyl, cycloalkyl, heteroaryl, heterocyclic, phenyl, and benzyl may be unsubstitued or the group may be substituted with one or more substituents as defined in formula (I).

"Oxo" refers to a C=O group.

"Pharmaceutically acceptable" means a compound which is suitable for pharmaceutical use. Salts and solvates (e.g. hydrates and hydrates of salts) of compounds of the invention which are suitable for use in medicine are those where in the counterion or associated solvent is pharmaceutically acceptable. However, salts and solvates having non-pharmaceutically acceptable counterions or associated solvents are within the scope of the present invention, for example, for use as intermediates in the preparation of other compounds of the invention and their pharmaceutically acceptable salts and solvates.

"Substituted" in reference to a group such as alkyl, phenyl, benzyl, heteroaryl, and heterocyclic, indicates that one or more hydrogen atoms attached to an atom within the group is replaced with a substituent selected from the group of defined substituents. It should be understood that the term "substituted" includes the implicit provision that such substitution be in accordance with permitted valence of the substituted atom and the substituent, and that the substitution results in a stable compound (i.e. one that does not spontaneously undergo transformation, for example, by hydrolysis, rearrangement, cyclization, or elimination and that is sufficiently robust to survive isolation from a reaction mixture). When it is stated that a group may contain one or more substituents, one or more (as appropriate) atoms within the group may be substituted. In addition, a single atom within the group may be substituted with more than one substituent as long as such substitution is accordance with the permitted valence of the atom. Suitable substituents are defined for each substituted or optionally substituted group.

The skilled artisan will appreciate that salts, including pharmaceutically acceptable salts, of the compounds according to formula (I) may be prepared. These salts may be prepared *in situ* during the final isolation and purification of the compound, or by separately reacting the purified compound in its free acid or free base form with a suitable base or acid, respectively.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts of the present invention can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

Solvates, including pharmaceutically acceptable solvates, of the compounds of formula (I) may also be prepared. "Solvate" refers to a complex of variable stoichiometry formed by a solute and solvent. Such solvents for the purpose of the invention may not interfere with the biological activity of the solute. Examples of suitable solvents include, but are not limited to, water, MeOH, EtOH, and AcOH. Solvates wherein water is the solvent molecule are typically referred to as hydrates. Hydrates include compositions containing stoichiometric amounts of water, as well as compositions containing variable amounts of water.

The compounds of formula (I), including salts and solvates thereof, may exist in crystalline forms, non-crystalline forms, or mixtures thereof. The compound or salt or solvate thereof may also exhibit polymorphism, i.e. the capacity of occurring in different crystalline forms. These different crystalline forms are typically known as "polymorphs". Polymorphs have the same chemical composition but differ in packing, geometrical arrangement, and other descriptive properties of crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, all of which may be used for identification. One of ordinary skill in the art will appreciate that different polymorphs may be produced, for example, by changing or adjusting the conditions used in crystallizing/recrystallizing a compound of formula (I).

Herein are also described various isomers of the compounds of formula (I), whose structural difference may be in constitution (geometric isomers) or in the ability to rotate the plane of polarized light (stereosiomers). With regard to stereoisomers, the compounds of formula (I) may have one or more asymmetric carbon atom and may occur as racemates, racemic mixtures and as individual enantiomers or diastereomers. All such stereo and geometric isomeric forms are included within the present invention, including mixtures thereof. If the compound contains a double bond, the substituent may be in the E or Z configuration. If the compound contains a disubstituted cycloalkyl, the cycloalkyl substituent may have a cis- or trans-configuration. All tautomeric forms are also intended to be included.

Any asymmetric atom (e.g., carbon or the like) of a compound of formula (I) can be present in racemic or enantiomerically enriched, for example the (*R*)-, (*S*)- or (*R,S*)-configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)- configuration. Substituents at atoms with unsaturated double bonds may, if possible, be present in *cis-* (*Z*)- or *trans-* (*E*)- form.

Accordingly, as used herein a compound of formula (I) can be in the form of one of the possible isomers, rotamers, atropisomers, tautomers or mixtures thereof, for example, as substantially pure geometric (*cis* or *trans*) isomers, diastereomers, optical isomers (antipodes), racemates or mixtures thereof.

Any resulting mixtures of isomers can be separated on the basis of the physicochemical differences of the constituents, into the pure or substantially pure geometric or optical isomers, diastereomers, racemates, for example, by chromatography and/or fractional crystallization.

Any resulting racemates of final products or intermediates can be resolved into the optical antipodes by known methods, *e.g*., by separation of the diastereomeric salts thereof, obtained with an optically active acid or base, and liberating the optically active acidic or basic compound. In particular, a basic moiety may thus be employed to resolve the compounds of the present invention into their optical antipodes, *e.g*., by fractional crystallization of a salt formed with an optically active acid, *e.g*., tartaric acid, dibenzoyl tartaric acid, diacetyl tartaric acid, di-*O,O'-p*-toluoyl tartaric acid, mandelic acid, malic acid or camphor-10-sulfonic acid. Racemic products can also be resolved by chiral chromatography, e.g., high pressure liquid chromatography (HPLC) using a chiral adsorbent. Herein described are unlabeled forms as well as isotopically labeled forms of compounds of formula (I). Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, and chlorine, such as ²H, ³H, ¹¹C, ¹³C, ¹⁴C, ¹⁵N, ¹⁸F ³¹P, ³²P, ³⁵S, ³⁶Cl, ¹²⁵I respectively. The description includes various isotopically labeled compounds as defined herein, for example those into which radioactive isotopes, such as ³H and ¹⁴C, or those into which non-radioactive isotopes, such as ²H and ¹³C are present. Such isotopically labelled compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F or labeled compound may be particularly desirable for PET or SPECT studies. Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

Furthermore, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent of a compound of the formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

### Representative Embodiments

Various embodiments of the invention are described herein. It will be recognized that features specified in each embodiment may be combined with other specified features to provide for further embodiments.

In one embodiment of the present invention R¹ is hydrogen or methyl.

In another embodiment of the present invention R³ and R⁴ are both hydrogen.

In another embodiment of the present invention R⁵ is hydrogen or halo. Suitably R⁵ is hydrogen or fluoro.

In another embodiment of the present invention R⁶ is hydrogen, halo or methyl. Suitably R⁶ is hydrogen, fluoro, chloro or methyl.

In another embodiment of the present invention R⁵ is hydrogen and R⁶ is hydrogen, halo or methyl. Suitably R⁶ is hydrogen, fluoro, chloro or methyl.

In another embodiment of the present invention R⁶ is hydrogen and R⁵ is hydrogen or halo. Suitably R⁵ is hydrogen or fluoro.

In another embodiment R⁵ and R⁶ are both hydrogen.

In another embodiment R⁷ is:

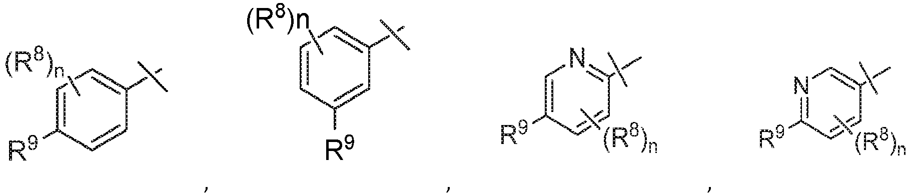

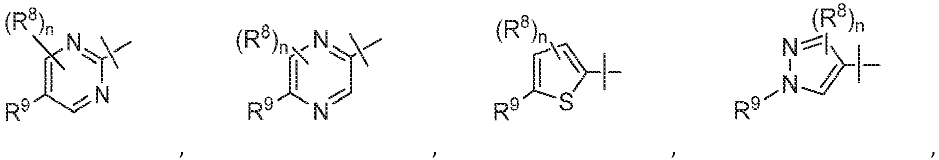

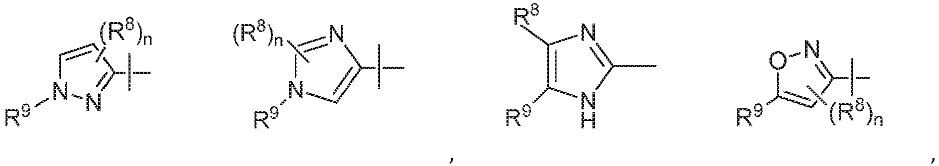

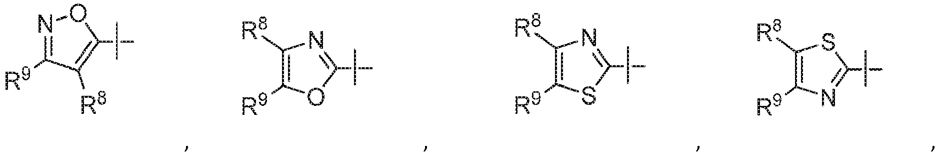

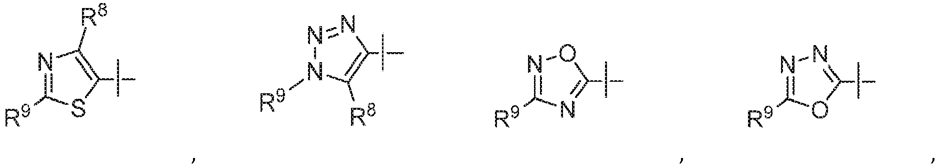

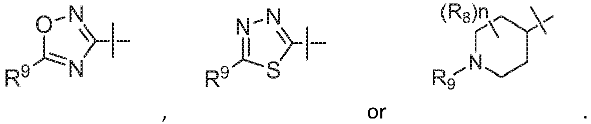

In another embodiment R⁷ is: 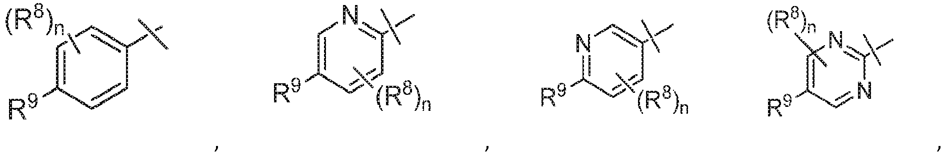 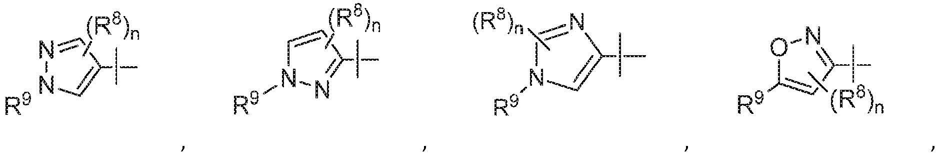 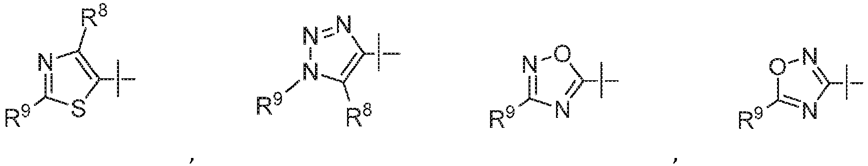 or

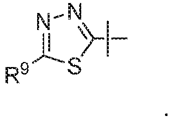

In another embodiment R⁸ is hydrogen, fluoro, chloro or methyl and n is 1. In another embodiment each R⁸ is fluoro and n is 2.

In another embodiment R⁹ is hydrogen, -OCF₃, halo, C₁₋₃ haloalkyl, optionally substituted 5 or 6 membered heterocyclic, optionally substituted optionally substituted aryl, optionally substituted heteroaryl, CH₂R^{9b} or CHCH₃R^{9b} wherein said aryl and heteroaryl are optionally substituted with one to three substituents each independently selected from the group consisting of: halo, C₁₋₆ alkyl, and C₁₋₆ haloalkyl.

In another embodiment R⁹ is hydrogen, halo, -OCF₃, or C₁₋₃ haloalkyl. Suitably R⁹ is hydrogen, -OCF₃, fluoro, CF₂CH₃ or C(CH₃)₂F.

In another embodiment R⁹ is morpholinyl.

In another embodiment R⁹ is phenyl optionally substituted with one or two substituents each independently selected from the group consisting of: fluoro, chloro, methyl, OCF₃ and C₁₋₄ haloalkyl. Suitably R⁹ is phenyl optionally substituted with one or two substituents each independently selected from the group consisting of: fluoro, chloro, methyl, OCF₃ and CF₂H. Suitably R⁹ is phenyl substituted in the para position with chloro, fluoro or CF₂H.

In another embodiment R⁹ is optionally substituted heteroaryl. Suitably R⁹ is optionally substituted pyrazolyl or pyridinyl. Suitably R⁹ is pyrazolyl or pyridinyl optionally substituted with one or two substituents each independently selected from the group consisting of C₁₋₆ alkyl and C₁₋₆ haloalkyl, for example, methyl and CF₃.

In another embodiment R⁹ is CH₂R^{9b} or CHCH₃R^{9b} wherein R^{9b} is optionally substituted heterocyclic. Suitably R^{9b} is piperidinyl, piperazinyl, morpholinyl, 3,8-diazabicyclo[3.2.1]octanyl or 3-azabicyclo[3.1.0]hexanyl each of which is optionally substituted with one to four substituents each independently selected from the group consisting of: hydroxyl, CH₂OH, -NRR, -NRC(O)CH₃, 4 to 6 membered heterocyclic, cyano, halo, C₁₋₃ alkyl, C₁₋₃ haloalkyl, and C₁₋₃ alkoxy. Suitably R^{9b} is piperidinyl or piperazinyl each of which is optionally substituted with one to four substituents each independently selected from the group consisting of: fluoro, hydroxyl, CH₂OH, fluoro, NH₂, N(CH₃)₂, NHCH₃, NHC(O)CH₃, -NH-cyclopropyl, azetidinyl, morpholinyl, methyl, and CF₃.

In another embodiment each R is independently hydrogen, methyl, or cyclopropyl.

Another embodiment of the present invention is a compound according to formula (II)

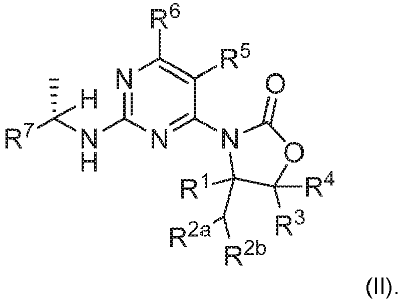

Another embodiment of the present invention is a compound according to formula (III).

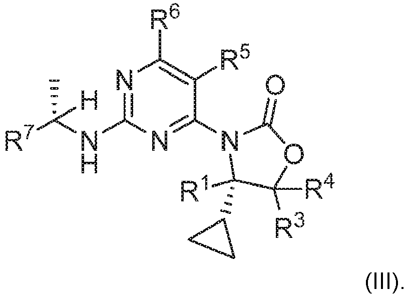

Another embodiment of the present invention is a compound according to formula (IV) 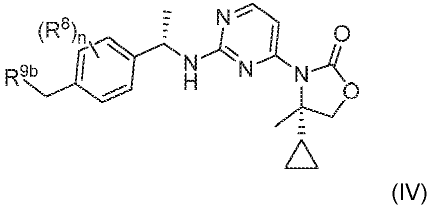 wherein R^{9b} is optionally substituted heterocyclic. Suitably R^{9b} is piperidinyl, piperazinyl, morpholinyl, 3,8-diazabicyclo[3.2.1]octanyl or 3-azabicyclo[3.1.0]hexanyl each of which is optionally substituted with one to four substituents each independently selected from the group consisting of: hydroxyl, CH₂OH, -NRR, -NRC(O)CH₃, 4 to 6 membered heterocyclic, cyano, halo, C₁₋₃ alkyl, C₁₋₃ haloalkyl, and C₁₋₃ alkoxy.

Another embodiment of the present invention is a compound according to formula (V) 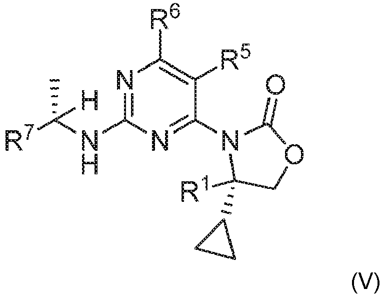 wherein:
R¹ is hydrogen or methyl;
R⁵ and R⁶ are each independently hydrogen, halo or methyl;
R⁷ is 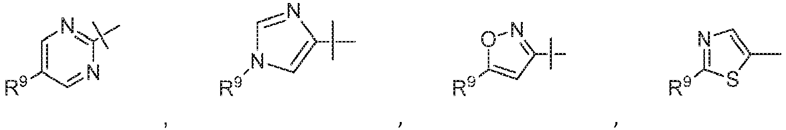 or 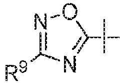 ; and
R⁹ is phenyl or pyridinyl optionally substituted with one or two substituents each independently selected from the group consisting of: fluoro, chloro, methyl, OCF₃, CF₂H, and CF₃.

Preferred compounds of the invention include:
(S)-3-(2-(((S)-1-(1-(4-chlorophenyl)-1H-imidazol-4-yl)ethyl)amino)-6-methylpyrimidin-4-yl)-4-cyclopropyloxazolidin-2-one;
(S)-3-(2-(((S)-1-(5-(4-chlorophenyl)isoxazol-3-yl)ethyl)amino)-6-methylpyrimidin-4-yl)-4-cyclopropyloxazolidin-2-one;
(S)-4-cyclopropyl-3-(2-(((S)-1-(2-(2-(trifluoromethyl)pyridin-4-yl)thiazol-5-yl)ethyl)amino)pyrimidin-4-yl)oxazolidin-2-one;
(S)-4-cyclopropyl-3-(2-(((S)-1-(2-(4-(difluoromethyl)phenyl)thiazol-5-yl)ethyl)amino)pyrimidin-4-yl)oxazolidin-2-one;
(S)-4-cyclopropyl-3-(2-(((S)-1-(4-((4-(dimethylamino)piperidin-1-yl)methyl)-3-fluorophenyl)ethyl)amino)pyrimidin-4-yl)-4-methyloxazolidin-2-one;
(S)-4-cyclopropyl-3-(2-(((S)-1-(3-fluoro-4-((4-hydroxy-4-(trifluoromethyl)piperidin-1-yl)methyl)phenyl)ethyl)amino)pyrimidin-4-yl)-4-methyloxazolidin-2-one ;
(S)-4-cyclopropyl-3-(2-(((S)-1-(4-((4-(dimethylamino)piperidin-1-yl)methyl)-2-fluorophenyl)ethyl)amino)pyrimidin-4-yl)-4-methyloxazolidin-2-one;
(S)-4-cyclopropyl-3-(2-(((S)-1-(2-fluoro-4-((4-hydroxy-4-methylpiperidin-1-yl)methyl)phenyl)ethyl)amino)pyrimidin-4-yl)-4-methyloxazolidin-2-one;
(S)-4-cyclopropyl-3-(2-(((S)-1-(2-fluoro-4-((4-hydroxy-4-(trifluoromethyl)piperidin-1-yl)methyl)phenyl)ethyl)amino)pyrimidin-4-yl)-4-methyloxazolidin-2-one;
(S)-3-(2-(((S)-1-(4-((4-(azetidin-1-yl)piperidin-1-yl)methyl)-3-fluorophenyl)ethyl)amino)pyrimidin-4-yl)-4-cyclopropyl-4-methyloxazolidin-2-one;
(S)-4-cyclopropyl-3-(2-(((S)-1-(4-((4-(cyclopropylamino)piperidin-1-yl)methyl)-3-fluorophenyl)ethyl)amino)pyrimidin-4-yl)-4-methyloxazolidin-2-one;
(S)-3-(2-(((S)-1-(4-((4-amino-4-(hydroxymethyl)piperidin-1-yl)methyl)-3-fluorophenyl)ethyl)amino)pyrimidin-4-yl)-4-cyclopropyl-4-methyloxazolidin-2-one;
(S)-3-(2-(((S)-1-(4-((4-amino-4-methylpiperidin-1-yl)methyl)-3-fluorophenyl)ethyl)amino)pyrimidin-4-yl)-4-cyclopropyl-4-methyloxazolidin-2-one;
(R)-3-(2-(((S)-1-(3-(4-chlorophenyl)-1,2,4-oxadiazol-5-yl)ethyl)amino)pyrimidin-4-yl)-4-cyclopropyl-4-methyloxazolidin-2-one;
(S)-4-cyclopropyl-3-(2-(((S)-1-(5-(4-fluoro-3-methylphenyl)pyrimidin-2-yl)ethyl)amino)pyrimidin-4-yl)-4-methyloxazolidin-2-one;
(R)-4-cyclopropyl-4-methyl-3-(2-(((S)-1-(4-((3,3,4-trimethylpiperazin-1-yl)methyl)phenyl)ethyl)amino)pyrimidin-4-yl)oxazolidin-2-one; and
(S)-3-(2-(((S)-1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one.

### General Synthetic Procedures

The compounds of the present invention may be made by a variety of methods, including standard chemistry. Suitable synthetic routes are depicted in the Schemes given below.

The compounds of formula (I) may be prepared by methods known in the art of organic synthesis as set forth in part by the following synthetic schemes. In the schemes described below, it is well understood that protecting groups for sensitive or reactive groups are employed where necessary in accordance with general principles or chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of their execution, shall be consistent with the preparation of compounds of formula (I).

Those skilled in the art will recognize if a stereocenter exists in the compounds of formula (I). Accordingly, the present invention includes both possible stereoisomers and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be effected by any suitable method known in the art. See, for example, "Stereochemistry of Organic Compounds" by E. L. Eliel, S. H. Wilen, and L. N. Mander (Wiley-Interscience, 1994).

The compounds described herein may be made from commercially available starting materials or synthesized using known organic, inorganic, and/or enzymatic processes. 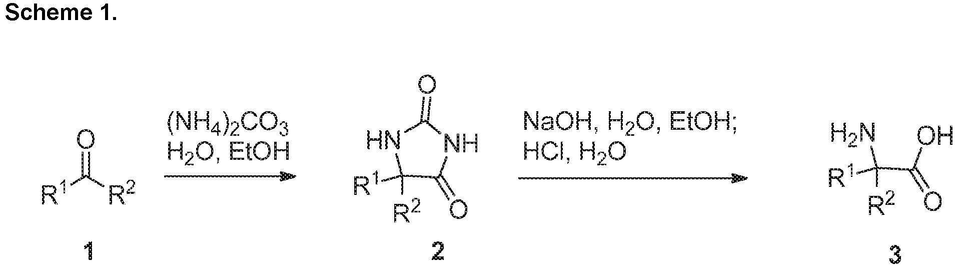 wherein R² is CH₂R^{2a}R^{2b}

Non-commercial aminoacids can be prepared following the procedures of Scheme 1. Conversion of ketone **1** to the corresponding imidazolidine-2,4-dione **2** followed by hydrolysis provides aminoacid **3.** 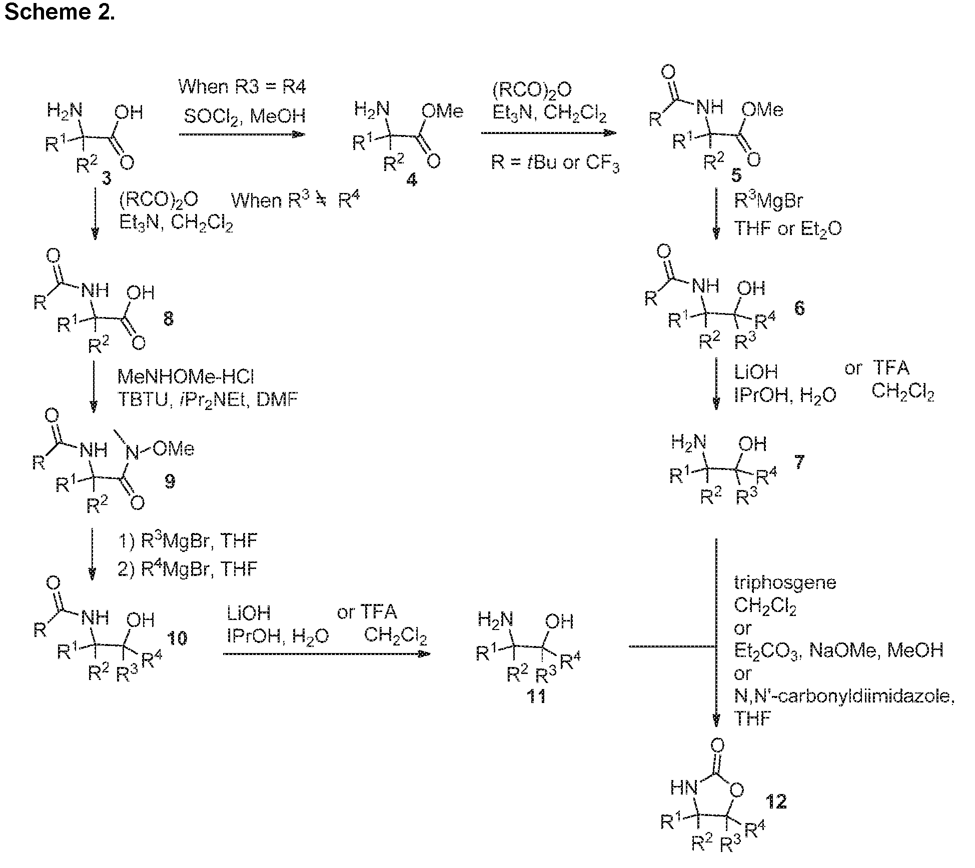 wherein R² is CH₂R^{2a}R^{2b}

When aminoalcohol, precursor of oxazolidinone, is not commercially available, it can be prepared from aminoacid **3** following the procedures of Scheme 2. When R³ = R⁴, protected aminoester **5** is treated with an appropriate Grignard reagent to give protected aminoalcohol **6** which goes through basic or acidic deprotection step. When R³ = R⁴, protected aminoacid **8** is converted into Weinreb amide 9 which is treated with different Grignard reagents sequentially to provide protected aminoalcohol **10.** Either basic or acidic deprotection of **10** gives **11.** Insertion of CO unit into **7** or **11** to provide oxazolidinone **12** is accomplished with several reagents, including (but not limited to) triphosgene, Et₂CO₃ or N-N'-darbonyldiimidazole, as shown in Scheme 2. 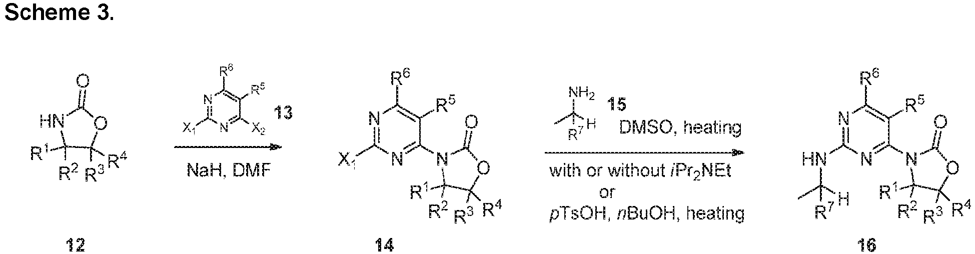 wherein R² is CH₂R^{2a}R^{2b}

Oxazolidinone **12** is coupled with dihalogen-pyrimidine **13** in the presence of NaH and the resulting **14** is treated with primary amine **15** under several different reaction conditions as shown in Scheme 3 to provide **16.** 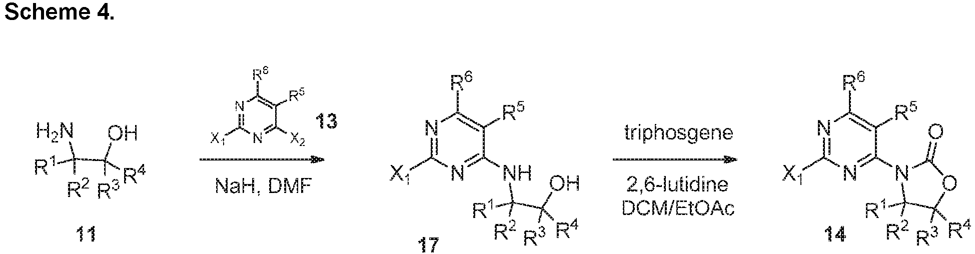 wherein R² is CH₂R^{2a}R^{2b}

Alternately intermediate **14** can be prepared by coupling the amino alcohol **11** and dihalogen-pyrimidine **13** in the presence of a base such as diisopropylethyl amine resulting in intermediate **17** which can be treated with triphosgene in the presence of a base such as 2,6-lutidine resulting in intermediate **14.**

### Methods of Use

The compounds of the present invention are inhibitors of a mutant IDH protein having a neomorphic activity and are therefore useful in the treatment of diseases or disorders associated with such proteins including, but not limited to, cell proliferation disorders, such as cancer.

Examples of a mutant IDH protein having a neomorphic activity are mutant IDH1 and mutant IDH2. A neomorphic activity associated with mutant IDH1 and mutant IDH2 is the ability to produce 2-hydroxyglutarate (2-HG neomorphic activity), specifically R-2-HG (R-2-HG neomorphic activity). Mutations in IDH1 associated with 2-HG neomorphic activity, specifically R-2-HG neomorphic activity, include mutations at residues 97, 100, and 132, e.g. G97D, R100Q, R132H, R132C, R132S, R132G, R132L, and R132V. Mutations in IDH2 associated with 2-HG neoactivity, specifically R-2-HG neomorphic activity, include mutations at residues 140 and 172, e.g. R140Q, R140G, R172K, R172M, R172S, R172G, and R172W.

Cell-proliferation disorders associated with a mutant IDH protein having a neomorphic activity include, but are not limited to, cancer. Examples of such cancers include Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood; Carcinoid Tumor, Childhood; Carcinoid Tumor, Gastrointestinal; Carcinoma, Adrenocortical; Carcinoma, Islet Cell; Carcinoma of Unknown Primaiy; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma, Childhood Brain Stem; Glioma, Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS- Related; Lymphoma, Central Nervous System (Primary); Lymphoma, Cutaneous T-Cell; Lymphoma, Hodgkin's, Adult; Lymphoma, Hodgkin's, Childhood; Lymphoma, Hodgkin's During Pregnancy;Lymphoma, Non-Hodgkin' s, Adult; Lymphoma, Non-Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplastic Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; steosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood; Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T- Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor.

In another embodiment the cancer associated with a mutant IDH protein having a neomorphic acitvity is brain cancer, such as astrocytic tumor (e.g., pilocytic astrocytoma, subependymal giant-cell astrocytoma, diffuse astrocytoma, pleomorphic xanthoastrocytoma, anaplastic astrocytoma, astrocytoma, giant cell glioblastoma, glioblastoma, secondary glioblastoma, primary adult glioblastoma, and primary pediatric glioblastoma); oligodendroglial tumor (e.g., oligodendroglioma, and anaplastic oligodendroglioma); oligoastrocytic tumor (e.g., oligoastrocytoma, and anaplastic oligoastrocytoma); ependymoma (e.g., myxopapillary ependymoma, and anaplastic ependymoma); medulloblastoma; primitive neuroectodermal tumor, schwannoma, meningioma, meatypical meningioma, anaplastic meningioma; and pituitary adenoma. In another embodiment, the brain cancer is glioma, glioblastoma multiforme, paraganglioma, or suprantentorial primordial neuroectodermal tumors (sPNET).

In another embodiment the cancer associated with a mutant IDH protein having a neomorphic acitvity is leukemia, such as acute myeloid leukemia (AML), myelodysplastic syndrome (MDS), chronic myelogenous leukemia (CML), myeloproliferative neoplasm (MPN), MDS.MPN including chronic myelomonocytic leukemia, post MDS AML, post MPN AML, post MDS/MPN AML, del(5q)-associated high risk MDS or AML, blast-phase chronic myelogenous leukemia, angioimmunoblastic lymphoma and acute lymphoblastic leukemia.

In another embodiment the cancer associated with a mutant IDH protein having a neomorphic activity is skin cancer, including melanoma.

In another embodiment the cancer associated with a mutant IDH protein having a neomorphic activity is prostate cancer, thyroid cancer, colon cancer, or lung cancer.

In another embodiment the cancer associated with a mutant IDH protein having a neomorphic activity is sarcoma, including central chondrosarcoma, central and periosteal chondroma, and fibrosarcoma.

In another embodiment the cancer associated with a mutant IDH protein having a neomorphic activity is cholangiocarcinoma.

Another disease or disorder associated with a mutant IDH protein having R-2-HG neomorphic activity is D-2-hydroxyglutaric aciduria.

Another disease or disorder associated with a mutant IDH protein having R-2-HG neomorphic activity is Diller disease and Mafucci syndrome.

As used herein the term "neomorphic activity" refers to a gain of novel activity of a protein that the wild-type protein does not have or does not exhibit to a significant degree. For example, a neomorphic activity associated with a mutant form of IDH1 and IDH2 is the ability to reduce alpha-ketoglutarate to 2-hydroxyglutarate (i.e. 2-HG, specifically R-2-HG). The wild type form of IDH1 and IDH2 does not have the ability to reduce alpha-ketoglutarate to 2-hydroxyglutarate (i.e. 2-HG, specifically R-2-HG) or if it does have this ability, it does not produce significant (i.e. harmful or disease causing) amounts of 2-HG.

As used herein, the term "subject" refers to an animal. Typically the animal is a mammal. A subject also refers to for example, primates (*e.g*., humans, male or female), cows, sheep, goats, horses, dogs, cats, rabbits, rats, mice, fish, birds and the like. In certain embodiments, the subject is a primate. In yet other embodiments, the subject is a human.

As used herein, the term "therapeutically effective amount" in reference to a compound of the invention means an amount of the compound sufficient to treat the subject's disease or condition, but low enough to avoid serious sides effects (at a reasonable benefit/risk ratio) within the scope of sound medical judgment. A therapeutically effective amount of a compound will vary with the particular compound chosen (e.g. consider the potency, efficacy, and half-life of the compound); the route of administration chosen; the condition being treated; the severity of the condition being treated; the age, size, weight, and physical condition of the subject being treated; the medical history of the subject being treated; the duration of the treatment; the nature of the concurrent therapy; the desired therapeutic effect; and like factors and can be routinely determined by the skilled artisan.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g.,* stabilization of a discernible symptom), physiologically, (*e.g.,* stabilization of a physical parameter), or both. In yet another embodiment, "treat", "treating" or "treatment" refers to preventing or delaying the onset or development or progression of the disease or disorder.

As used herein, a subject is "in need of" a treatment if such subject would benefit biologically, medically or in quality of life from such treatment.

The compounds of the present invention may be administered by any suitable route including oral and parenteral administration. Parenteral administration is typically by injection or infusion and includes intravenous, intramuscular, and subcontaneous injection or infusion.

The compounds of the invention may be administered once or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. Suitable dosing regimens for a compound of the invention depend on the pharmacokinetic properties of that compound, such as absorption, distribution and half life which can be determined by the skilled artisan. In addition, suitable dosing regimens, including the duration such regimens are administered, for a compound of the invention depend on the disease or condition being treated, the severity of the disease or condition, the age and physical condition of the subject being treated, the medical history of the subject being treated, the nature of concurrent therapy, the desired therapeutic effect, and like factors within the knowledge and expertise of the skilled artisan. It will be further understood by such skilled artisans that suitable dosing regimens may require adjustment given an individual subject's response to the dosing regimen or over time as the individual subject needs change. Typical daily dosages may vary depending upon the particular route of administration chosen. Typical daily dosages for oral administration, to a human weighing approximately 70kg would range from about 5mg to about 500mg of a compound of formula (I).

One embodiment of the present invention provides for compounds for use in a method of treating a disease or disorder associated with a mutant form of IDH having a neomorphic activity comprising administration of a therapeutically effective amount of a compound of formula (I) to a subject in need of treatment thereof. In one embodiment, the disease or disorder associated with a mutant form of IDH having a neomorphic activity is a cell proliferation disorder. In another embodiment, the cell proliferation disorder is cancer. In another embodiment, the cancer is a cancer associated with mutant IDH1 having 2-HG neomorphic activity or mutant IDH2 having 2-HG neomorphic activity. In another embodiment the neomorphic activity is R-2-HG neomorphic activity. In another embodiment the cancer is associated with mutant IDH1 having 2-HG or R-2-HG neomorphic activity having a mutation at residues 97, 100, or 132, such as G97D, R100Q, R132H, R132C, R132S, R132G, R132L, and R132V. In another embodiment the cancer is associated with mutant IDH2 having 2-HG or R-2-HG neomorphic activity having a mutation at residues 140 or 172, e.g. R140Q, R140G, R172K, R172M, R172S, R172G, and R172W. In another embodiment the cancer is brain cancer, leukemia, skin cancer, prostate cancer, thyroid cancer, colon cancer, lung cancer or sarcoma. In another embodiment the cancer is glioma, glioblastoma multiforme, paraganglioma, suprantentorial primordial neuroectodermal tumors, acute myeloid leukemia, myelodysplastic syndrome, chronic myelogenous leukemia, melanoma, prostate, thyroid, colon, lung, central chondrosarcoma, central and periosteal chondroma tumors, fibrosarcoma, and cholangiocarcinoma.

Another embodiment of the present invention provides for compounds for use in a method of treating a disease or disorder associated with a mutant form of IDH having R-2-HG neomorphic activity comprising administration of a therapeutically effective amount of a compound according to formula (I) to a subject in need thereof wherein the disease or disorder is D-2-hydroxyglutaric aciduria, Ollier Disease, or Mafucci Syndrome.

Another embodiment of the present invention provides for the use of a compound of formula (I) in therapy. In a further embodiment the therapy is a disease or disorder associated with a mutant form of IDH having a neomorphic activity. In another embodiment the therapy is a cell proliferation disorder associated with a mutant form of IDH having a neomorphic activity. In another embodiment the therapy is cancer. In another embodiment the therapy is a cancer associated with a mutant IDH protein having a neomorphic activity, such as mutant IDH1 having 2-HG neomorphic activity or mutant IDH2 having 2-HG neomorphic activity. In another embodiment the neomorphic activity is R-2-HG neomorphic activity. In another embodiment the cancer is associated with mutant IDH1 having 2-HG or R-2-HG neomorphic activity having a mutation at residues 97, 100, or 132, such as G97D, R100Q, R132H, R132C, R132S, R132G, R132L, and R132V. In another embodiment the cancer is associated with mutant IDH2 having 2-HG or R-2-HG neomorphic activity having a mutation at residue at residues R140 or 172, e.g. R140Q, R140G, R172K, R172M, R172S, R172G, and R172W. In another embodiment the cancer is brain cancer, leukemia, skin cancer, prostate cancer, thyroid cancer, colon cancer, lung cancer or sarcoma. In another embodiment the cancer is glioma, glioblastoma multiforme, paraganglioma, suprantentorial primordial neuroectodermal tumors, acute myeloid leukemia, myelodysplastic syndrome, chronic myelogenous leukemia, melanoma, prostate, thyroid, colon, lung, central chondrosarcoma, central and periosteal chondroma tumors, fibrosarcoma, and cholangiocarcinoma.

Another embodiment of the present invention provides for the use of a compound of formula (I) in therapy wherein the therapy is D-2-hydroxyglutaric aciduria, Ollier Disease, or Mafucci Syndrome.

Another embodiment of the present invention provides for the use of a compound according to formula (I) in the manufacture of a medicament for the treatment of disease or disorder associated with a mutant form of IDH having a neomorphic activity. In one embodiment the disease or disorder associated with a mutant form of IDH having a neomorphic activity is a cell proliferation disorder. In another embodiment, the cell proliferation disorder is cancer. In another embodiment the cancer is a cancer associated with a mutant IDH protein having a neomorphic activity, such as mutant IDH1 having 2-HG neomorphic activity or mutant IDH2 having 2-HG neomorphic activity. In another embodiment the neomorphic activity is R-2-HG neomorphic activity. In another embodiment the cancer is associated with mutant IDH1 having 2-HG or R-2-HG neomorphic activity having a mutation at residues 97, 100, or 132, such as G97D, R100Q, R132H, R132C, R132S, R132G, R132L, and R132V. In another embodiment the cancer is associated with mutant IDH2 having 2-HG or R-2-HG neomorphic activity having a mutation at residue at residues 140 or 172, e.g. R140Q, R140G, R172K, R172M, R172S, R172G, and R172W. In another embodiment the cancer is brain cancer, leukemia, skin cancer, prostate cancer, thyroid cancer, colon cancer, lung cancer or sarcoma. In another embodiment the cancer is glioma, glioblastoma multiforme, paraganglioma, suprantentorial primordial neuroectodermal tumors, acute myeloid leukemia, myelodysplastic syndrome, chronic myelogenous leukemia, melanoma, prostate, thyroid, colon, lung, central chondrosarcoma, central and periosteal chondroma tumors, fibrosarcoma, and cholangiocarcinoma.

Another embodiment of the present invention provides for the use of a compound according to formula (I) in the manufacture of a medicament for the treatment of disease or disorder associated with a mutant form of IDH having R-2-HG neomorphic activity wherein the disease or disorder is D-2-hydroxyglutaric aciduria, Ollier Disease, or Mafucci Syndrome.

### Compositions

In another aspect, the present invention provides a pharmaceutical composition comprising a compound of formula (I) and a pharmaceutically acceptable carrier or excipient.

The pharmaceutical compositions of the invention may be prepared and packaged in bulk form wherein a therapeutically effective amount of a compound of the invention can be extracted and then given to a subject, such as with powders or syrups. Alternatively, the pharmaceutical compositions of the invention may be prepared and packaged in unit dosage form wherein each physically discrete unit contains a therapeutically effective amount of a compound of the invention. When prepared in unit dosage form, the pharmaceutical compositions of the invention typically contain from about 5mg to 500mg of a compound of formula (I).

As used herein the term "pharmaceutically acceptable carrier or excipient" means a pharmaceutically acceptable material, composition or vehicle that, for example, are involved in giving form or consistency to the pharmaceutical composition. Each excipient must be compatible with the other ingredients of the pharmaceutical composition when commingled such that interactions which would substantially reduce the efficacy of the compound of the invention when administered to a subject and interactions which would result in pharmaceutical compositions that are not pharmaceutically acceptable are avoided. In addition, each excipient must, of course, be of sufficiently high purity to render it pharmaceutically acceptable.

The compound of the invention and the pharmaceutically acceptable carrier or excipient(s) will typically be formulated into a dosage form adapted for administration to the subject by the desired route of administration. For example, dosage forms include those adapted for (1) oral administration such as tablets, capsules, caplets, pills, troches, powders, syrups, elixirs, suspensions, solutions, emulsions, sachets, and cachets; and (2) parenteral administration such as sterile solutions, suspensions, and powders for reconstitution. Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting of the compound or compounds of the invention, once administered to the subject, from one organ or portion of the body to another organ or another portion of the body. Certain pharmaceutically acceptable excipients may be chosen for their ability to enhance patient compliance.

Suitable pharmaceutically acceptable excipients include the following types of excipients: diluents, lubricants, binders, disintegrants, fillers, glidants, granulating agents, coating agents, wetting agents, solvents, co-solvents, suspending agents, emulsifiers, sweeteners, flavoring agents, flavor masking agents, coloring agents, anti-caking agents, hemectants, chelating agents, plasticizers, viscosity increasing agents, antioxidants, preservatives, stabilizers, surfactants, and buffering agents.

Skilled artisans possess the knowledge and skill in the art to enable them to select suitable pharmaceutically acceptable carriers and excipients in appropriate amounts for the use in the invention. In addition, there are a number of resources available to the skilled artisan, which describe pharmaceutically acceptable carriers and excipients and may be useful in selecting suitable pharmaceutically acceptable carriers and excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some methods commonly used in the art are described in Remington's Pharmaceutical Sciences (Mack Publishing Company).

In one aspect, the invention is directed to a solid oral dosage form such as a tablet or capsule comprising a therapeutically effective amount of a compound of the invention and a diluent or filler. Suitable diluents and fillers include lactose, sucrose, dextrose, mannitol, sorbitol, starch (e.g. corn starch, potato starch, and pre-gelatinized starch), cellulose and its derivatives, (e.g. microcrystalline cellulose), calcium sulfate, and dibasic calcium phosphate. The oral solid dosage form may further comprise a binder. Suitable binders include starch (e.g. corn starch, potato starch, and pre-gelatinized starch) gelatin, acacia, sodium alginate, alginic acid, tragacanth, guar gum, povidone, and cellulose and its derivatives (e.g. microcrystalline cellulose). The oral solid dosage form may further comprise a disintegrant. Suitable disintegrants include crospovidone, sodium starch glycolate, croscarmelose, alginic acid, and sodium carboxymethyl cellulose. The oral solid dosage form may further comprise a lubricant. Suitable lubricants include stearic acid, magnesium stearate, calcium stearate, and talc.

Where appropriate, dosage unit formulations for oral administration can be microencapsulated. The composition can also be prepared to prolong or sustain the release as, for example, by coating or embedding particulate material in polymers, wax, or the like.

The compounds of the invention may also be coupled with soluble polymers as targetable drug carriers. Such polymers can include polyvinylpyrrolidone, pyrancopolymer, polyhydroxypropylmethacrylamidephenol, polyhydroxyethylaspartamidephenol, or polyethyleneoxidepolylysine substituted with palmitoyl residues. Furthermore, the compounds of the invention may be coupled to a class of biodegradable polymers useful in achieving controlled release of a drug, for example polylactic acid, polepsilon caprolactone, polyhydroxy butyric acid, polyorthoesters, polyacetals, polydihydropyrans, polycyanacrylates and crosslinked or amphipathic block copolymers of hydrogels.

In another aspect, the invention is directed to a liquid oral dosage form. Oral liquids such as solution, syrups and elixirs can be prepared in dosage unit form so that a given quantity contains a predetermined amount of a compound of the invention. Syrups can be prepared by dissolving the compound of the invention in a suitably flavored aqueous solution; while elixirs are prepared through the use of a non-toxic alcoholic vehicle. Suspensions can be formulated by dispersing the compound of the invention in a non-toxic vehicle. Solubilizers and emulsifiers such as ethoxylated isostearyl alcohols and polyoxy ethylene sorbitol ethers, preservatives, flavor additives such as peppermint oil or other natural sweeteners or saccharin or other artificial sweeteners and the like can also be added.

In another aspect, the invention is directed to parenteral administration. Pharmaceutical compositions adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

### Combinations

The compound of the present invention may be administered either simultaneously with, or before or after, one or more other therapeutic agent(s). The compound of the present invention may be administered separately, by the same or different route of administration, or together in the same pharmaceutical composition as the other agent(s).

In one embodiment, the invention provides a product comprising a compound of formula (I) and at least one other therapeutic agent as a combined preparation for simultaneous, separate or sequential use in therapy. In one embodiment, the therapy is the treatment of a disease or disorder associated with a mutant form of IDH. Products provided as a combined preparation include a composition comprising the compound of formula (I) and the other therapeutic agent(s) together in the same pharmaceutical composition, or the compound of formula (I) and the other therapeutic agent(s) in separate form, e.g. in the form of a kit.

In one embodiment, the invention provides a pharmaceutical composition comprising a compound of formula (I) and another therapeutic agent(s). Optionally, the pharmaceutical composition may comprise a pharmaceutically acceptable excipient, as described above.

In one embodiment, the invention provides a kit comprising two or more separate pharmaceutical compositions, at least one of which contains a compound of formula (I). In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

In the combination therapies of the invention, the compound of the invention and the other therapeutic agent may be manufactured and/or formulated by the same or different manufacturers. Moreover, the compound of the invention and the other therapeutic agent may be brought together into a combination therapy: (i) prior to release of the combination product to physicians (*e.g*. in the case of a kit comprising the compound of the invention and the other therapeutic agent); (ii) by the physician themselves (or under the guidance of the physician) shortly before administration; (iii) in the patient themselves, *e.g*. during sequential administration of the compound of the invention and the other therapeutic agent.

Accordingly, the invention provides the use of a compound of formula (I) for treating a disease or disorder associated with a mutant form of IDH, wherein the medicament is prepared for administration with another therapeutic agent. The invention also provides the use of another therapeutic agent for treating a disease or disorder associated with a mutant form of IDH, wherein the medicament is administered with a compound of formula (I).

The invention also provides a compound of formula (I) for use in a method of treating a disease or disorder associated with a mutant form of IDH, wherein the compound of formula (I) is prepared for administration with another therapeutic agent. The invention also provides another therapeutic agent for use in a method of treating a disease or disorder associated with a mutant form of IDH, wherein the other therapeutic agent is prepared for administration with a compound of formula (I). The invention also provides a compound of formula (I) for use in a method of treating a disease or disorder associated with a mutant form of IDH, wherein the compound of formula (I) is administered with another therapeutic agent. The invention also provides another therapeutic agent for use in a method of treating a disease or disorder associated with a mutant form of IDH, wherein the other therapeutic agent is administered with a compound of formula (I).

The invention also provides the use of a compound of formula (I) for treating a disease or disorder associated with a mutant form of IDH, wherein the patient has previously (*e.g*. within 24 hours) been treated with another therapeutic agent. The invention also provides the use of another therapeutic agent for treating a disease or disorder associated with a mutant form of IDH, wherein the patient has previously (*e.g*. within 24 hours) been treated with a compound of formula (I).

In one embodiment, the other therapeutic agent is selected from: vascular endothelial growth factor (VEGF) receptor inhibitors, topoisomerase II inhibitors, smoothen inhibitors, alkylating agents, anti-tumor antibiotics, anti-metabolites, retinoids, and other cytotoxic agents.

Examples of vascular endothelial growth factor (VEGF) receptor inhibitors include, but are not limited to, bevacizumab (sold under the trademark Avastin® by Genentech/Roche), axitinib, (N-methyl-2-[[3-[(E)-2-pyridin-2-ylethenyl]-1*H*-indazol-6-yl]sulfanyl]benzamide, also known as AG013736, and described in PCT Publication No. WO 01/002369), Brivanib Alaninate ((*S*)-((*R*)-1-(4-(4-Fluoro-2-methyl-1*H*-indol-5-yloxy)-5-methylpyrrolo[2,1-*f*][1,2,4]triazin-6-yloxy)propan-2-yl)2-aminopropanoate, also known as BMS-582664), motesanib (N-(2,3-dihydro-3,3-dimethyl-1H-indol-6-yl)-2-[(4-pyridinylmethyl)amino]-3-pyridinecarboxamide, and described in PCT Publication No. WO 02/066470), pasireotide (also known as SOM230, and described in PCT Publication No. WO 02/010192), and sorafenib (sold under the tradename Nexavar®).

Examples of topoisomerase II inhibitors, include but are not limited to, etoposide (also known as VP-16 and Etoposide phosphate, sold under the tradenames Toposar®, VePesid® and Etopophos®), and teniposide (also known as VM-26, sold under the tradename Vumon®).

Examples of alkylating agents, include but are not limited to, temozolomide (sold under the tradenames Temodar® and Temodal® by Schering-Plough/Merck), dactinomycin (also known as actinomycin-D and sold under the tradename Cosmegen®), melphalan (also known as L-PAM, L-sarcolysin, and phenylalanine mustard, sold under the tradename Alkeran®), altretamine (also known as hexamethylmelamine (HMM), sold under the tradename Hexalen®), carmustine (sold under the tradename BiCNU®), bendamustine (sold under the tradename Treanda®), busulfan (sold under the tradenames Busulfex® and Myleran®), carboplatin (sold under the tradename Paraplatin®), lomustine (also known as CCNU, sold under the tradename CeeNU®), cisplatin (also known as CDDP, sold under the tradenames Platinol® and Platinol®-AQ), chlorambucil (sold under the tradename Leukeran®), cyclophosphamide (sold under the tradenames Cytoxan® and Neosar®), dacarbazine (also known as DTIC, DIC and imidazole carboxamide, sold under the tradename DTIC-Dome®), altretamine (also known as hexamethylmelamine (HMM) sold under the tradename Hexalen®), ifosfamide (sold under the tradename Ifex®), procarbazine (sold under the tradename Matulane®), mechlorethamine (also known as nitrogen mustard, mustine and mechloroethamine hydrochloride, sold under the tradename Mustargen®), streptozocin (sold under the tradename Zanosar®), thiotepa (also known as thiophosphoamide, TESPA and TSPA, and sold under the tradename Thioplex®.

Examples of anti-tumor antibiotics include, but are not limited to, doxorubicin (sold under the tradenames Adriamycin® and Rubex®), bleomycin (sold under the tradename lenoxane®), daunorubicin (also known as dauorubicin hydrochloride, daunomycin, and rubidomycin hydrochloride, sold under the tradename Cerubidine®), daunorubicin liposomal (daunorubicin citrate liposome, sold under the tradename DaunoXome®), mitoxantrone (also known as DHAD, sold under the tradename Novantrone®), epirubicin (sold under the tradename Ellence™), idarubicin (sold under the tradenames Idamycin®, Idamycin PFS®), and mitomycin C (sold under the tradename Mutamycin®).

Examples of anti-metabolites include, but are not limited to, claribine (2-chlorodeoxyadenosine, sold under the tradename leustatin®), 5-fluorouracil (sold under the tradename Adrucil®), 6-thioguanine (sold under the tradename Purinethol®), pemetrexed (sold under the tradename Alimta®), cytarabine (also known as arabinosylcytosine (Ara-C), sold under the tradename Cytosar-U®), cytarabine liposomal (also known as Liposomal Ara-C, sold under the tradename DepoCyt™), decitabine (sold under the tradename Dacogen®), hydroxyurea (sold under the tradenames Hydrea®, Droxia™ and Mylocel™), fludarabine (sold under the tradename Fludara®), floxuridine (sold under the tradename FUDR®), cladribine (also known as 2-chlorodeoxyadenosine (2-CdA) sold under the tradename Leustatin™), methotrexate (also known as amethopterin, methotrexate sodim (MTX), sold under the tradenames Rheumatrex® and Trexall™), and pentostatin (sold under the tradename Nipent®).

Examples of retinoids include, but are not limited to, alitretinoin (sold under the tradename Panretin®), tretinoin (all-trans retinoic acid, also known as ATRA, sold under the tradename Vesanoid®), Isotretinoin (13-*cis*-retinoic acid, sold under the tradenames Accutane®, Amnesteem®, Claravis®, Clarus®, Decutan®, Isotane®, Izotech®, Oratane®, Isotret®, and Sotret®), and bexarotene (sold under the tradename Targretin®).

Examples of other cytotoxic agents include, but are not limited to, arsenic trioxide (sold under the tradename Trisenox®), asparaginase (also known as L-asparaginase, and Erwinia L-asparaginase, sold under the tradenames Elspar® and Kidrolase®).

### Intermediates and Examples

The following examples are intended to be illustrative only and not limiting in any way. Unless otherwise noted, the following Intermediates and Examples were purified vial silica gel column chromatograph using RediSep® Rf columns from Teledyne Isco, Inc. Abbreviations used are those conventional in the art or the following:
ACN acetonitrile
BSA bovine serum albumin
C Celsius
d doublet
dd doublet of doublets
DAST diethylaminosulfur trifluoride
DIPEA NN-diisopropylethylamine
DMF N,N-dimethylformamide
DMSO dimethylsulfoxide
DTT dithiothreitol
EtOAc ethyl acetate
EtOH ethanol
g gram
h hour(s)
HATU 2-(1H-7-azabenzotriazol-1-yl)-1,1,3,3-tetramethyluronium hexafluorophosphate
HEPES 4-(2-hydroxyethyl)-1-piperazineethylanesulfonic acid
HPLC high pressure liquid chromatography
IPA isopropyl alcohol
kg kilogram
L liter
LAH lithium aluminum hydride
LC liquid chromatography
LCMS liquid chromatography and mass spectrometry
MeOH methanol
MS mass spectrometry
m multiplet
min minutes
mL milliliter(s)
µM micromolar
m/z mass to charge ratio
nm nanometer
nM nanomolar
N normal
NADPH nicotinamide adenine dinucleotide phosphate
NMP N-methylpyrrolidone
NMR nuclear magnetic resonance
PdCl₂(dppf).CH₂Cl₂ 1,1'-bis(diphenylphosphino)ferrocene-palladium(II)dichloride dichloromethane complex
rac racemic
Rt retention time
s singlet
sat. saturated
SFC supercritical fluid chromatography
t triplet
TCEP tris(2-carboxyethyl)phosphine
TEA triethylamine
TFA trifluoroacetic acid
THF tetrahydrofuran

### Instrumentation

### LCMS:

Unless otherwise noted, LCMS data (also reported herein as simply MS) were recorded using a Waters System (Acuity UPLC and a Micromass ZQ mass spectrometer; Column: Acuity HSS C18 1.8-micron, 2.1 x 50 mm; gradient: 5-95 % acetonitrile in water with 0.05 % TFA over a 1.8 min period; flow rate 1.2 mL/min; molecular weight range 200-1500; cone Voltage 20 V; column temperature 50 °C or Column: Inertsil C8 Column, 3.0 µm, 3.0 x 30 mm, column temperature 50 °C, eluents =A: Water (5 mM Ammonium formate, 2% ACN); B: AC, Flow Rate =2 mL/min. Gradient =0 min 5% B; 5% to 95% B in 1.70 min; 0.3 min 95% B; 2.1 min 1%B or Column: XBridge C18 3.5-micron, 3 x 30 mm; Eluent A: Water + 3.75 mM Ammonium Acetate + 5% Water, Eluent B: Acetonitrile; gradient: 5-95 % B in 1.8 min period; flow 2 mL/min). All masses reported are those of the protonated parent ions unless recorded otherwise.

High Resolution Mass Spectrometry (HRMS):
HRMS Method A: ESI-MS data were recorded using a Synapt G2 HDMS (TOF mass spectrometer, Waters) with electrospray ionization source. The resolution of the MS system was approximately 15000. Leucine Enkephalin was used as lock mass (internal standards) infused from lockspary probe. The compound was infused into the mass spectrometer by UPLC (Acquity, Waters) from sample probe. The separation was performed on Acquity UPLC BEH C18 1x50 mm column at 0.2 mL/min flow rate with the gradient from 5% to 95% in 3 min. Solvent A was Water with 0.1 % Formic Acid and solvent B was Acetonitrile with 0.1 % Formic Acid. The mass accuracy of the system has been found to be <5 ppm with lock mass.

HRMS Method B: LC-MS/ESI-MS data were recorded on an Acquity G2 Xevo QTof - Rs(FWHM) > 20000 Accuracy < 5 ppm. The separation was performed on Acquity CSH 1.7µm 2.1x50mm - 50°C column Eluent A: Water + 3.75 mM ammonium acetate + 0.0001% formic acid. Eluent B: Acetonitrile. Gradient: from 2 to 98% B in 4.4 min - flow 1.0 mL/min.

HRMS Method C: LC-MS/ESI-MS data were recorded on an Acquity LCTp Tof - Rs(FWHM) > 12000 Accuracy < 5 ppm. The separation was performed on Acquity BEHC18 1.7µm 2.1x50mm - 50°C column Eluent A: Water + 0.1 % Formic Acid + 3.75 mM Ammonium Acetate. Eluent B: Acetonitrile + 0.04% Formic Acid + 3.75 mM Ammonium Acetate + 5% Water. Gradient: from 0.2 to 30% B in 5.0 min - flow 1.0 mL/min.

HRMS methods A, B and C are referred to throughout as HRMS(A), HRMS(B) and HRMS(C) respectively.

### Intermediates

### Intermediate A: (S)-4-cyclopropyloxazolidin-2-one

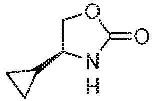

### Step 1: Preparation of (S)-methyl 2-(tert-butoxycarbonylamino)-2-cyclopropylacetate

To (S)-2-(tert-butoxycarbonylamino)-2-cyclopropylacetic acid (5.01 g, 23.28 mmol) in MeOH (50mL) was added portionwise to trimethylsilyldiazomethane (18.62 ml, 37.2 mmol) until no bubbles. The reaction was stirred for 30 minutes and quenched with drops of HOAc (0.1 mL). The reaction mixture was then concentrated under vacuum to give crude product as a light tan oil (5.35 g). *m*/*z* 230.2 (M + H)⁺

### Step 2: Preparation of (S)-tert-butyl 1-cyclopropyl-2-hydroxyethylcarbamate

To a solution of (S)-methyl 2-(tert-butoxycarbonylamino)-2-cyclopropylacetate (5.35 g, 23.33 mmol) in Et₂O (100 ml) was added LiBH₄ (0.762 g, 35.0 mmol), followed by dropwise addition of methanol (1.420 ml, 35.0 mmol). The reaction was refluxed at 40 °C for one hour. The reaction mixture was then cooled to 0 °C, and quenched with HCl (1M) until pH=2 for aqueous layer. The phases were separated and the aqueous layer was extracted with DCM (3x100mL). The organic was then dried (Na₂SO₄) and concentrated in vacuo. to give crude product (4.16 g).) *m*/*z* 224.1 (M + Na)⁺

### Step 3: Preparation of (S)-4-cyclopropyloxazolidin-2-one

To (S)-tert-butyl 1-cyclopropyl-2-hydroxyethylcarbamate (4.01 g, 19.92 mmol) in THF (100 ml) was added portionwise potassium 2-methylpropan-2-olate (2.91 g, 25.9 mmol). The reaction was stirred for five hours and quenched with HCl (1M, 27mL) to pH=2. The reaction mixture was then concentrated under vacuum to about one third of the volume, and diluted with water (50mL). The aqueous layer was then extracted with DCM (3x100mL). The combined organic was washed with brine (20 mL), dried (Na₂SO₄) and concentrated to give crude product as a light tan oil (2.35 g). *m*/*z* 128.1 (M + H)⁺

### Intermediate B: 4-cyclopropyl-4-methyloxazolidin-2-one

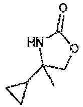

To a cooled (0 °C) solution of 2-amino-2-cyclopropylpropan-1-ol (6.86 g, 59.6 mmol) in DCM (140 ml) was added triethylamine (24.11 g, 238 mmol) followed by the dropwise addition of triphosgene (6.27 g, 21.1 mmol) in DCM (25 ml). The solution was stirred at room temperature for 15 min. Ice-bath was removed and the solution was allowed to stir at room temperature for 1 h. DCM was removed in vaccuo and the residue was dissolved in EtOAc (100 ml) then washed with sat. aqueous NH₄Cl (100 ml). Organic layer was dried over Na₂SO₄, filtered and concentrated to give 4-cyclopropyl-4-methyloxazolidin-2-one (7.78 g, 55.1 mmol, 93%) ¹H NMR (400 MHz, MeOD) δ 4.22 (d, *J* = 8.4 Hz, 1H), 4.13 (d, *J* = 8.4 Hz, 1H), 1.36 (s, 3H), 1.05 (tt, *J* = 8.4, 5.4 Hz, 1H), 0.57 - 0.43 (m, 2H), 0.43 - 0.30 (m, 2H).

### Intermediate C: 3-(2-chloropyrimidin-4-yl)-4-cyclopropyl-4-methyloxazolidin-2-one

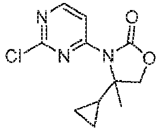

To a solution of 2-(2-chloropyrimidin-4-ylamino)-2-cyclopropylpropan-1-ol (55 mg, 0.242 mmol) in 2 mL THF was added triethylamine (109 mg, 1.08 mmol) followed by dropwise addition of a solution of triphosgene (35 mg, 0.121 mmol) in 1 mL of THF. After addition was completed, the mixture was heated to 50 °C for 2 h. The reaction mixture was diluted with water (30 mL) and extracted with EtOAc (2 x 30 mL). Organic layers were washed with water, brine, dried over Na₂SO₄, filtered and concentrated. The residue was purified by column chromatography (0 - 50% EtOAc/hept) to give title compound (25 mg, 0.099 mmol).
¹H NMR (400 MHz, MeOD) δ 8.52 (d, *J* = 5.9 Hz, 1H), 8.08 (d, *J* = 6.0 Hz, 1H), 4.12 - 3.94 (m, 2H), 1.90 - 1.73 (m, 1H), 1.69 (s, 3H), (m, 2H), 0.59 - 0.31 (m, 2H). MS 254.1 *m*/*z* (M+H).

### Intermediate D: 4-cyclopropyl-3-(2-fluoropyrimidin-4-yl)-4-methyloxazolidin-2-one

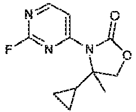

A cooled (0 °C) solution of 4-cyclopropyl-4-methyloxazolidin-2-one (3.40 g, 24.1 mmol) in DMF (50 ml) was treated with sodium hydride (1.45 g, 36.1 mmol, 60% in oil) and stirred for 10 min. 2,4-difluoropyrimidine (2.80 g, 24.1 mmol) was added and the resulting mixture was stirred for 1 h. Ice-bath was removed and the yellow solution was stirred for an additional 2 h at room temperature. The reaction mixture was diluted with EtOAc (100 ml) and washed with 4% aqueous NaCl solution (2 x 150 ml and 2 x 50 ml). The combined aqueous layers were extracted with EtOAc (100 ml). Combined organic layers were washed with brine (100 ml), dried over Na₂SO₄, filtered and concentrated. The residue was purified through silica gel column chromatography (Redi 80g, 0 - 40% EtOAc/heptane) to give 4-cyclopropyl-3-(2-fluoropyrimidin-4-yl)-4-methyloxazolidin-2-one (3.50 g, 14.7 mmol, 61.3%) ¹H NMR (400 MHz, MeOD) δ 8.54 (dd, *J* = 5.8, 2.5 Hz, 1H), 8.06 (dd, *J* = 5.8, 3.9 Hz, 1H), 4.03 (d, *J* = 0.8 Hz, 2H), 1.86 (tt, *J* = 8.5, 5.6 Hz, 1H), 1.72 (s, 3H), 0.76 - 0.58 (m, 2H), 0.58 - 0.37 (m, 2H). MS m/z 238.0 (M+H).

### Intermediate E: (S)-4-cyclopropyl-3-(2-fluoropyrimidin-4-yl)-4-methyloxazolidin-2-one

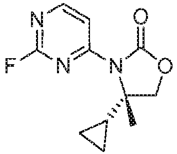

Intermediate D (3.5 g) was separated by by SFC (IC 20x250mm, 5% IPA in CO₂, 85g/min., 263UV) to give (S)-4-cyclopropyl-3-(2-fluoropyrimidin-4-yl)-4-methyloxazolidin-2-one (1.4 g) and (R)-4-cyclopropyl-3-(2-fluoropyrimidin-4-yl)-4-methyloxazolidin-2-one (1.3 g).
First eluted product: (S)-4-cyclopropyl-3-(2-fluoropyrimidin-4-yl)-4-methyloxazolidin-2-one ¹H NMR (400 MHz, CDCl₃) δ 8.47 (dd, *J* = 5.8, 2.3 Hz, 1H), 8.05 (dd, *J* = 5.8, 3.8 Hz, 1H), 3.90 (s, 2H), 1.85 (tt, *J* = 8.4, 5.6 Hz, 1H), 1.76 - 1.67 (m, 3H), 0.75 - 0.61 (m, 1H), 0.61 - 0.46 (m, 2H), 0.46 - 0.32 (m, 1H). Stereochemistry was assigned to the best of our knowledge by comparing NMR of several final compounds with those of compounds with known stereochemisty.

### Intermediate F: (S)-4-cyclopropyl-3-(2,6-dichloropyrimidin-4-yl)oxazolidin-2-one

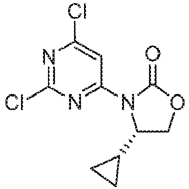

A solution of (S)-4-cyclopropyloxazolidin-2-one (0.200 g, 1.57 mmol) and 2,4,6-trichloropyrimidine (0.317 g, 1.73 mmol, 1.10 equiv) in DMF (6 mL) was treated with NaH (60 %, 0.075 g, 1.89 mmol, 1.20 equiv), then the resulting mixture (yellow) was stirred at room temperature for 1 h. The reaction mixture was diluted with EtOAc (20 mL), washed with saturated aqueous NaCl (2 x 20 mL), dried over Na₂SO₄, filtered and concentrated. Silica gel column chromatography (EtOAc/Heptane 0 to 40%) provided (S)-4-cyclopropyl-3-(2,6-dichloropyrimidin-4-yl)oxazolidin-2-one (0.267 g, white solid) in 62% yield. ¹H NMR (400 MHz, CDCl₃) δ 8.23 (s, 1H), 4.50 (t, J = 8.7 Hz, 1H), 4.39 (td, J = 8.7, 2.6 Hz, 1H), 4.31 (dd, J = 8.7, 2.6 Hz, 1H), 1.24 - 1.17 (m, 1H), 0.91 - 0.84 (m, 1H), 0.77 - 0.70 (m, 1H), 0.64 - 0.57 (m, 1H), 0.36 - 0.30 (m, 1H); MS *m*/*z* 274.0 (M + H)⁺; Rt-0.93 min.

### Intermediate G: (S)-3-(2-chloro-6-methylpyrimidin-4-yl)-4-cyclopropyloxazolidin-2-one Step 1: Preparation of (S)-2-amino-2-cyclopropylethanol hydrochloride

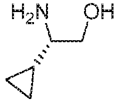

A solution of hydrochloric acid in 1,4-dioxane (4.0 M, 11.2 mL, 44.7 mmol, 3 equiv) was added dropwise to a stirring solution of (S)-tert-butyl (1-cyclopropyl-2-hydroxyethyl)carbamate (3.00 g, 14.9 mmol) in 1,4-dioxane (20 mL) at room temperature. A white precipitate formed and the slurry was stirred at room temperature for 1 hour. The mixture was then concentrated in vacuo to provide (S)-2-amino-2-cyclopropylethanol hydrochloride (2.7 g, white solid). No purification was required for the next step. ¹H NMR (400 MHz, CD₃OD) δ 3.84 (dd, J = 11.6, 3.7 Hz, 1H), 3.72 - 3.60 (m, 1H), 2.45 (ddd, J = 10.3, 7.0, 3.6 Hz, 1H), 1.00 (dtt, J = 10.1, 8.1, 8.1, 4.9, 4.9 Hz, 1H), 0.70 (m, 2H), 0.52 - 0.37 (m, 2H); MS *m*/*z* 102.0 (M + H)⁺; Rt-0.15 min.

### Step 2: Preparation of (S)-2-((2-chloro-6-methylpyrimidin-4-yl)amino)-2-cyclopropylethanol

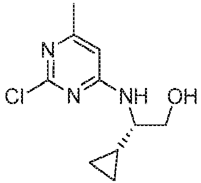

A solution of (S)-2-amino-2-cyclopropylethanol hydrochloride (1.5 g, 10.9 mmol), 2,4-dichloro-6-methylpyrimidine (2.1 g, 13.1 mmol, 1.2 equiv), and N-ethyl-N-isopropylpropan-2-amine (5.7 mL, 33 mmol, 3 equiv) in 1,4-dioxane (55 mL) was heated at 75 °C for 15 h. The reaction was cooled to room temperature and concentrated in vacuo. Silica gel column chromatography (EtOAc/Heptane) provided (S)-2-((2-chloro-6-methylpyrimidin-4-yl)amino)-2-cyclopropylethanol as a light yellow solid in 18% yield. ¹H NMR (400 MHz, CDCl₃) δ 6.14 (s, 1H), 5.24 (br s, 1H), 3.90 (m, 1H), 3.76 (m, 1H), 3.32 (br m, 1H), 2.32 (s, 3H), 1.00 (m, 1H), 0.59 (m, 2H), 0.40 (m, 2H); MS *m*/*z* 228.1 (M + H)⁺; Rt-0.42 min.

### Step 3: Preparation of (S)-3-(2-chloro-6-methylpyrimidin-4-yl)-4-cyclopropyloxazolidin-2-one

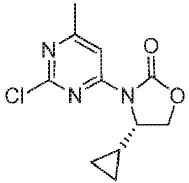

Triphosgene (235 mg, 0.791 mmol, 0.4 equiv) was added to a solution of (S)-2-((2-chloro-6-methylpyrimidin-4-yl)amino)-2-cyclopropylethanol (450 mg, 1.98 mmol) in DCM (20 mL) at - 78 °C, followed by the dropwise addition of 2,6-lutidine (0.92 mL, 7.9 mmol, 4 equiv). The solution was allowed to warm to room temperature and was then heated at 35 °C for 5 hours. The reaction was then cooled to room temperature and diluted with DCM (30 mL) and saturated aqueous sodium chloride (30 mL). The layers were separated and the organic layer was dried over Na₂SO₄, filtered and concentrated. Silica gel column chromatography (EtOAc/Heptane) provided (S)-3-(2-chloro-6-methylpyrimidin-4-yl)-4-cyclopropyloxazolidin-2-one (0.279 g, light yellow oil) in 84% yield. ¹H NMR (400 MHz, CDCl₃) δ 8.01 (s, 1H), 4.51 - 4.39 (m, 2H), 4.28 (dd, J = 8.0, 2.0 Hz, 1H), 2.53 (s, 3H), 1.21 (tdd, J = 8.2, 8.2, 4.9, 3.1 Hz, 1H), 0.86 (m, 1H), 0.70 (m, 1H), 0.56 (m, 1H), 0.32 (dq, J = 10.7, 5.0 Hz, 1H). MS *m*/*z* 254.0 (M + H)⁺; Rt-0.81 min.

### Intermediate H: (S)-4-cyclopropyl-3-(2-fluoro-6-methylpyrimidin-4-yl)oxazolidin-2-one

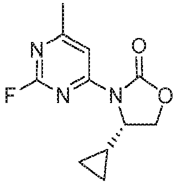

Potassium fluoride (1.26 g, 21.7 mmol, 20 equiv) was added to a solution of (S)-3-(2-chloro-6-methylpyrimidin-4-yl)-4-cyclopropyloxazolidin-2-one (275 mg, 1.08 mmol) in DMSO (11 mL). The suspension was heated at 110 °C for 5 hours and then cooled to room temperature. The reaction was diluted with ethyl acetate (50 mL) and dilute aqueous sodium chloride (100 mL). The layers were separated and the aqueous layer was extracted with ethyl acetate (30 mL). The combined organic layers were washed with saturated aqueous sodium chloride (30 mL), dried over Na₂SO₄, filtered and concentrated to give (S)-4-cyclopropyl-3-(2-fluoro-6-methylpyrimidin-4-yl)oxazolidin-2-one (230 mg, colorless oil) in 89% yield. ¹H NMR (400 MHz, CDCl₃) δ 7.99 (d, J = 3.8 Hz, 1H), 4.50 - 4.40 (m, 2H), 4.25 (m, 1H), 2.54 (s, 3H), 1.24 (m, 1H), 0.82 (m, 1H), 0.59 (m, 1H), 0.57 (m, 1H), 0.33 (m, 1H). MS *m*/*z* 238.1 (M + H)⁺; Rt-0.75 min.

### Intermediate 1: 4-chloro-N'-hydroxybenzimidamide

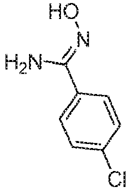

A solution of 4-chlorobenzonitrile (1.00 g, 7.27 mmol), hydroxylamine hydrochloride (0.758 g, 10.9 mmol, 1.5 equiv), and N-ethyl-N-isopropylpropan-2-amine (2.0 mL, 11.6 mmol, 1.6 equiv) in absolute ethanol (7.3 mL) was heated at reflux for 2 hours. The reaction was then cooled to room temperature and concentrated in vacuo. The residue was dissolved in ethyl acetate (100 mL) and washed sequentially with water (2 x 75 mL), saturated aqueous sodium chloride (50 mL), dried over anhydrous magnesium sulfate, filtered and concentrated in vacuo to give 4-chloro-N'-hydroxybenzimidamide (1.2 g, white solid) in 97% yield. The material was used without further purification. MS *m*/*z* 171.0 (M + H)⁺; Rt-0.35 min.

### Intermediate 2: (S)-tert-butyl 1-(3-(4-chlorophenyl)-1,2,4-oxadiazol-5-yl)ethylcarbamate

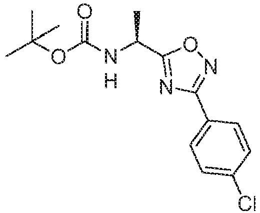

A solution of 4-chloro-N'-hydroxybenzimidamide (1.24 g, 7.27 mmol), (S)-2-(tert-butoxycarbonylamino)propanoic acid (1.38 g, 7.27 mmol, 1.0 equiv), and DCC (1.65 g, 8.00 mmol, 1.1 equiv) in 1,4-dioxane (73 mL) was heated at 100 °C for 18 hours. The reaction was then cooled to room temperature and concentrated in vacuo. Silica gel column chromatography (EtOAc/Heptane, 0 to 35%) provided (S)-tert-butyl 1-(3-(4-chlorophenyl)-1,2,4-oxadiazol-5-yl)ethylcarbamate (1.13 g, white solid) in 48% yield. ¹H NMR (400 MHz, CDCl₃) δ 8.03 (d, J = 8.8 Hz, 2H), 7.47 (d, J = 8.8 Hz, 2H), 5.18 (m, 1H), 1.64 (d, J = 6.8 Hz, 3H), 1.47 (s, 9H).

### Intermediate 3: (S)-1-(3-(4-chlorophenyl)-1,2,4-oxadiazol-5-yl)ethanamine

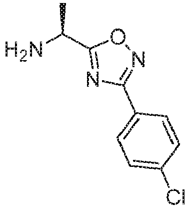

2,2,2-Trifluoroacetic acid (4 mL, 52 mmol) was added to a solution of (S)-tert-butyl 1-(3-(4-chlorophenyl)-1,2,4-oxadiazol-5-yl)ethylcarbamate (0.613 g, 1.89 mmol) in DCM (10 mL) at room temperature. The solution was stirred at room temperature for 1 hour and then concentrated in vacuo. The residue was dissolved in chloroform (100 mL) and washed with saturated aqueous sodium bicarbonate (100 mL). The layers were separated and the aqueous layer was extracted with chloroform (3 x 30 mL) and the combined organic layers were dried over anhydrous sodium sulfate, filtered and concentrated in vacuo to give (S)-1-(3-(4-chlorophenyl)-1,2,4-oxadiazol-5-yl)ethanamine (500 mg, yellow oil). The material was used without further purification. ¹H NMR (400 MHz, CDCl₃) δ 8.04 (d, J = 8.7 Hz, 2H), 7.47 (d, J = 8.6 Hz, 2H), 4.37 (q, J = 6.9 Hz, 1H), 1.62 (d, J = 6.9 Hz, 3H). MS *m*/*z* 224.0 (M + H)⁺; Rt-0.56 min.

### Intermediate 4: (S)-tert-butyl (1-hydrazinyl-1-oxopropan-2-yl)carbamate

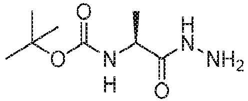

A solution of (S)-methyl 2-((tert-butoxycarbonyl)amino)propanoate (1.00 g, 4.92 mmol) and hydrazine (0.23 mL, 1.5 equiv) in THF (8 mL) was heated in a sealed tube at 72 °C for 15 hours. Additional hydrazine (0.23 mL, 1.5 equiv) was added and heating was continued for another 21 hours. The reaction was then cooled to room temperature and concentrated in vacuo to give crude (S)-tert-butyl (1-hydrazinyl-1-oxopropan-2-yl)carbamate (1 g, white solid), which was used without purification. ¹H NMR (400 MHz, CDCl₃) δ 4.20 (m, 1H), 1.44 (s, 9H), 1.36 (d, J = 7.1 Hz, 3H).

### Intermediate 5: (S)-tert-butyl (1-(2-(4-chlorobenzoyl)hydrazinyl)-1-oxopropan-2-yl)carbamate

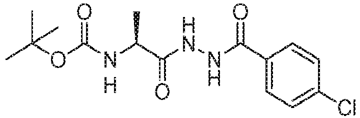

4-Chlorobenzoyl chloride (0.63 mL, 4.92 mmol, 1.0 equiv) was added to a solution of (S)-tert-butyl (1-hydrazinyl-1-oxopropan-2-yl)carbamate (1.0 g, 4.92 mmol) in DCM (25 mL) at 0 °C. A white precipitate formed. The mixture was stirred at 0 °C for 1 hour and the reaction mixture was then concentrated in vacuo to give crude (S)-tert-butyl (1-(2-(4-chlorobenzoyl)hydrazinyl)-1-oxopropan-2-yl)carbamate (1.55 g), which was used without purification. ¹H NMR (400 MHz, CD₃OD) δ 7.85 (d, J = 8.7 Hz, 2H), 7.49 (d, J = 8.7 Hz, 2H), 4.21 (q, J = 7.0 Hz, 1H), 1.45 (s, 9H), 1.41 (d, J = 7.2 Hz, 3H). MS *m*/*z* 342.1 (M + H)⁺; Rt-0.69 min.

### Intermediate 6: (S)-tert-butyl (1-(5-(4-chlorophenyl)-1,3,4-thiadiazol-2-yl)ethyl)carbamate

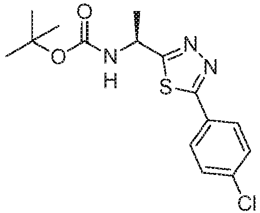

A solution of (S)-tert-butyl (1-(2-(4-chlorobenzoyl)hydrazinyl)-1-oxopropan-2-yl)carbamate (1.0 g, 2.93 mmol) and 2,4-bis(4-methoxyphenyl)-1,3,2,4-dithiadiphosphetane 2,4-disulfide (1.18 g, 2.93 mmol, 1.0 equiv) in THF (29 mL) was heated at reflux for 2 hours. The reaction wash then cooled to room temperature and filtered through a pad of celite, using THF to wash through. The filtrate was concentrated in vacuo. Silica gel column chromatography (EtOAc/Heptane, 0 to 30%) provided (S)-tert-butyl (1-(5-(4-chlorophenyl)-1,3,4-thiadiazol-2-yl)ethyl)carbamate (0.600 g, light green solid) in 60% yield. ¹H NMR (400 MHz, CDCl₃) δ 7.89 (d, J = 8.5 Hz, 2H), 7.46 (d, J = 8.5 Hz, 2H), 5.23 (m, 1H), 1.72 (d, J = 6.5 Hz, 3H), 1.48 (s, 9H). MS *m*/*z* 340.1 (M + H)⁺; Rt-0.99 min.

### Intermediate 7: (S)-1-(5-(4-chlorophenyl)-1,3,4-thiadiazol-2-yl)ethanamine

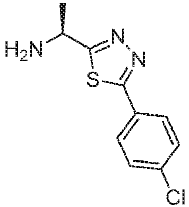

A solution of hydrogen chloride (4.0 M in 1,4-dioxane, 4 mL, 16 mmol, 9 equiv) was added to a solution of (S)-tert-butyl (1-(5-(4-chlorophenyl)-1,3,4-thiadiazol-2-yl)ethyl)carbamate (600 mg, 1.77 mmol) in 1,4-dioxane (5 mL) at room temperature. The solution was stirred for 3 hours, by which time a white precipitate had formed. The reaction was concentrated in vacuo to give the hydrochloride salt of (S)-1-(5-(4-chlorophenyl)-1,3,4-thiadiazol-2-yl)ethanamine (480 mg, white solid) in 97% yield. The material was used without purification. ¹H NMR (400 MHz, CD₃OD) δ 8.00 (d, J = 8.7 Hz, 2H), 7.58 (d, J = 8.7 Hz, 2H), 5.10 (q, J = 6.9 Hz, 1H), 1.82 (d, J = 6.9 Hz, 3H). MS *m*/*z* 240.0 (M + H)⁺; Rt-0.54 min.

### Intermediate 8: (S)-tert-butyl but-3-yn-2-ylcarbamate

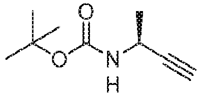

A solution of (S)-tert-butyl (1-oxopropan-2-yl)carbamate (500 mg, 2.89 mmol), dimethyl (1-diazo-2-oxopropyl)phosphonate (610 mg, 3.18 mmol, 1.1 equiv), and potassium carbonate (638 mg, 4.62 mmol, 1.6 equiv) in methanol (14.4 mL) was stirred at room temperature for 18 hours. The reaction was then diluted with ethyl acetate (30 mL) and saturated aqueous sodium chloride (40 mL). The layers were separated and the aqueous layer was extracted with ethyl acetate (30 mL) and the combined organic extracts were dried over Na₂SO₄, filtered and concentrated. Silica gel column chromatography (20% EtOAc in Heptane) provided (S)-tert-butyl but-3-yn-2-ylcarbamate (0.258 g, white solid) in 53% yield. ¹H NMR (400 MHz, CDCl₃) δ 4.49 (m, 1H), 2.26 (d, J = 2.2 Hz, 1H), 1.46 (s, 9H), 1.41 (d, J = 6.8 Hz, 3H).

### Intermediate 9: (S)-tert-butyl (1-(1-(4-chlorophenyl)-1H-1,2,3-triazol-4-yl)ethyl)carbamate

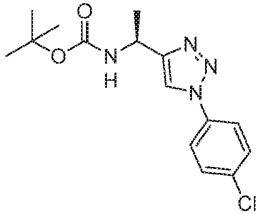

A solution of (S)-tert-butyl but-3-yn-2-ylcarbamate (250 mg, 1.48 mmol), 1-azido-4-chlorobenzene (227 mg, 1.48 mmol, 1.0 equiv), and N-ethyl-N-isopropylpropan-2-amine (0.77 mL, 4.43 mmol, 3.0 equiv) in anhydrous acetonitrile (14.8 mL) was stirred at room temperature for 10 min. Copper(I) iodide (563 mg, 2.95 mmol, 2.0 equiv) was then added in portions. The mixture was stirred at room temperature for 30 min. The reaction was quenched with saturated aqueous ammonium chloride (50 mL) and diluted with water (50 mL). The mixture was extracted with ethyl acetate (3 x 30 mL) and the combined organic extracts were washed with water (30 mL), saturated aqueous sodium chloride (30 mL), dried over Na₂SO₄, filtered and concentrated. Silica gel column chromatography (EtOAc /Heptane) provided (S)-tert-butyl (1-(1-(4-chlorophenyl)-1H-1,2,3-triazol-4-yl)ethyl)carbamate (0.428 g, white solid) in 90% yield. ¹H NMR (400 MHz, CDCl₃) δ 7.86 (s, 1H), 7.68 (d, J = 8.8 Hz, 2H), 7.50 (d, J = 8.8 Hz, 2H), 5.02 (m, 1H), 1.63 (d, J = 6.8 Hz, 3H), 1.46 (s, 9H). MS *m*/*z* 323.1 (M + H)⁺; Rt-0.92 min.

### Intermediate 10: (S)-1-(1-(4-chlorophenyl)-1H-1,2,3-triazol-4-yl)ethanamine

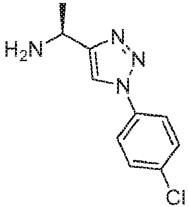

A solution of hydrogen chloride (4.0 M in 1,4-dioxane, 3.3 mL, 13.2 mmol, 10 equiv) was added to a solution of (S)-tert-butyl (1-(1-(4-chlorophenyl)-1H-1,2,3-triazol-4-yl)ethyl)carbamate (425 mg, 1.32 mmol) in 1,4-dioxane (5 mL) at room temperature. The solution was stirred for 1 hour, by which time a white precipitate had formed. The reaction was concentrated in vacuo to give the hydrochloride salt of (S)-1-(1-(4-chlorophenyl)-1H-1,2,3-triazol-4-yl)ethanamine (338 mg, white solid) in 99% yield. The material was used without purification. ¹H NMR (400 MHz, CD₃OD) δ 8.68 (s, 1H), 7.90 (d, J = 9.0 Hz, 2H), 7.63 (d, J = 9.0 Hz, 2H), 4.77 (q, J = 6.9 Hz, 1H), 1.78 (d, J = 6.9 Hz, 3H). MS *m*/*z* 223.1 (M + H)⁺; Rt-0.50 min.

### Intermediate 11: 2-(6-methylpyridin-3-yl)thiazole-5-carbaldehyde

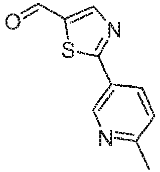

A mixture of 2-bromothiazole-5-carbaldehyde (400 mg, 2.08 mmol), (6-methylpyridin-3-yl)boronic acid (428 mg, 3.12 mmol, 1.5 equiv), [1,1'-bis(diphenylphosphino)ferrocene]dichloropalladium(II), complex with dichloromethane (170 mg, 0.21 mmol, 0.1 equiv) and 2.0 M aqueous sodium carbonate (5.2 mL, 10.4 mmol, 5 equiv) in 1,2-dimethoxyethane (6.9 mL) was heated in a microwave reactor at 110 °C for 20 minutes. The reaction was then diluted with ethyl acetate (50 mL) and water (50 mL). The layers were separated and the aqueous layer was extracted with ethyl acetate (2 x 25 mL). The combined organic extracts were washed with saturated aqueous sodium chloride (30 mL), dried over Na₂SO₄, filtered and concentrated. Silica gel column chromatography (EtOAc) provided 2-(6-methylpyridin-3-yl)thiazole-5-carbaldehyde (0.176 g, brown solid) in 41% yield. ¹H NMR (400 MHz, CDCl₃) δ 10.08 (s, 1H), 9.15 (d, J = 2.0 Hz, 1H), 8.47 (s, 1H), 8.19 (dd, J = 8.1, 2.4 Hz, 1H), 7.31 (d, J = 8.3 Hz, 1H), 2.66 (s, 3H). MS *m*/*z* 205.0 (M + H)⁺; Rt-0.36 min.

The intermediates in **Table 1** were prepared using a method similar to that described for the preparation of **Intermediate 11**

**Table 1.**

| | | |
|---|---|---|
| Intermediate 11 | Intermediate 12 | Intermediate 13 |
| 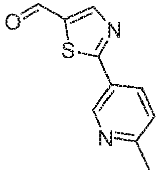 | 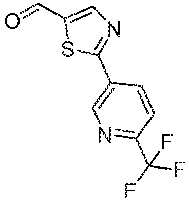 | 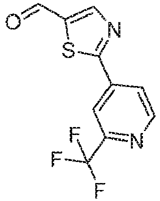 |

**Table 2. Chemical name, NMR chemical shifts and LCMS signal for each intermediate listed in Table 1.**

| **Intermediate: Name** | **¹H NMR (400 MHz, CDCl₃) δ ppm** | **LCMS** |
|---|---|---|
| 11: 2-(6-methylpyridin-3-yl)thiazole-5-carbaldehyde | 10.08 (s, 1H), 9.15 (d, J = 2.0 Hz, 1H), 8.47 (s, 1H), 8.19 (dd, J = 8.1, 2.4 Hz, 1H), 7.31 (d, J = 8.3 Hz, 1H), 2.66 (s, 3H). | MS *m*/*z* 205.0 (M + H)⁺; Rt-0.36 min |
| 12: 2-(6-(trifluoromethyl)pyridin-3-yl)thiazole-5-carbaldehyde | 10.13 (s, 1H), 9.37 (d, J = 1.6 Hz, 1H), 8.55 (s, 1H), 8.50 (dd, J = 8.2, 2.0 Hz, 1H), 7.85 (d, J = 8.2 Hz, 1H) | MS *m*/*z* 259.0 (M + H)⁺; Rt-0.74 min |
| 13: 2-(2-(trifluoromethyl)pyridin-4-yl)thiazole-5-carbaldehyde | 10.14 (s, 1H), 8.91 (d, J = 5.0 Hz, 1H), 8.57 (d, J = 0.6 Hz, 1H), 8.30 (s, 1H), 8.06 (d, J = 5.1 Hz, 1H) | MS *m*/*z* 259.0 (M + H)⁺; Rt-0.76 min |

### Intermediate 14: 1-(4-chlorophenyl)-4-(1,3-dioxolan-2-yl)-1H-imidazole

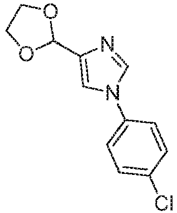

A solution of ethane-1,2-diol (0.081 mL, 1.45 mmol, 1.5 equiv), 1-(4-chlorophenyl)-1H-imidazole-4-carbaldehyde (200 mg, 0.968 mmol), and camphorsulfonic acid (45 mg, 0.19 mmol, 0.2 equiv) in toluene (10 mL) was heated at reflux with a Dean-Stark apparatus for 1 hour. The reaction was cooled to room temperature and quenched with saturated aqueous sodium bicarbonate (30 mL). The mixture was extracted with ethyl acetate (30 mL) and the organic layer was dried (Na₂SO₄), filtered, and concentrated in vacuo. Silica gel column chromatography (EtOAc with 7% methanol) provided 1-(4-chlorophenyl)-4-(1,3-dioxolan-2-yl)-1H-imidazole (0.100 g, tan solid) in 41% yield. ¹H NMR (400 MHz, CDCl₃) δ 7.80 (s, 1H), 7.46 (d, J = 8.4 Hz, 2H), 7.36 (s, 1H), 7.33 (d, J = 8.4 Hz, 2H), 5.96 (s, 1H), 4.20 (m, 2H), 4.05 (m, 2H). MS *m*/*z* 251.0 (M + H)⁺; Rt-0.53 min.

### Intermediate 15: 1-(6-(trifluoromethyl)pyridin-3-yl)-1H-pyrazole-3-carbaldehyde

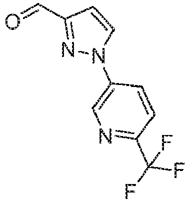

A mixture of 1H-pyrazole-3-carbaldehyde (0.700 g, 7.29 mmol), 5-bromo-2-(trifluoromethyl)pyridine (2.31 g, 10.2 mmol, 1.4 equiv), cesium carbonate (4.75 g, 14.6 mmol, 2.0 equiv), copper(I) iodide (0.069 g, 0.36 mmol, 0.05 equiv), and (1R,2R)-N1,N2-dimethylcyclohexane-1,2-diamine (0.23 mL, 1.46 mmol, 0.2 equiv) in DMF (9.5 mL) was heated in a sealed reaction vessel at 110 °C for 16 hours. The reaction was then cooled to room temperature and saturated aqueous ammonium chloride (100 mL) was added. The mixture was extracted with ethyl acetate (3 x 50 mL). The combined organic extracts were washed with saturated aqueous sodium chloride (50 mL), dried (Na₂SO₄), filtered, and concentrated in vacuo. Silica gel column chromatography (EtOAc/heptane) provided 1-(6-(trifluoromethyl)pyridin-3-yl)-1H-pyrazole-3-carbaldehyde (0.470 g, brown solid) in 27% yield. ¹H NMR (400 MHz, CDCl₃) δ 10.13 (s, 1H), 9.18 (d, J = 2.1 Hz, 1H), 8.34 (dd, J = 8.7, 2.0 Hz, 1H), 8.12 (m, 1H), 7.88 (d, J = 8.5 Hz, 1H), 7.10 (d, J = 1.8 Hz, 1H). MS *m*/*z* 241.9 (M + H)⁺; Rt-0.78 min.

### Intermediate 16: (R,E)-N-((5-(4-chlorophenyl)isoxazol-3-yl)methylene)-2-methylpropane-2-sulfinamide

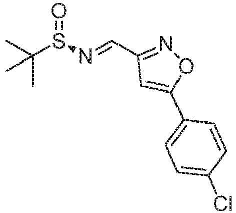

A suspension of 5-(4-chlorophenyl)isoxazole-3-carbaldehyde (2.00 g, 9.63 mmol), (R)-2-methylpropane-2-sulfinamide (1.28 g, 10.6 mmol, 1.1 equiv) and anhydrous copper(II) sulfate (2.31 g, 14.5 mmol, 1.5 equiv) in 1,2-dichloroethane (19 mL) was heated at 55 °C for 2 - 18 hours. The reaction was then cooled to room temperature and filtered through a pad of celite, using 1,2-dichloroethane to wash through. The filtrate was concentrated in vacuo to give crude (R,E)-N-((5-(4-chlorophenyl)isoxazol-3-yl)methylene)-2-methylpropane-2-sulfinamide as a green solid, which was used without further purification. MS *m*/*z* 311.0 (M + H)⁺; Rt-1.11 min. The intermediates in **Table 3** were prepared using a method similar to that described for the preparation of **Intermediate 16**

**Table 3.**

| | | |
|---|---|---|
| Intermediate 16 | Intermediate 17 | Intermediate 18 |
| 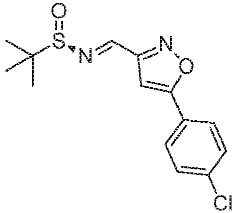 | 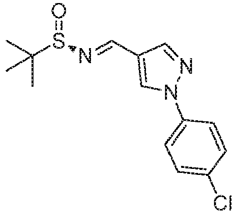 | 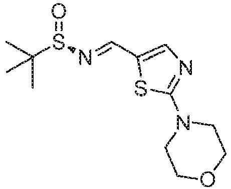 |
| Intermediate 19 | Intermediate 20 | Intermediate 21 |
| 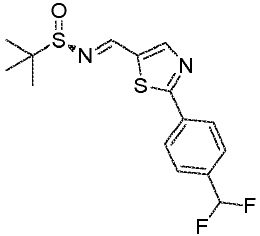 | 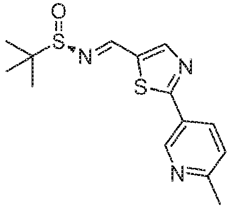 | 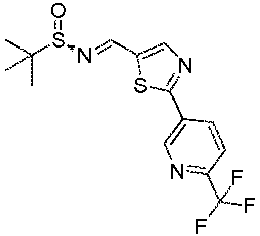 |
| Intermediate 22 | Intermediate 23 | |
| 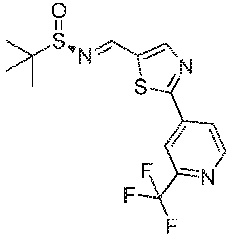 | 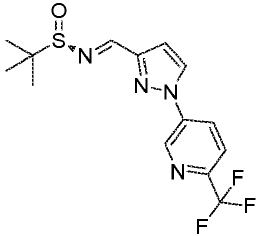 | |

**Table 4. Chemical name and analytical data for each intermediate listed in Table 3.**

| **Intermediate: Name** | **Analytical data** |
|---|---|
| 16: (R,E)-N-((5-(4-chlorophenyl)isoxazol-3-yl)methylene)-2-methylpropane-2-sulfinamide | MS *m*/*z* 311.0 (M + H)⁺; Rt-1.11 min |
| 17: (R,E)-N-((1-(4-chlorophenyl)-1H-pyrazol-4-yl)methylene)-2-methylpropane-2-sulfinamide | MS m/z 310.1 (M + H)⁺; Rt-1.00 min |
| 18: (R,E)-2-methyl-N-((2-morpholinothiazol-5-yl)methylene)propane-2-sulfinamide | MS m/z 302.1 (M + H)⁺; Rt-0.69 min |
| 19: (R,E)-N-((2-(4-(difluoromethyl)phenyl)thiazol-5-yl)methylene)-2-methylpropane-2-sulfinamide | MS m/z 342.9 (M + H)⁺; Rt-0.86 min |
| 20: (R,E)-2-methyl-N-((2-(6-methylpyridin-3-yl)thiazol-5-yl)methylene)propane-2-sulfinamide | MS m/z 308.1 (M + H)⁺; Rt-0.58 min |
| 21: (R,E)-2-methyl-N-((2-(6-(trifluoromethyl)pyridin-3-yl)thiazol-5-yl)methylene)propane-2-sulfinamide | MS m/z 362.1 (M + H)⁺; Rt-0.96 min |
| 22: (R,E)-2-methyl-N-((2-(2-(trifluoromethyl)pyridin-4-yl)thiazol-5-yl)methylene)propane-2-sulfinamide | MS m/z 362.1 (M + H)⁺; Rt-0.97 min |
| 23: (R,E)-2-methyl-N-((1-(6-(trifluoromethyl)pyridin-3-yl)-1H-pyrazol-3-yl)methylene)propane-2-sulfinamide | MS m/z 345.0 (M + H)⁺; Rt-0.95 min |

### Intermediate 24: (R)-N-((S)-1-(5-(4-chlorophenyl)isoxazol-3-yl)ethyl)-2-methylpropane-2-sulfinamide

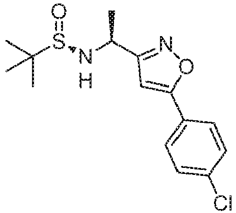

A solution of methylmagnesium bromide (3.0 M in diethyl ether, 12.8 mL, 38.4 mmol, 4 equiv) was added to a solution of (R,E)-N-((5-(4-chlorophenyl)isoxazol-3-yl)methylene)-2-methylpropane-2-sulfinamide (2.98 g, 9.6 mmol) in DCM (96 mL) at 0 °C. The solution became orange, then faded to yellow. The reaction was stirred at 0 °C for 30 min and then carefully quenched with saturated aqueous ammonium chloride (100 mL). The layers were separated and the aqueous layer was extracted with DCM (40 mL). The combined organic layers were washed with water (50 mL), saturated aqueous sodium chloride (50 mL), dried over Na₂SO₄, filtered and concentrated. Silica gel column chromatography (EtOAc/Heptane) provided (R)-N-((S)-1-(5-(4-chlorophenyl)isoxazol-3-yl)ethyl)-2-methylpropane-2-sulfinamide (1.0 g, white solid) in 32% yield. ¹H NMR (400 MHz, CDCl₃) δ 7.71 (d, J = 8.6 Hz, 2H), 7.45 (d, J = 8.6 Hz, 2H), 6.50 (s, 1H), 4.75 (m, 1H), 3.47 (m, 1H), 1.70 (d, J = 6.8 Hz, 3H), 1.25 (s, 9H). MS *m*/*z* 327.0 (M + H)⁺; Rt-0.94 min.

The intermediates in **Table 5** were prepared using a method similar to that described for the preparation of **Intermediate 24.**

**Table 5.**

| | | |
|---|---|---|
| Intermediate 24 | Intermediate 25 | Intermediate 26 |
| 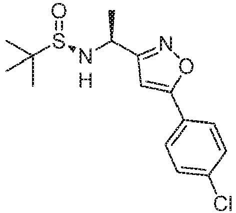 | 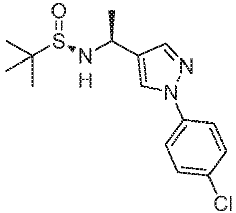 | 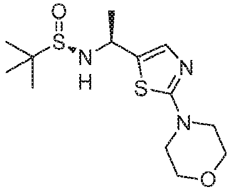 |
| Intermediate 27 | Intermediate 28 | Intermediate 29 |
| 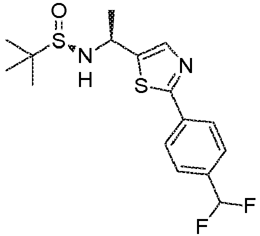 | 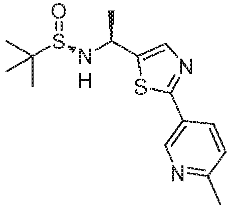 | 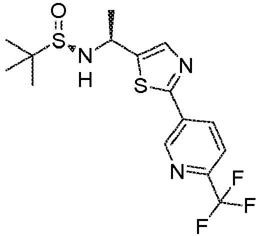 |
| Intermediate 30 | Intermediate 31 | |
| 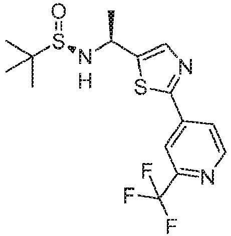 | 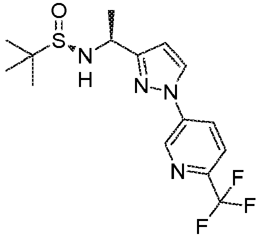 | |

**Table 6. Chemical name, NMR chemical shifts and LCMS signal for each intermediate listed in Table 5.**

| **Intermediate: Name** | **¹H NMR (400 MHz, CDCl₃) δ ppm** | **LCMS** |
|---|---|---|
| 24: (R)-N-((S)-1-(5-(4-chlorophenyl)isoxazol-3-yl)ethyl)-2-methylpropane-2-sulfinamide | 7.71 (d, J = 8.6 Hz, 2H), 7.45 (d, J = 8.6 Hz, 2H), 6.50 (s, 1H), 4.75 (m, 1H), 3.47 (m, 1H), 1.70 (d, J = 6.8 Hz, 3H), 1.25 (s, | MS *m*/*z* 327.0 (M + H)⁺; Rt-0.94 |
| | 9H). | min |
| 25: (R)-N-((S)-1-(1-(4-chlorophenyl)-1H-pyrazol-4-yl)ethyl)-2-methylpropane-2-sulfinamide | 7.84 (s, 1H), 7.63 (s, 1H), 7.61 (d, J = 8.7 Hz, 2H), 7.42 (d, J = 8.7 Hz, 2H), 4.64 (m, 1H), 3.31 (m, 1H), 1.62 (d, J = 6.7 Hz, 3H), 1.24 (s, 9H) | MS m/z 326.1 (M + H)⁺; Rt-0.89 min |
| 26: (R)-2-methyl-N-((S)-1-(2-morpholinothiazol-5-yl)ethyl)propane-2-sulfinamide | 7.06 (s, 1H), 4.69 (quin, J = 6.1 Hz, 1H), 3.81 (m, 4H), 3.44 (m, 4H), 3.32 (d, J = 4.9 Hz, 1H), 1.60 (d, J = 6.6 Hz, 3H), 1.22 (s, 9H) | |
| 27: (R)-N-((S)-1-(2-(4-(difluoromethyl)phenyl)thiazol-5-yl)ethyl)-2-methylpropane-2-sulfinamide | 8.01 (d, J = 8.0 Hz, 2H), 7.74 (s, 1H), 7.59 (d, J = 8.0 Hz, 2H), 6.69 (t, J = 56 Hz, 1H), 4.91 (m, 1H), 3.45 (d, J = 4.7 Hz, 1H), 1.72 (d, J = 6.6 Hz, 3H), 1.25 (s, 9H) | MS m/z 359.1 (M + H)⁺; Rt-0.89 min |
| 28: (R)-2-methyl-N-((S)-1-(2-(6-methylpyridin-3-yl)thiazol-5-yl)ethyl)propane-2-sulfinamide | 9.01 (d, J = 2.2 Hz, 1H), 8.10 (dd, J = 8.1, 2.3 Hz, 1H), 7.73 (s, 1H), 7.24 (d, J = 8.2 Hz, 1H), 4.91 (m, 1H), 3.46 (d, J = 4.8 Hz, 1H), 2.62 (s, 3H), 1.72 (d, J = 6.6 Hz, 3H), 1.25 (s, 9H) | MS m/z 324.1 (M + H)⁺; Rt-0.49 min |
| 29: (R)-2-methyl-N-((S)-1-(2-(6-(trifluoromethyl)pyridin-3-yl)thiazol-5-yl)ethyl)propane-2-sulfinamide | 9.23 (s, 1H), 8.40 (d, J = 8.3 Hz, 1H), 7.82 (s, 1H), 7.78 (d, J = 8.3 Hz, 1H), 4.95 (m, 1H), 3.49 (d, J = 4.1 Hz, 1H), 1.74 (d, J = 6.6 Hz, 3H), 1.26 (s, 9H) | MS m/z 378.1 (M + H)⁺; Rt-0.84 min |
| 30: (R)-2-methyl-N-((S)-1-(2-(2-(trifluoromethyl)pyridin-4-yl)thiazol-5-yl)ethyl)propane-2-sulfinamide | 8.82 (d, J = 5.0 Hz, 1H), 8.19 (s, 1H), 7.97 (dd, J = 5.0, 1.6 Hz, 1H), 7.85 (s, 1H), 4.95 (m, 1H), 3.49 (d, J = 4.5 Hz, 1H), 1.74 (d, J = 6.6 Hz, 3H), 1.26 (s, 9H) | MS m/z 378.1 (M + H)⁺; Rt-0.85 min |
| 31: (R)-2-methyl-N-((S)-1-(1-(6-(trifluoromethyl)pyridin-3-yl)-1H-pyrazol-3-yl)ethyl)propane-2-sulfinamide | 9.06 (d, J = 2.3 Hz, 1H), 8.22 (dd, J = 8.6, 1.9 Hz, 1H), 7.98 (dd, J = 2.6, 0.9 Hz, 1H), 7.78 (d, J = 8.6 Hz, 1H), 6.51 (dd, J = 2.5, 0.9 Hz, 1H), 4.76 (m, 1H), 3.48 (d, J = 4.1 Hz, 1H), 1.68 (d, J = 6.8 Hz, 3H), 1.24 (s, 9H) | MS m/z 361.1 (M + H)⁺; Rt-0.85 min |

### Intermediate 32: (R)-N-((S)-1-(2-fluoro-4-(1-methyl-1H-pyrazol-4-yl)phenyl)ethyl)-2-methylpropane-2-sulfinamide

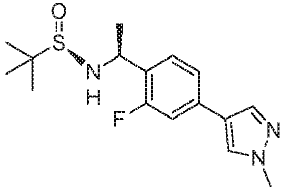

To a two microwave vials with stir bars were added (R)-N-((S)-1-(4-bromo-2-fluorophenyl)ethyl)-2-methylpropane-2-sulfinamide (1.5 g, 4.65 mmol), 1-methyl-4-1H-pyrazoleboronic acid pinacol ester (2.91 g, 13.9 mmol), DME (20 mL), sodium carbonate (11.6 mL, 23.3 mmol, 2.0 M aq) and PdCl₂(dppf).CH₂Cl₂ adduct (190 mg, 0.23 mmol) divided between the two vials. The vials were capped and heated by microwave irradiation for 20 min at 100 °C respectively. The reaction mixtures combined, diluted with a saturated solution of NH₄Cl and EtOAc. The phases were partitioned and the aqueous phase extracted with EtOAc. Organic phases combined, washed with water, brine, dried (Na₂SO₄), filtered and concentrated onto silica gel. Silica gel column chromatography (EtOAc/Heptane 40 to 100%) provided a orange crystalline of (R)-N-((S)-1-(2-fluoro-4-(1-methyl-1H-pyrazol-4-yl)phenyl)ethyl)-2-methylpropane-2-sulfinamide (1.07 g, 3.31 mmol, 71 % yield. ¹H NMR (400 MHz, CDCL₃) δ ppm 1.21 (s, 9 H) 1.60 (d, *J*=6.80 Hz, 3 H) 3.36 (d, *J*=4.25 Hz, 1 H) 3.96 (s, 3 H) 4.79 - 4.91 (m, 1 H) 7.13 (dd, *J*=11.69, 1.61 Hz, 1 H) 7.23 (dd, *J*=8.00, 1.64 Hz, 1 H) 7.30-7.37 (m, 1 H) 7.60 (s, 1 H) 7.74 (s, 1 H). LCMS *m*/*z* 324.0 (M + H)⁺, Rt 0.74 min.

The Intermediates in **Table 7** were prepared by a method similar to the one described for the preparation of **intermediate 32.**

**Table 7.**

| | |
|---|---|
| Intermediate 33 | Intermediate 34 |
| 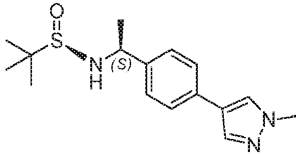 | 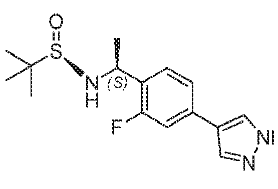 |

**Table 8. Chemical name, NMR chemical shifts and LCMS signal for each intermediate listed in Table 7.**

| Intermediate: Name | ¹H NMR (400 MHz) δ ppm | LCMS |
|---|---|---|
| 33: (R)-2-methyl-N-((S)-1-(4-(1-methyl-1H-pyrazol-4-yl)phenyl)ethyl)propane-2-sulfinamide | | MS *m*/*z* 306.0 (M + H)⁺, Rt 0.71 min. |
| 34: (R)-N-((S)-1-(2-fluoro-4-(1H-pyrazol-4-yl)phenyl)ethyl)-2-methylpropane-2-sulfinamide | (DMSO) 1.10 (s, 9 H) 1.47 (d, *J*=6.75 Hz, 3 H) 4.60 - 4.70 (m, 1 H) 5.41 (d, *J*=5.48 Hz, 1 H) 7.38 - 7.44 (m, 3 H) 7.96 (br. s., 1 H) 8.23 (br. s., 1 H) 12.97 (br. s., 1 H) | MS *m*/*z* 310.0 (M + H)⁺, Rt 0.67 min. |

### Intermediate 35: (R)-N-((S)-1-(2,5-difluoro-4-(1-methyl-1H-pyrazol-4-yl)phenyl)ethyl)-2-methylpropane-2-sulfinamide

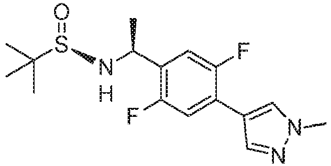

### Step 1

To a round bottom flask with stir bar was added 4-bromo-2,5-difluorobenzaldehyde (5.3 g, 24.0 mmol), (R)-2-methylpropane-2-sulfinamide (3.2 g, 26.4 mmol) and DCE (80 mL). To this mixture was then added copper (II) sulfate (5.74 g, 36.0 mmol). The reaction mixture was heated in a preheated oil bath at 60 °C for 18 hours. The reaction mixture was filtered through a pad celite, washing the solids with DCE. The filtrate was concentrated to afford a viscous green oil of (R,E)-N-(4-bromo-2,5-difluorobenzylidene)-2-methylpropane-2-sulfinamide (7.2 g, 22.2 mmol, 93 % yield). Material was taken onto next step without further purification. LCMS *m*/*z* 326.0 (M + H)⁺, Rt 1.04 min.

### Step 2

To a solution of (R,E)-N-(4-bromo-2,5-difluorobenzylidene)-2-methylpropane-2-sulfinamide (7.2 g, 22.2 mmol in CH₂Cl₂ (200 mL) cooled to 0°C (water/ice bath) under nitrogen, was added 3M methyl magnesium bromide (29.6 mL, 89 mmol) in Et₂O. Reaction mixture allowed to stir for 5 hours at 0°C then quenched with the slow addition of a saturated solution of NH₄Cl. Aqueous mixture adjusted to pH 8 with HCl (1 N) and extracted with DCM. Organic phases combined, washed with water, brine, dried (Na₂SO₄), filtered and concentrated onto silica gel. Silica gel column chromatography (EtOAc/Heptane 30 to 100%) provided (R)-N-((S)-1-(4-bromo-2,5-difluorophenyl)ethyl)-2-methylpropane-2-sulfinamide (6.86 g, 20.2 mmol, 91% yield) LCMS *m*/*z* 342.1 (M + H)⁺, Rt 0.96 min.

### Step 3

To two microwave vials with stir bars were added (R)-N-((S)-1-(4-bromo-2,5-difluorophenyl) ethyl)-2-methylpropane-2-sulfinamide (500 mg, 1.47 mmol), 1-Methyl-4-1H-pyrazoleboronic acid, pinacol ester (917 mg, 4.41 mmol), DME (6 ml), Na₂CO₃ (3.67 ml, 7.35 mmol) (2.0 M aq) and PdCl₂(dppf).CH₂Cl₂ adduct (60.0 mg, 0.07 mmol) divided evenly between the two vessels. Vessels were capped and heated by microwave irradiation for 20 min at 100 °C. Reaction mixtures were combined, diluted with a saturated solution of NH₄Cl and EtOAc. Phases partitioned. Aqueous phase exctracted with EtOAc and organic phases combined, washed with water, brine, dried (Na₂SO₄), filtered and concentrated onto silica gel. Silica gel column chromatography (EtOAc/Heptane 60 to 100%) provided (R)-N-((S)-1-(2,5-difluoro-4-(1-methyl-1H-pyrazol-4-yl)phenyl)ethyl)-2-methylpropane-2-sulfinamide (370 mg, 1.08 mmol, 73.7 % yield). ¹H NMR (400 MHz, CDCL₃) δ ppm 1.23 (s, 9 H) 1.57 - 1.60 (m, 3 H) 3.33 (d, *J*=4.06 Hz, 1 H) 3.97 (s, 3 H) 4.79 - 4.88 (m, 1 H) 7.10 (dd, *J*=11.20, 6.06 Hz, 1 H) 7.20 (dd, *J*=10.78, 6.19 Hz, 1 H) 7.76 (d, *J*=2.20 Hz, 1 H) 7.82 (s, 1 H). LCMS *m*/*z* 342.1 (M + H)⁺, Rt 0.77 min.

### Intermediate 36: (S)-N-((S)-1-(1-(4-chlorophenyl)-1H-imidazol-4-yl)ethyl)-2-methylpropane-2-sulfinamide

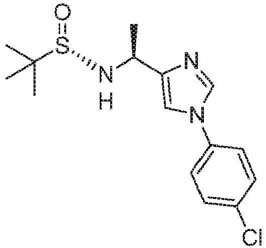

### Step 1

To a mixture of 1H-imidazole-4-carbaldehyde (3.71 g, 38.6 mmol), 1-chloro-4-iodobenzene (13.81 g, 57.9 mmol), (1R,2R)-N1,N2-dimethylcyclohexane-1,2-diamine (1.10 g, 7.72 mmol), copper(I) iodide (0.368 g, 1.93 mmol) and cesium carbonate (25.2 g, 77 mmol) was added DMF (50 mL). The reaction was sealed and heated to 110 °C for 18 hours. The reaction mixture was then cooled to RT and diluted with a saturated solution of NH₄Cl. A brown solid develops. Solid was collected, washed with water and air dried. Solid material was then dissolved in 10%MeOH:90%DCM solution and dried (Na₂SO₄), filtered and concentrated to afford a dark brown solid 1-(4-chlorophenyl)-1H-imidazole-4-carbaldehyde (8.55 g, 41.4 mmol, 107 % yield). Material as used without further purification. LCMS *m*/*z* 207.1 (M + H)⁺, Rt 0.58 min.

### Step 2

To a suspension of (S)-(-)tert-Butanesulfinamide (2.35 g, 19.4 mmol) and 1-(4-chlorophenyl)-1H-imidazole-4-carbaldehyde (4 g, 19.4 mmol) in DCE (39 mL) was added CuSO₄ (4.63 g, 29.0 mmol). The reaction mixture was heated at 60 °C for 18 hours in a oil bath. A dark brown suspension resulted. The reaction mixture was then cooled to room temperature, filtered through a pad of celite, rinsed with DCM. The solution was then concentrated onto silica gel. Silica gel column chromatography (EtOAc/Heptane 0 to 100%) provided (S,E)-N-((1-(4-chlorophenyl)-1H-imidazol-4-yl)methylene)-2-methylpropane-2-sulfinamide (1.69 g, 5.45 mmol, 28.2 % yield) as a light brown solid. LCMS *m*/*z* 310.0 (M + H)⁺, Rt 0.75 min.

### Step 3

To a solution of (S,E)-N-((1-(4-chlorophenyl)-1H-imidazol-4-yl)methylene)-2-methylpropane-2-sulfinamide (1.69 g, 5.45 mmol) in DCM (27 mL), cooled to -40°C (acetone/dry ice) under N₂, was added 3M MeMgBr (7.27 ml, 21.8 mmol) in diethyl ether. Reaction mixture allowed to stir for 1 hr at -40 °C. Reaction mixture was quenched with the slow addition of a saturated solution of NH₄Cl and diluted with EtOAc. Phases partitioned, aqueous phase exctracted with EtOAc and the organic layers combined washed with water, brine, dried (Na₂SO₄), filtered and concentrated onto silica gel. Silica gel column chromatography (EtOAc/MeOH:EtOAc 0 to 5%) provided (S)-N-((S)-1-(1-(4-chlorophenyl)-1H-imidazol-4-yl)ethyl)-2-methylpropane-2-sulfinamide (1.11 g, 3.41 mmol, 62 % yield). ¹H NMR (400 MHz, CDCL₃) δ 1.25 (s, 9 H) 1.58 (d, *J*=6.65 Hz, 3 H) 3.80 (d, *J*=5.48 Hz, 1 H) 4.59 (quin, *J*=6.36 Hz, 1 H) 7.26 (s, 1 H) 7.33 (d, *J*=8.61 Hz, 2 H) 7.41 - 7.47 (m, 2 H) 7.76 (d, *J*=1.17 Hz, 1 H). LCMS *m*/*z* 326.1 (M + H)⁺, Rt 0.59 min.

### Intermediate 37: (S)-1-(5-(4-chlorophenyl)isoxazol-3-yl)ethanamine

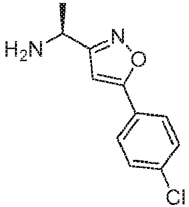

A solution of hydrochloric acid (4.0 M in 1,4-dioxane, 2.1 mL, 8.2 mmol, 2 equiv) was added to a solution of (R)-N-((S)-1-(5-(4-chlorophenyl)isoxazol-3-yl)ethyl)-2-methylpropane-2-sulfinamide (1.34 g, 4.1 mmol) in 1,4-dioxane at room temperature. A precipitate formed. The suspension was stirred for 1 hour and then concentrated in vacuo to give the hydrochloride salt of (S)-1-(5-(4-chlorophenyl)isoxazol-3-yl)ethanamine (1.1 g, light yellow solid), which was used without purification. ¹H NMR (400 MHz, CD₃OD) δ 7.87 (d, J = 8.8 Hz, 2H), 7.56 (d, J = 8.8 Hz, 2H), 6.98 (s, 1H), 4.72 (q, J = 6.9 Hz, 1H), 1.72 (d, J = 7.0 Hz, 3H). MS *m*/*z* 223.1 (M + H)⁺; Rt-0.59 min.

The intermediates in **Table 9** were prepared using a method similar to that described for the preparation of **Intermediate 37.**

**Table 9.**

| | | |
|---|---|---|
| Intermediate 37 | Intermediate 38 | Intermediate 39 |
| 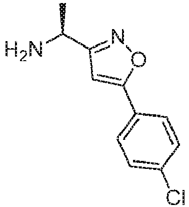 | 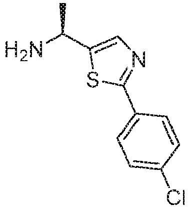 | 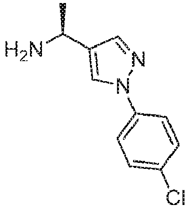 |
| Intermediate 40 | Intermediate 41 | Intermediate 42 |
| 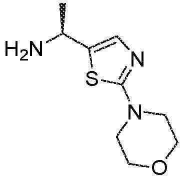 | 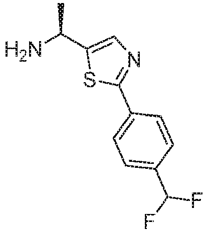 | 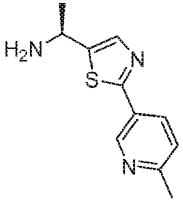 |
| Intermediate 43 | Intermediate 44 | Intermediate 45 |
| 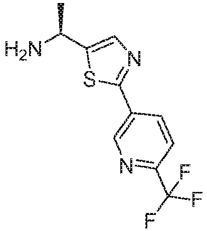 | 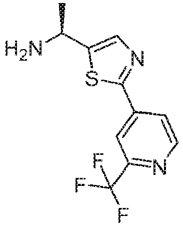 | 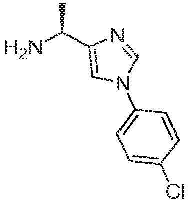 |
| Intermediate 46 | Intermediate 47 | |
| 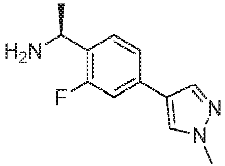 | 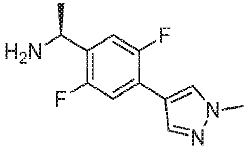 | |

**Table 10. Chemical name, NMR chemical shifts and LCMS signal for each intermediate listed in Table 9.**

| **Intermediate: Name** | **¹H NMR (400 MHz, CD₃OD) δ ppm** | **LCMS** |
|---|---|---|
| 37: (S)-1-(5-(4-chlorophenyl)isoxazol-3-yl)ethanamine | 7.87 (d, J = 8.8 Hz, 2H), 7.56 (d, J = 8.8 Hz, 2H), 6.98 (s, 1H), 4.72 (q, J = 6.9 Hz, 1H), 1.72 (d, J = 7.0 Hz, 3H). | MS *m*/*z* 223.1 (M + H)⁺; Rt-0.59 min |
| 38: (S)-1-(2-(4-chlorophenyl)thiazol-5-yl)ethanamine | 7.97 (d, J = 8.7 Hz, 2H), 7.94 (s, 1H), 7.52 (d, J = 8.7 Hz, 2H), 4.95 (m, 1H), 1.78 (d, J = 6.8 Hz, 3H) | MS m/z 239.9 (M + H)⁺; Rt-0.59 min |
| 39: (S)-1-(1-(4-chlorophenyl)-1H-pyrazol-4-yl)ethanamine | 8.43 (s, 1H), 7.87 (s, 1H), 7.78 (d, J = 9.0 Hz, 2H), 7.51 (d, J = 9.0 Hz, 2H), 4.61 (q, J = 6.9 Hz, 1H), 1.72 (d, J = 6.9 Hz, 3H) | MS m/z 223.1 (M + H)⁺; Rt-0.54 min |
| 40: (S)-1-(2-morpholinothiazol-5-yl)ethanamine | 7.56 (s, 1H), 4.78 (quin, J = 6.8 Hz, 1H), 3.88 (m, 4H), 3.68 (m, 4H), 1.71 (d, J = 6.9 Hz, 3H) | MS m/z 197.0 (M - NH₂)⁺; Rt-0.26 min |
| 41: (S)-1-(2-(4-(difluoromethyl)phenyl)thiazol-5-yl)ethanamine | 8.09 (d, J = 7.7 Hz, 2H), 7.99 (s, 1H), 7.68 (d, J = 7.7 Hz, 2H), 6.84 (t, J = 56 Hz, 1H), 4.96 (m, 1H), 1.78 (d, J = 6.9 Hz, 3H) | MS m/z 256.0 (M + H)⁺; Rt-0.56 min |
| 42: (S)-1-(2-(6-methylpyridin-3-yl)thiazol-5-yl)ethanamine | 9.30 (d, J = 2.0 Hz, 1H), 8.98 (dd, J = 8.5, 2.0 Hz, 1H), 8.14 (s, 1H), 8.06 (d, J = 8.5 Hz, 1H), 5.03 (q, J = 6.9 Hz, 1H), 2.87 (s, 3H), 1.82 (d, J = 6.9 Hz, 3H) | MS m/z 220.1 (M + H)⁺; Rt-0.27 min |
| 43: (S)-1-(2-(6-(trifluoromethyl)pyridin-3-yl)thiazol-5-yl)ethanamine | 9.31 (d, J = 2.1 Hz, 1H), 8.59 (dd, J = 8.2, 1.7 Hz, 1H), 8.10 (s, 1H), 7.98 (d, J = 8.3 Hz, 1H), 5.01 (q, J = 6.8 Hz, 1H), 1.81 (d, J = 6.8 Hz, 3H) | MS m/z 274.0 (M + H)⁺; Rt-0.51 min |
| 44: (S)-1-(2-(2-(trifluoromethyl)pyridin-4-yl)thiazol-5-yl)ethanamine | 8.87 (d, J = 5.1 Hz, 1H), 8.34 (dd, J = 1.5, 0.7 Hz, 1H), 8.17 (m, 1H), 5.02 (q, J = 6.9 Hz, 1H), 1.82 (d, J = 6.9 Hz, 3H) | MS m/z 274.0 (M + H)⁺; Rt-0.51 min |
| 45: (S)-1-(1-(4-chlorophenyl)-1H-imidazol-4-yl)ethanamine | (D2O) 1.74 (d, J=6.65 Hz, 3 H) 4.76 - 4.85 (m, 1 H) 7.61 (q, J=9.00 Hz, 4 H) 8.00 (s, 1 H) 9.04 (s, 1 H) | MS m/z 222.1 (M + H)+, Rt 0.44 min. |
| 46: (S)-1-(2-fluoro-4-(1-methyl-1H-pyrazol-4-yl)phenyl)ethanamine | | MS m/z 220.1 (M + H)+, Rt 0.43 min. |
| 47: (S)-1-(2,5-difluoro-4-(1-methyl-1H-pyrazol-4-yl)phenyl)ethanamine | (D2O) 1.65 (d, J=6.94 Hz, 3 H) 3.53 (q, J=7.11 Hz, 1 H) 3.91 (s, 3 H) 7.28 (dd, J=11.10, 6.26 Hz, 1 H) 7.44 (dd, J=11.18, 6.24 Hz, 1 H) 7.94 (s, 1 H) 8.06 (d, J=1.86 Hz, 1 H) | MS m/z 239.1 (M + H)+, Rt 0.45 min. |

### Intermediate 48: (S)-1-(2-fluoro-4-(2-fluoropropan-2-yl)phenyl)ethanamine

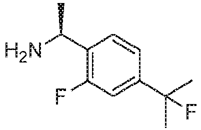

### Step 1

To a round bottom flask containing (R)-N-((S)-1-(2-fluoro-4-(prop-1-en-2-yl)phenyl)ethyl)-2-methylpropane-2-sulfinamide (1.02 g, 3.60 mmol) was added dioxane (7 mL). To this homogenous solution was then added HCl in Dioxane (1.80 mL, 7.20 mmol, 4 M). Resulting reaction mixture allowed to stir 10 min at RT. Volatiles removed. Et₂O was added and mixture sonnicated briefly. Volatiles removed again. Et₂O was added and the solid collected and washed with Et₂O to afford a white HCl salt of (S)-1-(2-fluoro-4-(prop-1-en-2-yl)phenyl)ethanamine (742 mg, 3.44 mmol, 96 % yield). ¹H NMR (400 MHz, D₂O) δ 1.65 (d, *J*=6.94 Hz, 3 H) 2.12 (s, 3 H) 5.23 (s, 1 H) 5.50 (s, 1 H) 7.37 (d, *J*=13.06 Hz, 1 H) 7.43 (m, 2H). LCMS *m*/*z* 163.2 (deamino fragment) (M + H)⁺, Rt 0.56 min.

### Step 2

To a RBF containing (S)-1-(2-fluoro-4-(prop-1-en-2-yl)phenyl)ethanamine (742 mg, 3.44 mmol) was added NMP (7 mL). To this solution was then added TEA (959 µl, 6.88 mmol) followed by the addition of Di-tert-butyl dicarbonate (976 mg, 4.47 mmol). Resulting reaction mixture allowed to stir 2 hr at room temperature. Reaction mixture was diluted with water and exctracted with EtOAc. Organic phases combined, washed with water, brine, dried (Na₂SO₄), filtered and concentrated onto silica gel. Silica gel column chromatography (EtOAc/Heptanes 0 to 100%) provided (S)-tert-butyl (1-(2-fluoro-4-(prop-1-en-2-yl)phenyl)ethyl)carbamate (1.28 g, 4.58 mmol, 133 % yield) as a white crystalline. ¹H NMR (400 MHz, CDCl₃) δ 1.40 - 1.48 (m, 12 H) 2.12 (d, *J*=0.44 Hz, 3 H) 4.98 (br. s., 2 H) 5.10 - 5.12 (m, 1 H) 5.37 (s, 1 H) 7.11 - 7.16 (m, 1 H) 7.19 - 7.24 (m, 2 H). LCMS *m*/*z* 163.0 (deamino fragment) (M + H)⁺, Rt 1.13 min.

### Step 3

To a round bottom flask containing (S)-tert-butyl (1-(2-fluoro-4-(prop-1-en-2-yl)phenyl) ethyl)carbamate (1.28 g, 4.58 mmol) was added DCM (23 mL). The homogenous solution was cooled to -70 °C in a acetone/dry ice bath. Ozone (g) was then gently bubbled through the solution for 25 min at which time the solution becomes pale blue in color. Dimethyl sulfide (1.02 mL, 13.8 mmol) was then added to the cold solution and mixture gradually allowed to warm to room temperature and stirred for 30 min. Reaction mixture was diluted with a water. Phases partitioned. Aqueous phase exctracted with DCM. Organic phases combined, washed with brine, dried (Na₂SO₄), filtered and concentrated onto silica gel. Silica gel column chromatography (EtOAc/Heptane 0 to 60%) provided (S)-tert-butyl (1-(4-acetyl-2-fluorophenyl)ethyl)carbamate (296 mg, 1.05 mmol, 23 % yield) as a colorless oil that crystallizes upon standing. ¹H NMR (400 MHz, CDCl₃) δ 1.38 - 1.49 (m, 12 H) 2.59 (s, 3 H) 5.01 (br. s., 1 H) 7.40 (t, *J*=7.65 Hz, 1 H) 7.62 (dd, *J*=11.20, 1.57 Hz, 1 H) 7.71 (dd, *J*=7.95, 1.54 Hz, 1 H). LCMS *m*/*z* 267.1 (carboxylic acid fragment + CH₃CN) (M + H)⁺, Rt 0.89 min.

### Step 4

To a solution of (S)-tert-butyl (1-(4-acetyl-2-fluorophenyl)ethyl)carbamate (296 mg, 1.05 mmol) in DCM (5.2 mL), cooled to 0°C (water/ice bath) under N₂, was added 3M MeMgBr (1.4 mL, 4.21 mmol) in diethyl ether. Reaction mixture allowed to stir for 5 min at 0 °C. Then gradually allowed to warm to RT and stirred for 30 min at RT. Reaction mixture was cooled to 0 °C then quenched with the slow addition of a saturated solution of NH₄Cl and diluted with DCM. Phases partitioned, aqueous phase exctracted with DCM and the organic layers combined washed with water, brine, dried (Na₂SO₄), filtered and concentrated to (S)-tert-butyl (1-(2-fluoro-4-(2-hydroxypropan-2-yl)phenyl)ethyl)carbamate (288 mg, 0.97 mmol, 92 % yield) afford as a colorless oil which slowly crystallizes upon standing. ¹H NMR (400 MHz, CDCl₃) δ 1.39 - 1.48 (m, 12 H) 1.57 (s, 6 H) 7.15 - 7.25 (m, 2 H) 7.31 - 7.36 (m, 1 H).

### Step 5

To a round bottom flask containing (S)-tert-butyl (1-(2-fluoro-4-(2-hydroxypropan-2-yl)phenyl)ethyl) carbamate (288 mg, 0.97 mmol) was added DCM (5 mL) the resulting colorless solution was cooled to -70 °C in a dry ice/acetone bath. To this cold solution under N₂ was then added DAST (0.26 mL, 1.94 mmol) resulting reaction mixture allowed to stir 1 hr at -70 °C. To the cold solution was added ice and resulting mixture allowed to warm to room temperature. Mixture diluted with DCM, phases partioned and the aqueous phase exctracted with DCM. Organic layers combined, washed with brine, dried (Na₂SO₄), filtered and concentrated to onto silica gel. Silica gel column chromatography (EtOAc/Heptane 0 to 50%) provided (S)-tert-butyl (1-(2-fluoro-4-(2-fluoropropan-2-yl)phenyl)ethyl)carbamate (126 mg, 0.42 mmol, 44 % yield) as a white solid. ¹H NMR (400 MHz, CDCl₃) δ 1.39 - 1.49 (m, 12 H) 1.66 (d, *J*=21.52 Hz, 6 H) 4.97 (br. s., 1 H) 7.04 - 7.12 (m, 2 H) 7.22 - 7.26 (m, 1 H). LCMS *m*/*z* 285.1 (carboxylic acid fragment + CH₃CN) (M + H)⁺, Rt 1.06 min.

### Step 6

To a round bottom flask containing (S)-tert-butyl (1-(2-fluoro-4-(2-fluoropropan-2-yl)phenyl)ethyl)carbamate (126 mg, 0.42 mmol) was added HCl in dioxane (2.1 mL, 8.42 mmol). Resulting reaction mixture allowed to stir 1 hr at room temperature. Volatiles were removed. Et₂O was then added and the mixture sonnicated briefly. Volatiles were once again removed to a afford an HCl salt of (S)-1-(2-fluoro-4-(2-fluoropropan-2-yl)phenyl)ethanamine (104 mg, 0.44 mmol, 105 % yield) as a white solid. ¹H NMR (400 MHz, D₂O) δ 1.59 - 1.80 (m, 9 H) 7.24 - 7.37 (m, 2 H) 7.43 - 7.56 (m, 1 H). LCMS *m*/*z* 200.1 (M + H)⁺, Rt 0.54 min.

### Intermediate 49: (R,E)-N-((1-(4-chlorophenyl)-1H-pyrazol-3-yl)methylene)-2-methylpropane-2-sulfinamide

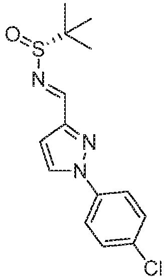

To a mixture of 1H-pyrazole-3-carbaldehyde (1.52 g, 15.82 mmol), 1-chloro-4-iodobenzene (5.66 g, 23.73 mmol), (1R,2R)-N1,N2-dimethylcyclohexane-1,2-diamine (0.450 g, 3.16 mmol), copper(I) iodide (0.151 g, 0.791 mmol) and K₂CO₃ (4.37 g, 31.6 mmol) was added toluene (20 mL). The reaction was sealed and heated to 110 °C for 18 hours. The reaction mixture was then cooled to room temperature and diluted with water (50 mL), and extracted with EtOAc (3 x 30 mL). The combined organic was then dried (Na₂SO₄) and concentrated to give crude 1-(4-chlorophenyl)-1H-pyrazole-3-carbaldehyde (1.86 g, 9.0 mmol), to which was added (R)-2-methylpropane-2-sulfinamide (1.20 g, 9.90 mmol), CuSO4 (2.155 g, 13.50 mmol) and DCE (30 ml). The reaction was sealed, heated at 60 °C for 18 hours. A dark green suspension resulted. The reaction mixture was then cooled to 20 °C, filtered through a pad of celite, rinsed with DCM. The solution was then concentrated to give final crude product as a light green oil. The residue was purified via silica gel chromatography (EtOAc/Heptane). *m*/*z* 310.3 (M + H)⁺

### Intermediate 50: (R)-N-((R)-1-(1-(4-chlorophenyl)-1H-pyrazol-3-yl)ethyl)-2-methylpropane-2-sulfinamide

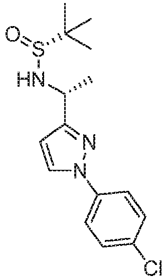

To a solution of (R,E)-N-((1-(4-chlorophenyl)-1H-pyrazol-3-yl)methylene)-2-methylpropane-2-sulfinamide (2.12 g, 6.84 mmol) in DCM (40 ml) at -40 °C was added methylmagnesium bromide (9.12 ml, 27.4 mmol). The reaction was stirred at -40 °C for 3 hours. The reaction mixture was then quenched with saturated NH₄Cl solution (20 mL). The aqueous layer was adjusted to pH 8 with HCl (1 M) and extracted with DCM (2 x 200 mL). The combined organic was dried (Na₂SO₄) and concentrated. The residue was then purified via silica gel chromatography (EtOAC/heptane) to give (R)-N-((R)-1-(1-(4-chlorophenyl)-1H-pyrazol-3-yl)ethyl)-2-methylpropane-2-sulfinamide (1.11 g). *m*/*z* 326.3 (M + H)⁺

### Intermediate 51: (R)-1-(1-(4-chlorophenyl)-1H-pyrazol-3-yl)ethanamine

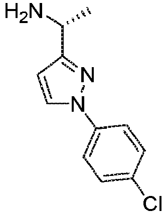

To a solution of (R)-N-((R)-1-(1-(4-chlorophenyl)-1H-pyrazol-3-yl)ethyl)-2-methylpropane-2-sulfinamide (0.98 g, 3.01 mmol) in dioxane (10 ml) was added dropwise HCl (1.504 ml, 6.01 mmol). The reaction was stirred at room temperature for 30 minutes. LCMS indicated complete conversion to product. The reaction mixture was concentrated and DCM (20mL) and saturated NaHCO₃ solution (10mL) was added to the residue. The mixture was stirred for 10 minutes and phases were separated. Aqueous layer was then extracted with DCM (2 x 10mL), and the combined organic was dried (Na₂SO₄) and concentrated to give product (0.556 g). *m*/*z* 222.2 (M + H)⁺

### Intermediate 52: (R)-N-((S)-1-(1-(4-chlorophenyl)-1H-pyrazol-3-yl)ethyl)-2-methylpropane-2-sulfinamide

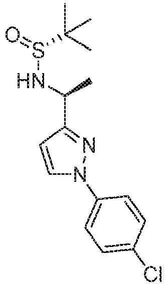

To a solution of (R,E)-N-((1-(4-chlorophenyl)-1H-pyrazol-3-yl)methylene)-2-methylpropane-2-sulfinamide (2.12 g, 6.84 mmol) in DCM (40 ml) at -40 °C was added methylmagnesium bromide (9.12 ml, 27.4 mmol). The reaction was stirred at -40 °C for 3 hours. The reaction mixture was then quenched with saturated NH₄Cl solution (20mL). The aqueous layer was adjusted to pH=8 with HCl (1M) and extracted with DCM (2 x 200 mL). The combined organic was dried (Na₂SO₄) and concentrated. The residue was then purified via silica gel chromatography (EtOAC/heptane) to give (R)-N-((R)-1-(1-(4-chlorophenyl)-1H-pyrazol-3-yl)ethyl)-2-methylpropane-2-sulfinamide (1.01 g). *m*/*z* 326.3 (M + H)⁺

### Intermediate 53: (S)-1-(1-(4-chlorophenyl)-1H-pyrazol-3-yl)ethanamine

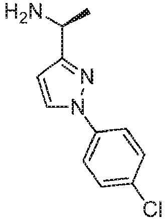

To a solution of (R)-N-((S)-1-(1-(4-chlorophenyl)-1H-pyrazol-3-yl)ethyl)-2-methylpropane-2-sulfinamide (0.98 g, 3.01 mmol) in dioxane (10 ml) was added dropwise HCl (1.504 ml, 6.01 mmol). The reaction was stirred at room temperature for 30 minutes. LCMS indicated complete conversion to product. The reaction mixture was concentrated and DCM (20mL) and saturated NaHCO₃ solution (10mL) was added to the residue. The mixture was stirred for 10 minutes and phases were separated. Aqueous layer was then extracted with DCM (2x10mL), and the combined organic was dried (Na₂SO₄) and concentrated to give crude product (0.501 g). *m*/*z* 222.2 (M + H)⁺

### Intermediate 54: (S)-1-(1-(4-fluorophenyl)-1H-imidazol-4-yl)ethanamine hydrochloride

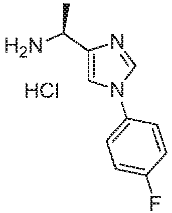

### Step 1: preparation of tert-butyl 4-formyl-1H-imidazole-1-carboxylate

To di-tert-butyl dicarbonate (23.25 g, 107 mmol) and 1H-imidazole-4-carbaldehyde (9.75 g, 101 mmol) in THF (200mL) was added DMAP (100 mg, 0.819 mmol). The reaction was stirred for two hours. The reaction mixture was then diluted with saturated NaHCO₃ solutuion/EtOAc (100 mL/100 mL). The aqueous was then extracted with EtOAc (2 x 100 mL) and the combined organic was dried (Na₂SO₄) and concentrated to give crude product (19.9 g). *m*/*z* 197.2 (M + H)⁺

### Step 2: preparation of (S,E)-tert-butyl 4-(((tert-butylsulfinyl)imino)methyl)-1H-imidazole-1-carboxylate

To CuSO₄ (24.28 g, 152 mmol) and tert-butyl 4-formyl-1H-imidazole-1-carboxylate (19.9 g, 101 mmol) in DCE (100 mL) was added (S)-2-methylpropane-2-sulfinamide (13.52 g, 112 mmol). The reaction was heated to 65 °C for 18 hours. The reaction mixture was then cooled to room temperature and filtered through a pad of celite. The pad was rinsed with DCM (200 mL) and the filtrated was concentrated. The residue was then run through a pad of silica gel with heptane/EtOAc (3:1) as eluent. The filtrate was concentrated to give crude (S,E)-tert-butyl 4-(((tert-butylsulfinyl)imino)methyl)-1H-imidazole-1-carboxylate (22 g). *m*/*z* 300.2 (M + H)⁺

### Step 3: preparation of (S,E)-N-((1H-imidazol-4-yl)methylene)-2-methylpropane-2-sulfinamide

To (S,E)-tert-butyl 4-(((tert-butylsulfinyl)imino)methyl)-1H-imidazole-1-carboxylate (18.61 g, 62.2 mmol) in DCM (250 mL) at -70 °C was added dropwise methylmagnesium bromide (83 mL, 249 mmol) in Et₂O. The reaction was stirre at -70 °C for 4 hours. The reaction mixture was then warmed to -40 °C and stirred for one hour. The reaction was then quenched with cautious addition of HCl (1N). Cold bath was removed and while with stirring the aqueous layer was adjusted pH=8. The aqueous layer was separated and extracted with DCM (3x100mL). The combined organic was dried (Na₂SO₄) and concentrated to give crude product as a mixture of tert-butyl 4-((S)-1-((S)-1,1-dimethylethylsulfinamido)ethyl)-1H-imidazole-1-carboxylate and (S,E)-N-((1H-imidazol-4-yl)methylene)-2-methylpropane-2-sulfinamide, to which was added DCM (300 mL) at 0 °C and formic acid (100 mL, 2651 mmol). The cold bath was then removed and he reaction was stirre for 2 hours. The reaction mixture was then concentrated under reduce pressure to remove DCM and formic acid. The residue was diluted with DCM (400 mL) and washed with saturated Na₂CO₃ aqueous solution (2 x 200 mL). The combined aqueous was extracted with DCM (2 x 200 mL). The combined organic was then dried (Na₂SO₄) and concentrated to give (S,E)-N-((1H-imidazol-4-yl)methylene)-2-methylpropane-2-sulfinamide (12.5 g). *m*/*z* 216.1 (M + H)⁺

### Step 4: preparation of (S)-1-(1-(4-fluorophenyl)-1H-imidazol-4-yl)ethanamine hydrochloride

To a 200mL round bottom flask was added toluene/dioxane (80 ml/20 mL). The flask was cooled to 0 °C and the mixture of solvents was evacuated under high vaccum for 2 minutes and then recharged with argon. The process was repeated three more times. This solvent was then used for the reaction.

A vial containing di-tert-butyl(2',4',6'-triisopropyl-3,4,5,6-tetramethyl-[1,1'-biphenyl]-2-yl)phosphine (55.8 mg, 0.116 mmol) and Pd₂(dba)₃ (42 mg, 0.046 mmol) was evacuated under high vaccum for 1 minute and then recharged with argon. The process was repeated three more times and the toluene/dioxane solvent (10 mL) prepared as above was added followed by the palladium/ligand complex prepared as above was then added to the reaction vial containing the other starting materials. The reaction mixture was sealed and heated to 120 °C and stirred for 5 minutes. The reaction was cooled to room temperature.

A separate reaction vial was charged with (S,E)-N-((1H-imidazol-4-yl)methylene)-2-methylpropane-2-sulfinamide (500 mg, 2.322 mmol), 1-bromo-4-fluorobenzene (447 mg, 2.55 mmol) and K₃PO₄ (986 mg, 4.64 mmol). The vial was evacuated under high vaccum for 1 minute and then recharged with argon. The process was repeated three more times and the palladium/ligand complex prepared as above was then added to the reaction vial containing the other starting materials. The reaction was sealed and heated to 120 °C for 18 hours. LCMS show complete conversion. The reaction mixture was then cooled to room temperature and filtered through a pad of celite. The solid was rinsed with EtOAc (30 mL). The filtrate was then washed with water (2 x 20 mL). The aqueous layer was then extracted with EtOAc (20 mL). The combined organic was then concentrated. The residue was purified via silica gel chromatography (EtOAc/Heptane 70%-100% with 5% MeOH) to give (S)-N-((S)-1-(1-(4-fluorophenyl)-1H-imidazol-4-yl)ethyl)-2-methylpropane-2-sulfinamide. *m*/*z* 310.2 (M + H)⁺

To the above intermediate product was added MeOH (5mL) and HCl (4M in dioxane, 1mL). The reaction mixture was stirred for one hour and LCMS showed complete conversion, The mixture was then concentrated to give (S)-1-(1-(4-fluorophenyl)-1H-imidazol-4-yl)ethanamine hydrochloride (300 mg). *m*/*z* 206.0 (M + H)⁺

### Intermediate 55: 5-chloro-6-(1,1-difluoroethyl)nicotinaldehyde

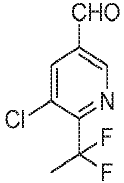

### Step 1: Preparation of ethyl 5,6-dichloronicotinate

To a solution of 5,6-dichloronicotinic acid (20.01 g, 104 mmol) in EtOH (500 mL) at 20 °C was added chlorotrimethylsilane (132 mL, 1042 mmol). The reaction was stirred for 72 hours. The reaction mixture was then concentrated and diluted with EtOAc (500mL), and washed with saturated NaHCO₃ (2x100mL) and brine (100mL). The organic was then dried (Na₂SO₄) and concentrated under reduced pressure to give final crude product (21.25 g). LCMS *m*/*z* 220.1 (M + H)⁺, Rt 0.94 min.

### Step 2: Preparation of ethyl 6-acetyl-5-chloronicotinate

To a suspension of ethyl 5,6-dichloronicotinate (5.26 g, 23.90 mmol) and tetraethylammonium-chloride (11.88 g, 71.7 mmol) in MeCN (50 mL) was added tributyl(1-ethoxyvinyl)stannane (9.50 g, 26.3 mmol) and PdCl₂(PPh₃)₂ (0.671 g, 0.956 mmol). The reaction was sealed, heated at 80 °C for 5 hours. A dark color clear solution resulted. The reaction mixture was then cooled to 20 °C, concentrated and diluted with EtOAc (200mL), and washed with water (50mL) and brine (50mL). The organic was then dried (Na₂SO₄) and concentrated to give crude ethyl 5-chloro-6-(1-ethoxyvinyl)nicotinate. The residue was then dissolved in THF (100mL) and HCl (20mL, 3M in H₂O) was added. The reaction mixture was stirred at 20 °C for 5 hours, and saturated NaHCO₃ solution was added until pH=8. The mixture was then diluted with EtOAc (200mL) and water (50mL). The phases were separated and the aqueous layer was extracted with EtOAc (2x50mL). The combined organics was washed with brine (20mL), dried (Na₂SO₄) and concentrated to afford the desired product (3.56 g). LCMS *m*/*z* 228.5 (M + H)⁺, Rt 0.83 min.

### Step 3: Preparation of ethyl 5-chloro-6-(1,1-difluoroethyl)nicotinate

To a solution of ethyl 6-acetyl-5-chloronicotinate (3.01 g, 13.22 mmol) in CHCl3 (7 mL) was added DAST (5.20 mL, 39.7 mmol) and ethanol (0.061 g, 1.32 mmol). The reaction was sealed, heated at 60 °C for 24 hours. A dark color clear solution resulted. The reaction mixture was then cooled to 20 °C, and added cautiously with cold concentrated NaHCO₃ aqueous solution (50mL). The aqueous layer was extracted with DCM (2x100mL). The combined organic was then dried (Na₂SO₄) and concentrated. The residue was purified via silica gel flash chromatography (0-20percent EtOAc-Hexanes) to afford the desired product as yellow oil (2.88 g). LCMS *m*/*z* 250.1 (M + H)⁺, Rt 0.99 min.

### Step 4: Preparation of (5-chloro-6-(1,1-difluoroethyl)pyridin-3-yl)methanol

To a solution of ethyl 5-chloro-6-(1,1-difluoroethyl)nicotinate (2.68 g, 10.74 mmol) in Et₂O (40mL) was added LiBH₄ (0.351 g, 16.10 mmol), followed by dropwise addition of methanol (0.653 mL, 16.10 mmol). The reaction was refluxed at 40 °C for one hour. The reaction mixture was then cooled to 0 °C, and quenched with HCl (1M) until pH=2 for aqueous layer. The phases were separated and the aqueous layer was extracted with DCM (3x50mL). The organic was then dried (Na₂SO₄) and concentrated under reduced pressure to give final crude product (2.12 g). LCMS *m*/*z* 208.0 (M + H)⁺, Rt 0.63 min.

### Step 5: Preparation of 5-chloro-6-(1,1-difluoroethyl)nicotinaldehyde

To a solution of (5-chloro-6-(1,1-difluoroethyl)pyridin-3-yl)methanol (2.12 g, 10.21 mmol) in DCM (100 ml) was added PCC (3.30 g, 15.32 mmol). The reaction was stirred at 20 °C for 3 hours. A dark color suspension resulted. LCMS showed clean conversion to the product. The reaction mixture was then filtered through a pad of celite, and washed with DCM (200mL). The filtrate was then concentrated to give crude product (1.78 g). LCMS *m*/*z* 224.0 (M + H2O + H)⁺, Rt 0.72 min.

### Intermediate 56: 5-chloro-6-(2,2,2-trifluoroethoxy)nicotinaldehyde

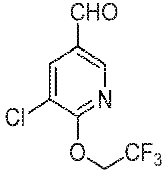

### Step 1: Preparation of ethyl 5-chloro-6-(2,2,2-trifluoroethoxy)nicotinate

To a solution of ethyl 5,6-dichloronicotinate (6.28 g, 28.5 mmol) and 2,2,2-trifluoroethanol (2.71 ml, 37.1 mmol) in THF (90 ml) at -73oC was added NaHMDS (37.1 ml, 37.1 mmol). The reaction was stirred at -73 °C for 30 minutes, then at 0 °C for 5 hours. The reaction was quenched with 30 mL saturated NH₄Cl solution. The reaction mixture was then poured into 50 mL brine and phases were separated. The aqueous layer was extracted with DCM (2x100mL). The combined organics were dried (Na₂SO₄) and concentrated. Silica gel chromatography with 100% heptane to 30% EtOAc in heptane provided final product (7.51 g). LCMS *m*/*z* 284.1 (M + H)⁺, Rt 1.07 min.

### Step 2: Preparation of (5-chloro-6-(2,2,2-trifluoroethoxy)pyridin-3-yl)methanol

To a solution of ethyl 5-chloro-6-(2,2,2-trifluoroethoxy)nicotinate (7.51 g, 26.5 mmol) in Et2O (200mL) was added LiBH₄ (0.865 g, 39.7 mmol), followed by drop wise addition of methanol (1.611 ml, 39.7 mmol). The reaction was refluxed at 40 °C for one hour. The reaction mixture was then cooled to 0 °C, and quenched with HCl (1M) until pH=2 for aqueous layer. The phases were separated and the aqueous layer was extracted with DCM (3x200mL). The organic was then dried (Na₂SO₄) and concentrated under reduced pressure to give final crude product (6.31 g). LCMS *m*/*z* 242.1 (M + H)⁺, Rt 0.77 min.

### Step 3: Preparation of 5-chloro-6-(2,2,2-trifluoroethoxy)nicotinaldehyde

To a solution of (5-chloro-6-(2,2,2-trifluoroethoxy)pyridin-3-yl)methanol (4.00 g, 16.56 mmol) in EtOAc (15 mL) was added manganese(IV) oxide (16.93 g, 166 mmol). The reaction was heated with microwave at 120 °C for 30 minutes. The mixture was then filtered through a pad of celite, and rinsed with EtOAc. The filtrated was concentrated to give crude product (3.38 g).

The intermediates in **Table 17** were prepared with procedures similar to those used to prepare **Intermediate 52.**

**Table 17.**

| **Intermediate: Name** | **Structure** | **LCMS** |
|---|---|---|
| 57: (R)-N-((S)-1-(5-chloro-6-(1,1-difluoroethyl)pyridin-3-yl)ethyl)-2-methylpropane-2-sulfinamide | 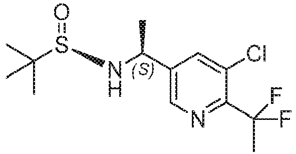 | MS *m*/*z* 325.2 (M + H)⁺, Rt 0.85 min. |
| 58: (R)-N-((S)-1-(5-chloro-6-(2,2,2-trifluoroethoxy)pyridin-3-yl)ethyl)-2-methylpropane-2-sulfinamide | 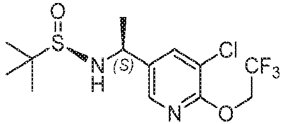 | MS *m*/*z* 359.1 (M + H)⁺, Rt 0.95 min. |

The intermediates in **Table 18** were prepared with procedures similar to those used to prepare **Intermediate 53.**

**Table 18.**

| **Intermediate: Name** | **Structure** | **LCMS** |
|---|---|---|
| 59: (S)-1-(5-chloro-6-(1,1-difluoroethyl)pyridin-3-yl)ethanamine | 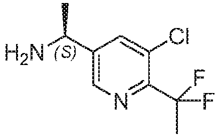 | MS *m*/*z* 221.1 (M + H)⁺, Rt 0.50 min. |
| 60: (S)-1-(5-chloro-6-(2,2,2-trifluoroethoxy)pyridin-3-yl)ethanamine | 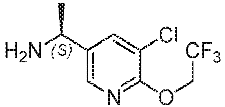 | MS *m*/*z* 255.1 (M + H)⁺, Rt 0.62 min. |

### Intermediate 61: (S)-tert-butyl (1-(5-bromopyridin-2-yl)ethyl)carbamate

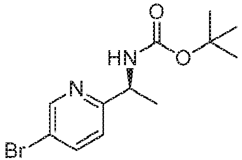

To a solution of (S)-1-(5-bromopyridin-2-yl)ethanamine (300 mg, 1.49 mmol) in DCM (7.5 mL) was added di-tert-butyl dicarbonate (358 mg, 1.64 mmol) and triethylamine (0.31 mL, 2.24 mmol). The solution was stirred for 16 h at room temperature then washed with water and brine. The organic layer was dried over Na2SO4, filtered and concentrated. Silica gel column chromatography (EtOAc/heptane 0 to 80%) provided a white solid (308 mg, 68.5% yield). ¹H NMR (400 MHz, CDCI3) δ 8.59 (d, *J* = *2.2* Hz, 1H), 7.76 (dd, *J* = 8.3, 2.4 Hz, 1H), 7.16 (d, *J* = 8.3 Hz, 1H), 5.57 - 5.42 (m, 1H), 4.86 - 4.73 (m, 1H), 1.43 (t, *J* = 3.4 Hz, 12H); MS m/z 303.4 (M + H).

The intermediates in **Table 27** were prepared using a method similar to that described for the preparation of **intermediate 61.**

**Table 27.**

| | | |
|---|---|---|
| Intermediate 62 | Intermediate 63 | Intermediate 64 |
| 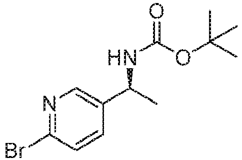 | 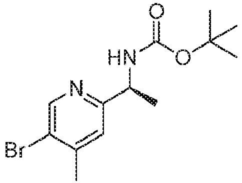 | 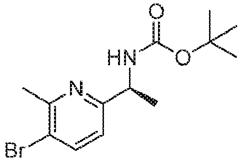 |
| Intermediate 65 | Intermediate 66 | |
| 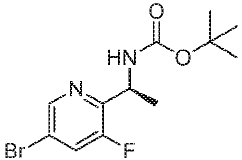 | 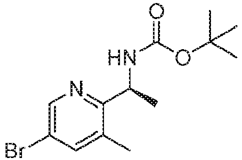 | |

**Table 28. Chemical name and analytical data for each intermediate listed in Table 27.**

| **Intermediate: Name** | **Analytical data** |
|---|---|
| 62: (S)-tert-butyl (1-(6-bromopyridin-3-yl)ethyl)carbamate | ¹H NMR (400 MHz, CDCl3) δ 8.33 (d, *J* = 2.6 Hz, 1H), 7.49 (dd, *J* = 8.2, 2.5 Hz, |
| | 1H), 7.44 (d, *J* = 8.3 Hz, 1H), 4.88 - 4.69 (m, 2H), 1.45 (d, *J* = 7.1 Hz, 3H), 1.41 (s, 9H); MS m/z 303.4 (M + H). |
| 63: (S)-tert-butyl (1-(5-bromo-4-methylpyridin-2-yl)ethyl)carbamate | LCMS tR = 1.31 min; MS m/z 317.0 (M+H) |
| 64: (S)-tert-butyl (1-(5-bromo-6-methylpyridin-2-yl)ethyl)carbamate | LCMS tR = 1.35 min; MS m/z 317.1 (M+H) |
| 65: (S)-tert-butyl (1-(5-bromo-3-fluoropyridin-2-yl)ethyl)carbamate | LCMS tR = 1.31 min; MS m/z 319.0 (M+H) |
| 66: (S)-tert-butyl (1-(5-bromo-3-methylpyridin-2-yl)ethyl)carbamate | LCMS tR = 1.37 min; MS m/z 317.0 (M+H) |

### Intermediate 67: (S)-tert-butyl (1-(5-(4-fluoro-3-methylphenyl)pyridin-2-yl)ethyl)carbamate

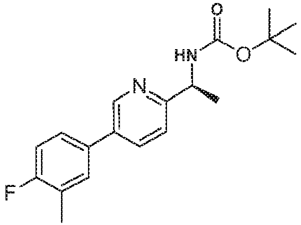

In a 5 mL microwave vial a solution of (S)-tert-butyl (1-(5-bromopyridin-2-yl)ethyl)carbamate (60 mg, 0.2 mmol), (4-fluoro-3-methylphenyl)boronic acid (37 mg, 0.24 mmol), Sodium bicarbonate (0.2 mL, 0.4 mmol, 2 M aqueous solution) in Dioxane (2 mL) was bubbled N₂ for 3 min then Cl₂Pd(dppf)CH₂Cl₂ (16 mg, 0.02 mmol) was added. The capped tube was heated to 100°C for 16 h. After cooling the reaction mixture was diluted with EtOAc (10 mL) and washed with water (10 mL). After separation, the aqueous phase was extracted with EtOAc (3 x 10 mL). Combined organics were dried over Na₂SO₄, filtered and concentrated. The crude material was purified through silica gel column chromatography (EtOAc in Heptane 12 to 100%) to give a white solid (66 mg, 80% yield). LCMS tR = 1.43 min; MS m/z 331.1 (M+H).

The intermediates in **Table 29** were prepared using a method similar to that described for the preparation of **intermediate 67.**

**Table 29.**

| | | |
|---|---|---|
| Intermediate 68 | Intermediate 69 | Intermediate 70 |
| 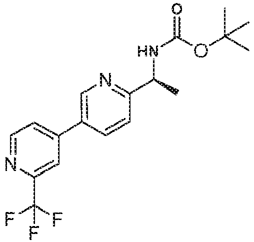 | 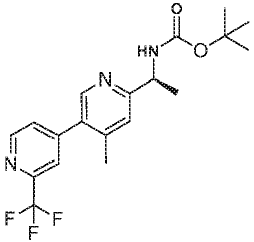 | 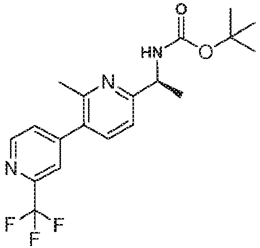 |
| Intermediate 71 | Intermediate 72 | |
| 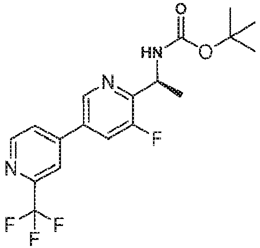 | 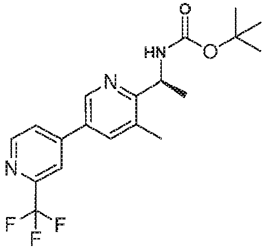 | |

**Table 30. Chemical name and analytical data for each intermediate listed in Table 29.**

| **Intermediate: Name** | **Analytical data** |
|---|---|
| 68: (S)-tert-butyl (1-(2'-(trifluoromethyl)-[3,4'-bipyridin]-6-yl)ethyl)carbamate | ¹H NMR (400 MHz, CDCl3) δ 8.83 (t, *J* = 3.9 Hz, 2H), 7.92 (dd, *J* = 8.3, 2.5 Hz, 1H), 7.87 (d, *J* = 1.7 Hz, 1H), 7.73 - 7.66 (m, 1H), 7.42 (d, *J* = 8.1 Hz, 1H), 5.59 (d, *J* = 7.6 Hz, 1H), 4.93 (p, *J* = 6.9 Hz, 1H), 1.50 (d, *J* = 7.0 Hz, 3H), 1.45 (s, 9H); MS m/z 368.2 (M + H). |
| 69: (S)-tert-butyl (1-(4-methyl-2'-(trifluoromethyl)-[3,4'-bipyridin]-6-yl)ethyl)carbamate | ¹H NMR (400 MHz, CDCl3) δ 8.83 (d, *J* = 4.9 Hz, 1H), 8.36 (s, 1H), 7.66 (s, 1H), 7.51 - 7.42 (m, 1H), 7.21 (s, 1H), 5.60 (d, J = 7.7 Hz, 1H), 4.87 (p, *J* = 6.9 Hz, 1H), 2.30 (s, 3H), 1.48 (d, *J* = 6.9 Hz, 3H), 1.45 (s, 9H); MS m/z 326.4 (M + H - 56). |
| 70: (S)-tert-butyl (1-(2-methyl-2'-(trifluoromethyl)-[3,4'-bipyridin]-6-yl)ethyl)carbamate | ¹H NMR (400 MHz, CDCl3) δ 8.81 (d, *J* = 5.0 Hz, 1H), 7.66 (s, 1H), 7.50 (d, *J* = 7.9 Hz, 1H), 7.47 (dd, *J* = 4.9, 1.6 Hz, 1H), 7.20 (d, *J* = 7.8 Hz, 1H), |
| | 5.75 (d, *J* = 7.5 Hz, 1H), 4.86 (p, *J* = 6.9 Hz, 1H), 2.51 (s, 3H), 1.48 (d, *J* = 6.9 Hz, 3H), 1.46 (s, 9H); MS m/z 326.4 (M + H - 56). |
| 71: (S)-tert-butyl (1-(5-fluoro-2'-(trifluoromethyl)-[3,4'-bipyridin]-6-yl)ethyl)carbamate | ¹H NMR (400 MHz, CDCl₃) δ 8.85 (d, *J* = 5.1 Hz, 1H), 8.66 (s, 1H), 7.87 - 7.84 (m, 1H), 7.70 - 7.67 (m, 1H), 7.65 (dd, *J* = 9.8, 1.9 Hz, 1H), 5.76 (d, *J* = 7.7 Hz, 1H), 5.31 - 5.23 (m, 1H), 1.47 (d, *J* = 6.8 Hz, 3H), 1.45 (s, 9H); MS m/z 386.1 (M + H). |
| 72: (S)-tert-butyl (1-(5-methyl-2'-(trifluoromethyl)-[3,4'-bipyridin]-6-yl)ethyl)carbamate | ¹H NMR (400 MHz, CDCl3) δ 8.81 (d, *J* = 5.1 Hz, 1H), 8.68 (d, *J* = 2.2 Hz, 1H), 7.88 - 7.85 (m, 1H), 7.71 (d, *J* = 1.7 Hz, 1H), 7.68 (dd, *J* = 5.0, 1.7 Hz, 1H), 5.99 (d, *J* = 8.2 Hz, 1H), 5.13 (p, *J* = 6.7 Hz, 1H), 2.48 (s, 3H), 1.45 (s, 9H), 1.42 (d, *J* = 6.6 Hz, 3H); MS m/z 382.2 (M + H). |

### Intermediate 73: (S)-4-(1-(tert-butoxycarbonylamino)ethyl)-2-fluorobenzoic acid

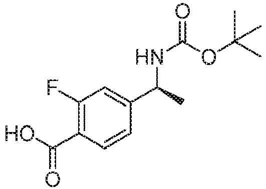

To a solution of (S)-4-(1-aminoethyl)-2-fluorobenzoic acid (5 g, 22.76 mmol) in water (66 mL) and THF (66 mL) was added di-tert-butyl dicarbonate (6.95 g, 31.9 mmol) and sodium carbonate (5.74 g, 68.3 mmol). The solution was stirred for 16 h at room temperature then THF was removed under reduced pressure. The aqueous solution was acidified with 1N HCl to pH 3-4 and extracted with EtOAc (3 x 60 mL). Combined organics were dried over Na₂SO₄, filtered and concentrated to give a white solid (1.94 g, 30.1 % yield). The crude product was used to next step without further purification.
¹H NMR (400 MHz, MeOD) δ 7.89 (t, *J* = 7.8 Hz, 1H), 7.20 (dd, *J* = 8.2, 1.7 Hz, 1H), 7.13 (dd, *J* = 12.0, 1.6 Hz, 1H), 4.70 (d, *J* = 7.1 Hz, 1H), 1.47 - 1.35 (m, 12H); MS m/z 282.0 (M - H).

### Intermediate 74: (S)-tert-butyl 1-(3-fluoro-4-(methoxy(methyl)carbamoyl) phenyl)ethylcarbamate

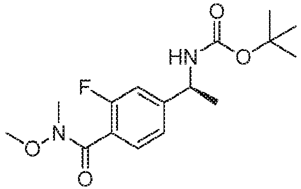

A solution of (S)-4-(1-(tert-butoxycarbonylamino)ethyl)-2-fluorobenzoic acid (1.416 g, 5mmol), N,O-dimethylhydroxylamine hydrochloride (732 mg, 7.5 mmol), HATU (2.85 g, 7.5 mmol) and DIPEA (3.49 mL, 20 mmol) in DMF (25 mL) was stirred at room temperature for 16 h. The reaction mixture was diluted with EtOAc and washed with water. After separation, the aqueous phase was washed with EtOAc (2 x 75 mL). Combined organics were dried over Na₂SO₄, filtered and concentrated. Silica gel column chromatography (EtOAc/heptane 12 to 100%) provided (S)-tert-butyl 1-(3-fluoro-4-(methoxy(methyl)carbamoyl)phenyl)ethylcarbamate as a white solid (1.5 g, 92 % yield).
¹H NMR (400 MHz, CDCl3) δ 7.40 (t, *J* = 7.4 Hz, 1H), 7.13 (dd, *J* = 7.8, 1.6 Hz, 1H), 7.04 (dd, *J* = 10.7, 1.6 Hz, 1H), 4.80 (br s, 1H), 3.56 (s, 3H), 3.34 (s, 3H), 1.50 - 1.29 (m, 12H); MS m/z 327.1 (M + H).

### Intermediate 75: (S)-tert-butyl 1-(3-fluoro-4-formylphenyl)ethylcarbamate

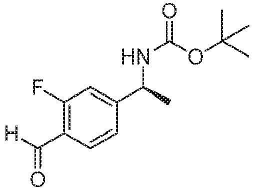

To a cooled (0 °C) solution of (S)-tert-butyl 1-(3-fluoro-4-(methoxy(methyl)carbamoyl)phenyl)ethylcarbamate (1.175 g, 3.6 mmol) in THF (36 mL) was added a solution of LAH in THF (1.0 M, 18 mL, 18 mmol) and the resulting mixture was stirred at 0 °C for 20 min. The reaction mixture was quenched by addition of a saturated Na2SO4 solution until gas evolution ceased. The reaction mixture was extracted with EtOAc (2 x 100 mL). Combined organics were dried over Na2SO4, filtered and concentrated. Silica gel column chromatography (EtOAc/heptane 12 to 100%) provided (S)-tert-butyl 1-(3-fluoro-4-formylphenyl)ethylcarbamate as a white solid (760 mg, 79% yield).
¹H NMR (400 MHz, CDCl3) δ 10.31 (s, 1H), 7.87 - 7.80 (m, 1H), 7.20 (dd, *J* = 8.2, 1.3 Hz, 1H), 7.11 (dd, *J* = 11.5, 1.4 Hz, 1H), 4.80 (br s, 1H), 1.45 (br s, 12H); MS m/z 212.1 (M - 56 + H).

### Intermediate 76: (S)-tert-butyl 1-(3-fluoro-4-((3,3,4-trimethylpiperazin-1-yl)methyl)phenyl)ethylcarbamate

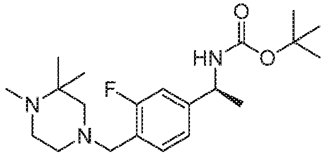

A solution of (S)-tert-butyl 1-(3-fluoro-4-formylphenyl)ethylcarbamate (267 mg, 1 mmol) and 1,2,2-trimethylpiperazine dihydrochloride (402 mg, 2 mmol) in THF (5 mL) was stirred at room temperature for 1 h and treated with sodium triacetoxyborohydride (848 mg, 4 mmol). The resulting mixture was stirred at room temperature for 16 h. The reaction mixture was quenched with saturated aqueous solution of NaHCO3 (15 mL) and extracted with EtOAc (3 x 25 mL). Combined organics were dried over Na2SO4, filtered and concentrated. Silica gel column chromatography (MeOH/CH2CI2 0 to 10%) provided (S)-tert-butyl 1-(3-fluoro-4-((3,3,4-trimethylpiperazin-1-yl)methyl)phenyl)ethylcarbamate as a white solid (186 mg, 49% yield). ¹H NMR (400 MHz, CDCl3) δ 7.35 (t, *J* = 7.7 Hz, 1H), 7.03 (dd, *J* = 7.9, 1.9 Hz, 1H), 6.95 (dd, *J* = 11.1, 1.8 Hz, 1H), 4.77 (s, 1H), 3.49 (s, 2H), 2.56 (br s, 4H), 2.24 (br s, 5H), 1.42 (br s, 12H), 1.04 (s, 6H); MS m/z 380.4 (M + H).

### Intermediate 77: (S)-tert-butyl 1-(4-((4,4-difluoropiperidin-1-yl)methyl)-3-fluorophenyl)ethylcarbamate

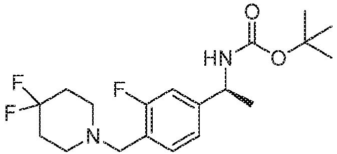

Following the procedure describing **intermediate 230,** title compound was prepared from (S)-tert-butyl 1-(3-fluoro-4-formylphenyl)ethylcarbamate and 4,4-difluoropiperidine hydrochloride as a white solid. LCMS tR = 1.63 min; MS m/z 371.5 (M - H).

### Intermediate 78: (S)-1-(5-(4-fluoro-3-methylphenyl)pyridin-2-yl)ethanamine

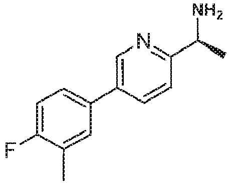

To a solution of (S)-tert-butyl (1-(5-(4-fluoro-3-methylphenyl)pyridin-2-yl)ethyl)carbamate (66 mg, 0.47 mmol) in DCM (2 mL) was added TFA (2 mL, 26 mmol) slowly at -78 °C. The reaction was stirred at room temperature for 1 h then concentrated and diluted with DCM (10 mL). The solution was stirred with 3 eq. of MP-carbonate resin (3.28 mmol/g, Biotage) for 1 hr at room temperature. The resin was removed by filtration and washed (2 x 5 mL) with DCM. The filtrate was concentrated and the crude residue was used to next step without further purification. LCMS tR = 0.97 min; MS m/z 231.1 (M + H).

The intermediates in **Table 37** were prepared using a method similar to that described for the preparation of **intermediate 78.**

**Table 37.**

| | | |
|---|---|---|
| Intermediate 79 | Intermediate 80 | Intermediate 81 |
| 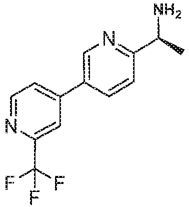 | 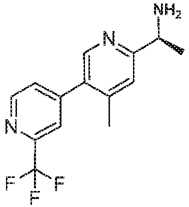 | 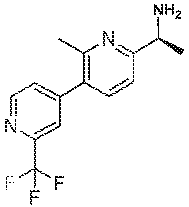 |
| Intermediate 82 | Intermediate 83 | Intermediate 84 |
| 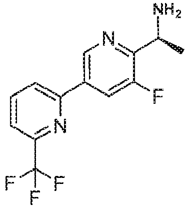 | 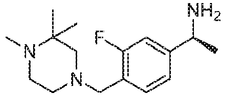 | 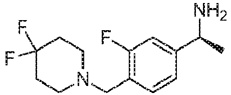 |

**Table 38. Chemical name and analytical data for each intermediate listed in Table 37.**

| **Intermediate: Name** | **Analytical data** |
|---|---|
| 79: (S)-1-(2'-(trifluoromethyl)-[3,4'-bipyridin]-6-yl)ethanamine | LCMS tR = 0.79 min; MS |
| | m/z 268.1 (M + H). |
| 80: (S)-1-(4-methyl-2'-(trifluoromethyl)-[3,4'-bipyridin]-6-yl)ethanamine | LCMS tR = 0.85 min; MS m/z 282.1 (M + H). |
| 81: (S)-1-(2-methyl-2'-(trifluoromethyl)-[3,4'-bipyridin]-6-yl)ethanamine | LCMS tR = 0.86 min; MS m/z 282.1 (M + H). |
| 82: (S)-1-(5-fluoro-2'-(trifluoromethyl)-[3,4'-bipyridin]-6-yl)ethanamine | LCMS tR = 0.88 min; MS m/z 286.1 (M + H). |
| 83: (S)-1-(3-fluoro-4-((3,3,4-trimethylpiperazin-1-yl)methyl)phenyl)ethanamine | LCMS tR = 0.29 min; MS m/z 280.2 (M + H). |
| 84: (S)-1-(4-((4,4-difluoropiperidin-1-yl)methyl)-3-fluorophenyl)ethanamine | LCMS tR = 0.29 min; MS m/z 273.2 (M + H). |

### Examples

### Example 1: (S)-4-cyclopropyl-3-(6-fluoro-2-((S)-1-phenylethylamino)pyrimidin-4-yl)oxazolidin-2-one

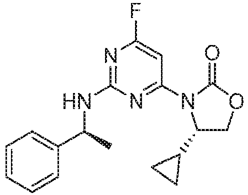

A solution of (S)-4-cyclopropyloxazolidin-2-one (0.054 g, 0.425 mmol) and (S)-4,6-difluoro-N-(1-phenylethyl)pyrimidin-2-amine (0.100 g, 0.425 mmol, 1.0 equiv) in DMF (2 mL) was treated with NaH (60 %, 0.034 g, 0.850 mmol, 2.0 equiv), then the resulting mixture (yellow) was stirred at room temperature for 1 h. The reaction mixture was diluted with EtOAc (20 mL), washed with saturated aqueous NaCl (2 x 20 mL), dried over Na₂SO₄, filtered and concentrated. Purification by reverse phase HPLC provided the trifluoroacetate salt of (S)-4-cyclopropyl-3-(6-fluoro-2-((S)-1-phenylethylamino)pyrimidin-4-yl)oxazolidin-2-one (0.017 g, white solid) in 8% yield. ¹H NMR (400 MHz, CDCI₃) δ 7.33 - 7.31 (m, 4H), 7.26 - 7.21 (m, 1H), 7.07 (s, 1H), 5.03 (br m, 1H), 4.59 (br m, 1H), 4.30 (t, J = 8.3 Hz, 1H), 3.97 (br m, 1H), 1.55 (d, J = 6.9 Hz, 3H), 0.85 (br m, 1H), 0.30 - 0.09 (br m, 4H); HRMS *m*/*z* 343.1566 (M + H)⁺; Rt-2.41 min.

### Example 2: (S)-3-(6-chloro-2-((S)-1-phenylethylamino)pyrimidin-4-yl)-4-cyclopropyloxazolidin-2-one

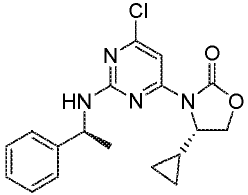

A solution of (S)-4-cyclopropyl-3-(2,6-dichloropyrimidin-4-yl)oxazolidin-2-one (70.0 mg, 0.255 mmol), (S)-(-)-1-phenylethanamine (0.033 mL, 0.255 mmol, 1.0 equiv), and N-ethyl-N-isopropylpropan-2-amine (0.067 mL, 0.383 mmol, 1.5 equiv) in DMSO (1.5 mL) was heated at 85 °C for 3 h. Purification by reverse phase HPLC provided the trifluoroacetate salt of (S)-3-(6-chloro-2-((S)-1-phenylethylamino)pyrimidin-4-yl)-4-cyclopropyloxazolidin-2-one (24.0 mg, white solid) in 20% yield. ¹H NMR (400 MHz, CDCl₃) δ 7.56 (s, 1H), 7.31 - 7.30 (m, 4H), 7.25 - 7.19 (m, 1H), 4.99 (br m, 1H), 4.66 (br m, 1H), 4.30 (t, J = 8.6 Hz, 1H), 3.94 - 3.92 (m, 1H), 1.56 (d, J = 7.0 Hz, 3H), 0.77 (br m, 1H), 0.20 (br m, 2H), 0.08 (br m, 2H); LCMS m/z 359.3 (M + H)⁺; Rt-1.07 min.

The compounds in **Table 39** were prepared using methods similar to those described for the preparation of **Example 2.**

**Table 39.**

| | | |
|---|---|---|
| 2 | 3 | 4 |
| 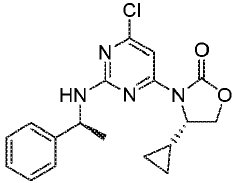 | 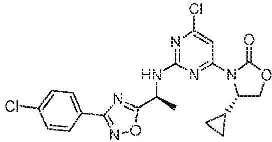 | 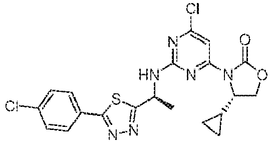 |
| 5 | 6 | 7 |
| 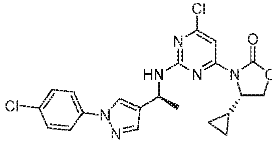 | 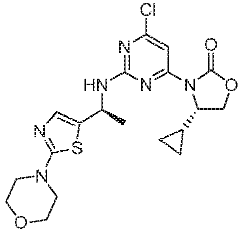 | 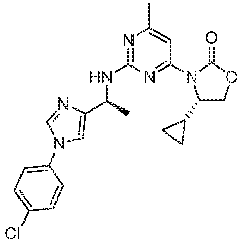 |
| 8 | 9 | 10 |
| 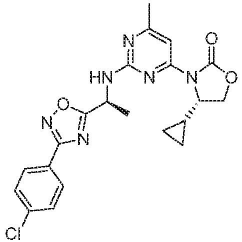 | 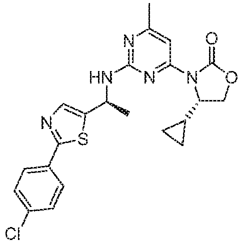 | 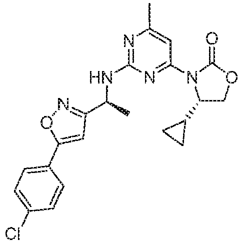 |
| 11 | 12 | 13 |
| 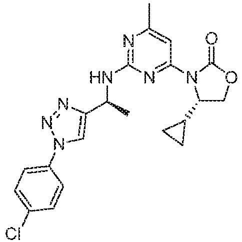 | 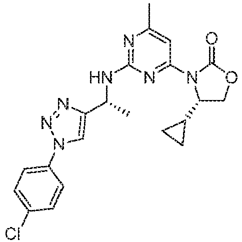 | 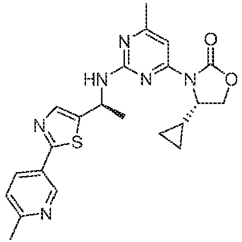 |
| 14 | 15 | 16 |
| 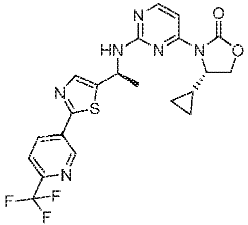 | 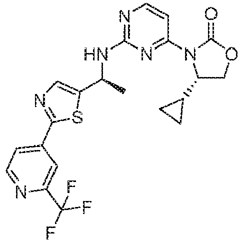 | 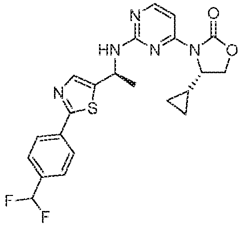 |
| 17 | 18 | 19 |
| 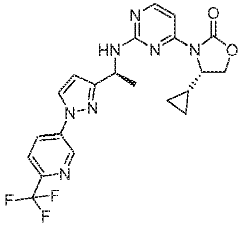 | 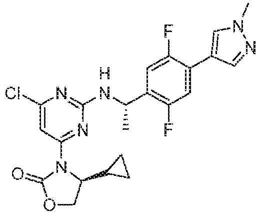 | 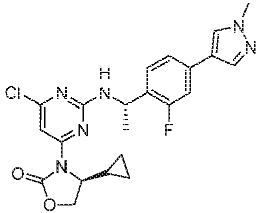 |
| 20 | 21 | 22 |
| 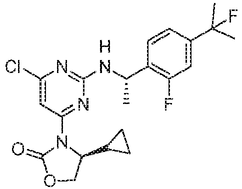 | 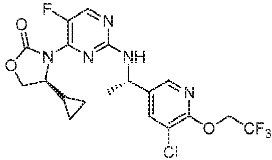 | 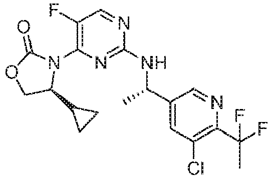 |
| 23 | 24 | 25 |
| 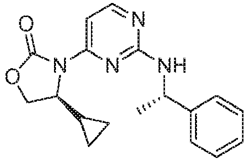 | 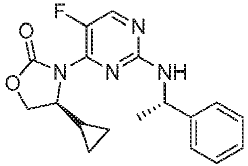 | 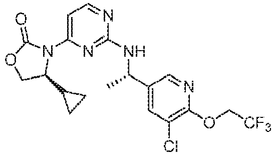 |
| 26 | 27 | 28 |
| 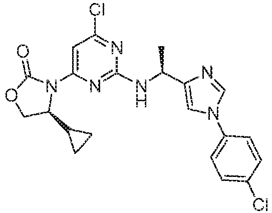 | 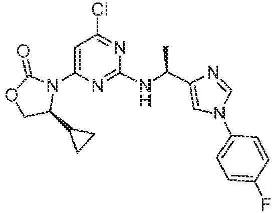 | 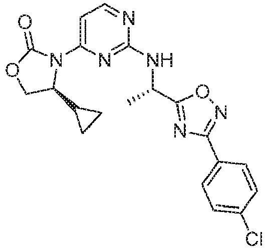 |
| 29 | | |
| 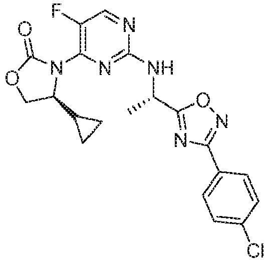 | | |

**Table 40. Chemical name, NMR chemical shifts and MS signal for each compound listed in Table 39.**

| **Example: Name** | **¹H NMR (400 MHz, CDCl₃) δ ppm** | **MS** |
|---|---|---|
| 2: (S)-3-(6-chloro-2-((S)-1-phenylethylamino)pyrimidi n-4-yl)-4-cyclopropyloxazolidin-2-one | 7.56 (s, 1H), 7.31 - 7.30 (m, 4H), 7.25 - 7.19 (m, 1H), 4.99 (br m, 1H), 4.66 (br m, 1H), 4.30 (t, J = 8.6 Hz, 1H), 3.94 - 3.92 (m, 1H), 1.56 (d, J = 7.0 Hz, 3H), 0.77 (br m, 1H), 0.20 (br m, 2H), 0.08 (br m, 2H) | LCMS *m*/*z* 359.3 (M + H)⁺; Rt-1.07 min |
| 3: (S)-3-(6-chloro-2-((S)-1-(3-(4-chlorophenyl)-1,2,4-oxadiazol-5-yl)ethylamino)pyrimidin-4-yl)-4-cyclopropyloxazolidin-2-one | 7.99 (d, J = 8.6 Hz, 2H), 7.65 (s, 1H), 7.46 (d, J = 8.6 Hz, 2H), 5.39 (br m, 1H), 4.70 (br m, 1H), 4.33 (t, J = 8.4 Hz, 1H), 4.02 - 4.00 (m, 1H), 1.77 (d, J = 7.1 Hz, 3H), 1.02 (br m, 1H), 0.31 - 0.18 (m, 4H) | HRMS (A) *m*/*z* 461.0899 (M + H)⁺; Rt-2.71 min |
| 4: (S)-3-(6-chloro-2-(((S)-1-(5-(4-chlorophenyl)-1,3,4-thiadiazol-2-yl)ethyl)amino)pyrimidin-4-yl)-4-cyclopropyloxazolidin-2-one | 7.84 (d, J = 8.6 Hz, 2H), 7.65 (s, 1H), 7.46 (d, J = 8.7 Hz, 2H), 5.54 (m, 1H), 4.71 (m, 1H), 4.34 (t, J = 8.3 Hz, 1H), 4.03 (m, 1H), 1.80 (d, J = 6.9 Hz, 3H), 1.00 - 0.77 (m, 1H), 0.51 - 0.03 (m, 4H) | HRMS (A) *m*/*z* 477.0677 (M + H)⁺; Rt-2.55 min |
| 5: (S)-3-(6-chloro-2-(((S)-1-(1-(4-chlorophenyl)-1H-pyrazol-4-yl)ethyl)amino)pyrimidin-4-yl)-4-cyclopropyloxazolidin-2-one | 7.85 (s, 1H), 7.71 (s, 1H), 7.62 (s, 1H), 7.57 (d, J = 8.9 Hz, 2H), 7.43 (d, J = 8.9 Hz, 2H), 5.20 (m, 1H), 4.62 (m, 1H), 4.38 (dd, J = 8.9, 8.0 Hz, 1H), 4.10 (dd, J = 8.9, 2.1 Hz, 1H), 1.62 (d, J = 6.9 Hz, 3H), 1.18 (m, 1H), 0.44 - 0.21 (m, 4H) | HRMS (A) *m*/*z* 459.1102 (M + H)⁺; Rt-2.67 min |
| 6: (S)-3-(6-chloro-2-(((S)-1-(2-morpholinothiazol-5-yl)ethyl)amino)pyrimidin-4-yl)-4-cyclopropyloxazolidin-2-one | 7.63 (s, 1H), 7.31 (s, 1H), 5.21 (m, 1H), 4.55 (m, 1H), 4.41 - 4.36 (m, 1H), 4.15 - 4.12 (m, 1H), 3.86 - 3.84 (m, 4H), 3.59 (m, 4H), 1.65 (d, J = 6.7 Hz, 3H), 1.24 (dd, J = 12.7, 7.0 Hz, 1H), 0.60 - 0.28 (m, 4H) | HRMS (A) *m*/*z* 451.1326 (M + H)⁺; Rt-1.92 min |
| 7: (S)-3-(2-(((S)-1-(1-(4-chlorophenyl)-1H-imidazol-4-yl)ethyl)amino)-6-methylpyrimidin-4-yl)-4-cyclopropyloxazolidin-2-one | 10.99 (d, J = 7.6 Hz, 1H), 8.46 (s, 1H), 7.65 (s, 1H), 7.60 (s, 1H), 7.56 (d, J = 8.7 Hz, 2H), 7.42 (d, J = 8.7 Hz, 2H), 5.69 - 5.57 (m, 1H), 4.79 (t, J = 6.9 Hz, 1H), 4.46 (t, J = 8.3 Hz, 1H), 4.22 (dd, J = 9.0, 1.3 Hz, 1H), 2.55 (s, 3H), 1.71 (d, J = 6.8 Hz, 3H), 1.17 (dt, J = 7.7, 5.1 Hz, 1H), 0.42 (m, 2H), 0.31 (m, 1H), 0.24 (m, 1H) | HRMS (A) *m*/*z* 439.1649 (M + H)⁺; 1.59 Rt-min |
| 8: (S)-3-(2-(((S)-1-(3-(4-chlorophenyl)-1,2,4-oxadiazol-5-yl)ethyl)amino)-6-methylpyrimidin-4-yl)-4-cyclopropyloxazolidin-2-one | 11.33 (d, J = 5.7 Hz, 1H), 7.98 (d, J = 8.8 Hz, 2H), 7.65 (s, 1H), 7.46 (d, J = 8.8 Hz, 2H), 5.37 - 5.28 (m, 1H), 4.78 (ddd, J = 7.6, 5.6, 1.9 Hz, 1H), 4.31 (dd, J = 9.2, 7.8 Hz, 1H), 3.97 (dd, J = 9.2, 1.9 Hz, 1H), 2.58 (s, 3H), 1.88 (d, J = 7.2 Hz, 3H), 0.93 (m, 1H), 0.26 (m, 2H), 0.11 (m, 2H) | HRMS (A) *m*/*z* 441.1440 (M + H)⁺; Rt-2.34 min |
| 9: (S)-3-(2-(((S)-1-(2-(4-chlorophenyl)thiazol-5-yl)ethyl)amino)-6-methylpyrimidin-4-yl)-4-cyclopropyloxazolidin-2-one | 10.94 (d, J = 6.9 Hz, 1H), 7.81 - 7.76 (m, 3H), 7.63 (s, 1H), 7.43 (d, J = 8.6 Hz, 2H), 5.42 (m, 1H), 4.89 - 4.83 (m, 1H), 4.42 - 4.35 (m, 1H), 4.05 (dd, J = 9.2, 1.7 Hz, 1H), 2.55 (s, 3H), 1.79 (d, J = 7.0 Hz, 3H), 1.13 - 1.09 (m, 1H), 0.47 (m, 1H), 0.39 (m, 1H), 0.23 (m, 2H) | HRMS (A) *m*/*z* 456.1266 (M + H)⁺; Rt-2.24 min |
| 10: (S)-3-(2-(((S)-1-(5-(4-chlorophenyl)isoxazol-3-yl)ethyl)amino)-6-methylpyrimidin-4-yl)-4-cyclopropyloxazolidin-2-one | 10.92 (d, J = 6.9 Hz, 1H), 7.69 (d, J = 8.7 Hz, 2H), 7.60 (s, 1H), 7.44 (d, J = 8.7 Hz, 2H), 6.67 (s, 1H), 5.33 (quin, J = 7.0 Hz, 1H), 4.92 - 4.85 (m, 1H), 4.39 (dd, J = 9.1, 7.7 Hz, 1H), 4.08 (dd, J = 9.1, 2.0 Hz, 1H), 2.53 (s, 3H), 1.72 (d, J = 7.1 Hz, 3H), 1.18 (tq, J = 8.3, 5.6 Hz, 1H), 0.50 (m, 1H), 0.36 (m, 1H), 0.28 (m, 2H) | HRMS (A) *m*/*z* 440.1487 (M + H)⁺; Rt-2.12 min |
| 11: (S)-3-(2-(((S)-1-(1-(4-chlorophenyl)-1H-1,2,3-triazol-4-yl)ethyl)amino)-6-methylpyrimidin-4-yl)-4-cyclopropyloxazolidin-2-one | 10.90 (d, J = 8.0 Hz, 1H), 8.19 (s, 1H), 7.69 (d, J = 9.0 Hz, 2H), 7.60 (s, 1H), 7.51 (d, J = 9.0 Hz, 2H), 5.54 (quin, J = 7.0 Hz, 1H), 4.80 (m, 1H), 4.43 (dd, J = 9.0, 7.8 Hz, 1H), 4.14 (dd, J = 9.1, 2.1 Hz, 1H), 2.54 (s, 3H), 1.73 (d, J = 7.0 Hz, 3H), 1.23 (m, 1H), 0.53 (m, 1H), 0.44 (m, 1H), 0.32 | HRMS (A) *m*/*z* 440.1601 (M + H)⁺; Rt-1.80 min |
| | (qt, J = 9.2, 4.8 Hz, 2H) | |
| 12: (S)-3-(2-(((R)-1-(1-(4-chlorophenyl)-1H-1,2,3-triazol-4-yl)ethyl)amino)-6-methylpyrimidin-4-yl)-4-cyclopropyloxazolidin-2-one | 8.20 (s, 1H), 7.70 (d, J = 9.0 Hz, 2H), 7.59 (s, 1H), 7.51 (d, J = 8.9 Hz, 2H), 5.54 (m, 1H), 4.75 (m, 1H), 4.43 (m, 1H), 4.23 (dd, J = 9.0, 2.0 Hz, 1H), 2.52 (s, 3H), 1.74 (7.0 Hz, 3H), 1.37 (m, 1H), 0.79 (m, 2H), 0.66 (m, 1H), 0.48 (m, 1H) | HRMS (A) *m*/*z* 440.1606 (M + H)⁺; Rt-1.82 min |
| 13: (S)-4-cyclopropyl-3-(6-methyl-2-(((S)-1-(2-(6-methylpyridin-3-yl)thiazol-5-yl)ethyl)amino)pyrimidin-4-yl)oxazolidin-2-one | 11.20 (d, J = 6.9 Hz, 1H), 9.23 (d, J = 1.7 Hz, 1H), 8.54 (dd, J = 8.3, 2.0 Hz, 1H), 7.83 (s, 1H), 7.65 (s, 1H), 7.59 (d, J = 8.4 Hz, 1H), 5.48 (quin, J = 6.9 Hz, 1H), 4.80 (m, 1H), 4.41 (dd, J = 8.9, 7.9 Hz, 1H), 4.10 (dd, J = 9.1, 1.8 Hz, 1H), 2.83 (s, 3H), 2.56 (s, 3H), 1.81 (d, J = 6.9 Hz, 3H), 1.11 (dtd, J = 8.3, 5.4, 2.9 Hz, 1H), 0.44 (m, 2H), 0.24 (m, 2H) | HRMS (A) *m*/*z* 437.1763 (M + H)⁺; Rt-1.46 min |
| 14: (S)-4-cyclopropyl-3-(2-(((S)-1-(2-(6-(trifluoromethyl)pyridin-3-yl)thiazol-5-yl)ethyl)amino)pyrimidin-4-yl)oxazolidin-2-one | 11.06 (d, J = 6.4 Hz, 1H), 9.20 (d, J = 2.0 Hz, 1H), 8.35 (dd, J = 8.2, 1.6 Hz, 1H), 8.01 (d, J = 7.1 Hz, 1H), 7.86 - 7.82 (m, 2H), 7.78 (dd, J = 8.2, 0.7 Hz, 1H), 5.50 (m, 1H), 4.86 (m, 1H), 4.41 (dd, J = 9.2, 7.7 Hz, 1H), 4.09 (dd, J = 9.2, 1.9 Hz, 1H), 1.83 (d, J = 7.0 Hz, 3H), 1.13 (tq, J = 8.3, 5.4 Hz, 1H), 0.50 (m, 1H), 0.43 (m, 1H), 0.27 (m, 2H) | HRMS (A) *m*/*z* 477.1327 (M + H)⁺; Rt-2.11 min |
| 15: (S)-4-cyclopropyl-3-(2-(((S)-1-(2-(2-(trifluoromethyl)pyridin-4-yl)thiazol-5-yl)ethyl)amino)pyrimidin-4-yl)oxazolidin-2-one | 11.11 (d, J = 5.8 Hz, 1H), 8.82 (d, J = 5.1 Hz, 1H), 8.15 (s, 1H), 8.00 (d, J = 7.1 Hz, 1H), 7.92 (dd, J = 5.0, 1.5 Hz, 1H), 7.87 (s, 1), 7.84 (d, J = 7.1 Hz, 1H), 5.51 (quin, J = 6.9 Hz, 1H), 4.85 (m, 1H), 4.41 (dd, J = 9.2, 7.7 Hz, 1H), 4.09 (dd, J = 9.2, 1.9 Hz, 1H), 1.83 (d, J = 7.0 Hz, 3H), 1.10 (m, 1H), 0.53 - 0.39 (m, 2H), 0.27 (m, 2H) | HRMS (A) *m*/*z* 477.1321 (M + H)⁺; Rt-2.08 min |
| 16: (S)-4-cyclopropyl-3-(2-(((S)-1-(2-(4-(difluoromethyl)phenyl)thiazol-5-yl)ethyl)amino)pyrimidin-4-yl)oxazolidin-2-one | 11.00 (br s, 1H), 8.01 - 7.92 (m, 3H), 7.82 (d, J = 7.0 Hz, 1H), 7.76 (s, 1H), 7.59 (d, J = 8.1 Hz, 2H), 6.68 (t, J = 56 Hz, 1H), 5.46 (m, 1H), 4.88 (m, 1H), 4.39 (m, 1H), 4.05 (m, 1H), 1.81 (d, J = 6.9 Hz, 3H), 1.10 (m, 1H), 0.49 (m, 1H), 0.40 (m, 1H), 0.25 (m, 2H) | HRMS (A) *m*/*z* 458.1468 (M + H)⁺; Rt-2.12 min |
| 17: (S)-4-cyclopropyl-3-(2-(((S)-1-(1-(6-(trifluoromethyl)pyridin-3-yl)-1H-pyrazol-3-yl)ethyl)amino)pyrimidin-4-yl)oxazolidin-2-one | 10.65 (d, J=7.8 Hz, 1 H), 9.06 (d, J=2.2 Hz, 1 H), 8.20 (dd, J=8.5, 2.4 Hz, 1 H), 7.94 - 8.00 (m, 2 H), 7.79 (d, J=7.2 Hz, 2 H), 6.65 (d, J=2.6 Hz, 1 H), 5.41 (quin, J=7.2 Hz, 1 H), 4.72 (td, J=7.4, 1.9 Hz, 1 H), 4.44 (dd, J=9.0, 7.7 Hz, 1 H), 4.17 (dd, J=9.0, 1.9 Hz, 1 H), 1.71 (d, J=7.0 Hz, 3 H), 1.17 - 1.29 (m, 1 H), 0.35 - 0.44 (m, 3 H), 0.21 - 0.31 (m, 1 H) | HRMS (A) *m*/*z* 460.1714 (M + H)⁺; Rt-1.85 min |
| 18: (S)-3-(6-chloro-2-(((S)-1-(2,5-difluoro-4-(1-methyl-1H-pyrazol-4-yl)phenyl)ethyl)amino) pyrimidin-4-yl)-4-cyclopropyl oxazolidin-2-one | 0.06 - 0.25 (m, 4 H) 0.84 (br. s., 1 H) 1.57 (d, J=6.94 Hz, 3 H) 3.98 (d, J=8.85 Hz, 1 H) 4.01 (s, 3 H) 4.34 (t, J=8.44 Hz, 1 H) 4.72 (br. s., 1 H) 5.27 (d, J=5.48 Hz, 1 H) 7.12 (dd, J=10.88, 6.28 Hz, 1 H) 7.19 (dd, J=10.56, 6.02 Hz, 1 H) 7.60 (s, 1 H) 7.78 (d, J=2.25 Hz, 1 H) 7.88 (s, 1 H) | HRMS (A) *m*/*z* 475.1454 (M + H)⁺; Rt-2.35 min |
| 19: (S)-3-(6-chloro-2-(((S)-1-(2-fluoro-4-(1-methyl-1H-pyrazol-4-yl) phenyl)ethyl)amino) pyrimidin-4-yl)-4-cyclo propyloxazolidin-2-one | 0.02 - 0.25 (m, 4 H) 0.73 - 0.92 (m, 1 H) 1.57 (d, *J*=6.94 Hz, 3 H) 3.97 (d, *J*=11.88 Hz, 1 H) 4.00 (s, 3 H) 4.33 (t, *J*=8.44 Hz, 1 H) 4.74 (br. s., 1 H) 5.32 (br. s., 1 H) 7.11 (dd, *J*=11.37, 1.54 Hz, 1 H) 7.19 (dd, *J*=8.02, 1.61 Hz, 1 H) 7.34 (t, *J*=7.95 Hz, 1 H) 7.58 (s, 1 H) 7.60 (s, 1 H) 7.80 (s, 1 H) | HRMS (A) *m*/*z* 457.1560 (M + H)⁺; Rt-2.29 min |
| 20: (S)-3-(6-chloro-2-(((S)-1-(2-fluoro-4-(2-fluoropropan-2-yl)phenyl) ethyl)amino)pyrimidin-4-yl)-4-cyclopropyl oxazolidin-2-one | 0.04 - 0.26 (m, 3 H) 0.74 - 0.94 (m, 1 H) 1.50 - 1.55 (m, 3 H) 1.61 (d, *J*=5.72 Hz, 3 H) 1.66 (d, *J*=5.72 Hz, 3 H) 4.00 (br. s., 1 H) 4.32 (t, *J*=8.39 Hz, 1 H) 4.62 (br. s., 1 H) 5.28 (br. s., 1 H) 5.53 (br. s., 1 H) 7.03 - 7.12 (m, 2 H) 7.28 - 7.34 (m, 1 H) 7.50 (s, 1 H) | HRMS (A) *m*/*z* 437.1555 (M + H)⁺; Rt-2.70 min |
| 21: (S)-3-(2-((S)-1-(5-chloro-6-(2,2,2-trifluoroethoxy)pyridin-3-yl)ethylamino)-5-fluoropyrimidin-4-yl)-4-cyclopropyloxazolidin-2-one | (CD3OD) 8.25 (d, J=2.0 Hz, 1 H), 8.09 (d, J=2.0 Hz, 1 H), 7.84 (d, J=2.0 Hz, 1 H), 4.85 - 4.94 (m, 3 H), 4.63 (t,J=8.8 Hz, 1 H), 4.23 (t, J=8.0 Hz, 1 H), 4.02 (br. d, J=7.8 Hz, 1 H), 1.50 (d, J=7.0 Hz, 3 H), 0.80 (br. s., 1 H), 0.31 (br. s., 1 H), -0.08 - 0.22 (m, 3H) | HRMS (A) m/z 476.1118 (M + H)+ RT=2.43 min. |
| 22: (S)-3-(2-((S)-1-(5-chloro-6-(1,1-difluoroethyl)pyridin-3-yl)ethylamino)-5-fluoropyrimidin-4-yl)-4-cyclopropyloxazolidin-2-one | (CD3OD) 8.56 (d, J=1.2 Hz, 1 H), 8.31 (br. s., 1 H), 7.99 (d, J=1.6 Hz, 1 H), 5.01 (br. d, J=6.7 Hz, 1 H), 4.67 (t, J=8.6 Hz, 1 H), 4.27 (t, J=7.8 Hz, 1 H), 4.04 (br. s., 1 H), 2.06 (t, J=18.8 Hz, 3 H), 1.58 (d, J=7.0 Hz, 3 H), 0.82 (br. s., 1 H), 0.25 - 0.43 (m, 2 H), -0.12 -0.22 (m, 2 H) | HRMS (A) m/z 442.1258 (M + H)+. RT=2.21 min. |
| 23: (S)-4-cyclopropyl-3-(2-((S)-1-phenylethylamino)pyrimidi n-4-yl)oxazolidin-2-one | (CD3OD) 7.95 (d, J=5.1 Hz, 1 H), 7.49 (d, J=7.0 Hz, 1 H), 7.10 - 7.27 (m, 4 H), 7.01 - 7.09 (m, 1 H), 4.97 (br. s., 1 H), 4.59 - 4.74 (m, 1 H), 4.21 (t, J=8.4 Hz, 1 H), 3.89 (br. s., 1 H), 1.41 (d, J=6.7 Hz, 3 H), 0.67 (br. s., 1 H), -0.12 -0.25 (m, 4 H) | HRMS (A) m/z 325.1657 (M + H)+ RT=1.72 min. |
| 24: (S)-4-cyclopropyl-3-(5-fluoro-2-((S)-1-phenylethylamino)pyrimidi n-4-yl)oxazolidin-2-one | (CD3OD) 8.27 (d, J=2.3 Hz, 1 H), 7.37 - 7.42 (m, 2 H), 7.28 - 7.36 (m, 2 H), 7.19 - 7.26 (m, 1 H), 4.86 - 5.00 (m, 19 H), 4.65 (t, J=8.8 Hz, 1 H), 4.24 (t, J=8.0 Hz, 1 H), 4.05 (d, J=8.2 Hz, 1 H), 1.53 (d, J=7.0 Hz, 3 H), 0.79 (br. s., 1 H), 0.31 (br. s., 1 H), 0.00 (br. s., 1 H) | HRMS (A) m/z 343.1561 (M + H)+. RT=2.15 min. |
| 25: (S)-3-(2-((S)-1-(5-chloro-6-(2,2,2-trifluoroethoxy)pyridin-3-yl)ethylamino)pyrimidin-4-yl)-4-cyclopropyloxazolidin-2-one | (CD3OD) 7.88 - 8.13 (m, 2 H), 7.72 (d, J=2.0 Hz, 1 H), 7.33 (d, J=6.3 Hz, 1 H), 4.94 (d, J=7.0 Hz, 1 H), 4.74 (q, J=8.6Hz, 2 H), 4.63 (br. s., 1 H), 4.23 (t, J=8.4 Hz, 1 H), 3.93 (d, J=8.2 Hz, 1 H), 1.40 (d, J=7.0 Hz, 3 H), 0.78 (br. s., 1 H), -0.13 - 0.33 (m, 4 H) | HRMS (A) m/z 458.1212, (M+H)+, RT=2.18 min. |
| 26: (S)-3-(6-chloro-2-(((S)-1-(1-(4-chlorophenyl)-1 H-imidazol-4-yl)ethyl)amino)pyrimidin-4-yl)-4-cyclopropyloxazolidin-2-one | (CD3OD) 8.90 (br. s., 1 H), 7.63 (s, 1 H), 7.38 - 7.46 (m, 2 H), 7.31 - 7.38 (m, 2 H), 6.95 (s, 1 H), 4.52 - 4.66 (m, 1 H),4.16 - 4.25 (m, 1 H), 4.12 (dd, J=8.2, 2.3 Hz, 1 H), 3.96 (dd, J=8.6, 2.7 Hz, 1 H), 1.38 (d, J=6.7 Hz, 3 H), 0.89 (dt, J=8.2, 4.9 Hz, 1 H), 0.51 (dd,J=9.6, 5.3 Hz, 1 H), 0.15 - 0.38 (m, 2 H), 0.03 (dd, J=9.8, 5.1 Hz, 1 H) | HRMS (A) m/z 459.1107, (M+H)+, RT=2.02 min. |
| 27: (S)-3-(6-chloro-2-(((S)-1-(1-(4-fluorophenyl)-1H-imidazol-4-yl)ethyl)amino)pyrimidin-4-yl)-4-cyclopropyloxazolidin-2-one | (CD3OD) 9.08 (br. s., 1 H), 7.77 (s, 1 H), 7.56 - 7.66 (m, 2 H), 7.33 (s, 1 H), 7.27 (t, J=8.6 Hz, 2 H), 5.23 (br. s., 1 H),4.66 - 4.90 (m, 1 H), 4.51 (br. s., 1 H), 4.32 (t, J=8.4 Hz, 1 H), 4.08 (br. s., 1 H), 1.57 (d, J=7.0 Hz, 3 H), 0.87 - 1.40 (m, 1 H), -0.11 - 0.82 (m, 4 H) | HRMS (A) m/z 443.1392, (M+H)+, RT=1.74 min. |
| 28: (S)-3-(2-((S)-1-(3-(4-chlorophenyl)-1,2,4-oxadiazol-5-yl)ethylamino)pyrimidin-4-yl)-4-cyclopropyloxazolidin-2-one | (CD3OD) 8.05 (br. s., 1 H), 7.81 (d, J=8.6 Hz, 2 H), 7.45 (br. s., 1 H), 7.33 (d, J=8.2 Hz, 2 H), 5.34 (q, J=7.0 Hz, 1 H),4.61 (br. s., 2 H), 4.19 (t, J=8.2 Hz, 1 H), 3.90 (d, J=8.2 Hz, 1 H), 1.59 (d, J=7.0 Hz, 3 H), 0.85 (br. s., 1 H), -0.13 - 0.32 (m, 4 H) | HRMS (A) m/z 427.1291, (M+H)+, RT=1.32 min. |
| 29: (S)-3-(2-((S)-1-(3-(4-chlorophenyl)-1,2,4-oxadiazol-5-yl)ethylamino)-5-fluoropyrimidin-4-yl)-4-cyclopropyloxazolidin-2-one | (CD3OD) 8.28 (br. s., 1 H), 7.98 (d, J=8.6 Hz, 2 H), 7.48 (d, J=8.6 Hz, 2 H), 5.30 (d, J=7.0 Hz, 1 H), 4.81 (s, 1 H), 4.58(t, J=8.6 Hz, 1 H), 4.19 (t, J=8.0 Hz, 1 H), 4.08 (q, J=8.0 Hz, 1 H), 1.68 (d, J=7.0 Hz, 3 H), 0.86 (br. s., 1 H), 0.26 (br. s., 2 H), 0.00 (br. s., 2 H) | HRMS (A) m/z 445.1193, (M+H)+, RT=2.40 min. |

### Example 30: (S)-4-cyclopropyl-3-(2-(((S)-1-(4-((4-(dimethylamino)piperidin-1-yl)methyl)-3-fluorophenyl)ethyl)amino)pyrimidin-4-yl)-4-methyloxazolidin-2-one

### Step1: Preparation of (S)-4-cyclopropyl-3-(2-(((S)-1-(3-fluoro-4-(hydroxymethyl)phenyl)ethyl)amino)pyrimidin-4-yl)-4-methyloxazolidin-2-one

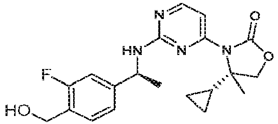

To a solution of of (S)-4-cyclopropyl-3-(2-fluoropyrimidin-4-yl)-4-methyloxazolidin-2-one (280 mg, 1.18 mmol) and (S)-(4-(1-aminoethyl)-2-fluorophenyl)methanol (220 mg, 1.30 mmol) in DMSO (6 ml) was added DIPEA (1480 mg, 11.45 mmol) and the resulting solution was stirred at 110 °C for 2 h. The reaction mixture was diluted with EtOAc (20 mL) and washed with water (100 mL). After separation, the aqueous phase was extracted with EtOAc (3 x 20 mL). Combined organics were dried over Na₂SO₄, filtered and concentrated. The residue was purified by column chromatography (Redi 80g, 40 - 100% EtOAc/heptane) to give (S)-4-cyclopropyl-3-(2-(((S)-1-(3-fluoro-4-(hydroxymethyl)phenyl)ethyl)amino)pyrimidin-4-yl)-4-methyloxazolidin-2-one (350 mg, 0.91 mmol, 77%).
¹H NMR (400 MHz, CDCI₃) δ 8.18 (d, *J* = 5.8 Hz, 1H), 7.39 - 7.27 (m, 2H), 7.10 (dd, *J* = 7.9, 1.7 Hz, 1H), 7.01 (dd, *J* = 11.0, 1.7 Hz, 1H), 5.39 (s, 1H), 5.06 - 4.90 (m, 1H), 4.69 (d, *J* = 5.1 Hz, 2H), 1.56 - 1.46 (m, 3H), 3.67 (d, *J* = 8.9 Hz, 1H), 3.54 (d, *J* = 9.0 Hz, 1H), 1.86 - 1.74 (m, 1H), 1.71 (s, 3H), 1.59 (s, 1H), 1.26 (t, *J* = 7.1 Hz, 2H), 0.20 (s, 2H). MS m/z 387.2 (M+H).

### Step2: Preparation of 4-((S)-1-((4-((S)-4-cyclopropyl-4-methyl-2-oxooxazolidin-3-yl)pyrimidin-2-yl)amino)ethyl)-2-fluorobenzaldehyde

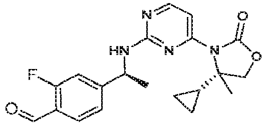

To a solution of (S)-4-cyclopropyl-3-(2-(((S)-1-(3-fluoro-4-(hydroxymethyl)phenyl)ethyl)amino)pyrimidin-4-yl)-4-methyloxazolidin-2-one (140 mg, 0.360 mmol) in DCM (15 mL) was added manganese (IV) oxide (1260 mg, 14.49 mmol). The resulting solution was stirred at room temperature for 2 h, filtered through nylon membrane (Whatman 0.45 um) and washed with ample DCM and MeOH. The filtrated was concentrated to give 4-((S)-1-((4-((S)-4-cyclopropyl-4-methyl-2-oxooxazolidin-3-yl)pyrimidin-2-yl)amino)ethyl)-2-fluorobenzaldehyde in quantitative yield which was used for the next reaction without further purification.

### Step3: Preparation of (S)-4-cyclopropyl-3-(2-(((S)-1-(4-((4-(dimethylamino)piperidin-1-yl)methyl)-3-fluorophenyl)ethyl)amino)pyrimidin-4-yl)-4-methyloxazolidin-2-one

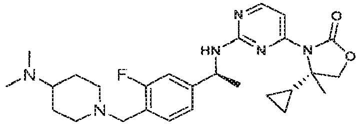

To a solution of 4-((S)-1-((4-((S)-4-cyclopropyl-4-methyl-2-oxooxazolidin-3-yl)pyrimidin-2-yl)amino)ethyl)-2-fluorobenzaldehyde (160 mg, 0.42 mmol) and N,N-dimethylpiperidine-4-amine (99 mg, 0.77 mmol) in MeOH (15 mL) was added acetic acid (37.5 mg, 0.62 mmol) and 5-ethyl-2-methylpyridine borane complex (79 mg, 0.58 mmol, sigma aldrich). The solution was stirred at 50 °C for 2 h followed by 16 h at room temperature. 5 drops of water were added and the solution was stirred at room temperature for 1 h. The solvents were removed by concentration and the crude material was purified through silica gel column chromatography (InterChim 12g, 0 - 20% 2N NH₃ in MeOH/EtOAc) to give (S)-4-cyclopropyl-3-(2-(((S)-1-(4-((4-(dimethylamino)piperidin-1-yl)methyl)-3-fluorophenyl)ethyl)amino)pyrimidin-4-yl)-4-methyloxazolidin-2-one as a white solid (126 mg, 0.25 mmol, 60.3% yield).
¹H NMR (400 MHz, MeOD) δ 8.16 (d, *J* = 5.9 Hz, 1H), 7.32 (t, *J* = 7.7 Hz, 1H), 7.21 (d, *J* = 5.8 Hz, 1H), 7.15 (dd, *J* = 7.9, 1.7 Hz, 1H), 7.07 (dd, *J* = 11.0, 1.7 Hz, 1H), 5.04 (q, *J* = 7.0 Hz, 1H), 3.79 (d, *J* = 9.1 Hz, 1H), 3.68 (d, *J* = 8.9 Hz, 1H), 3.52 (d, *J* = 1.5 Hz, 2H), 2.95 (dt, *J* = 11.3, 3.3 Hz, 2H), 2.28 (s, 6H), 2.19 (tt, *J* = 11.4, 3.7 Hz, 1H), 2.06 (t, *J* = 11.9 Hz, 2H), 1.84 (dd, *J* = 9.2, 6.3 Hz, 2H), 1.77 (s, 3H), 1.61 - 1.43 (m, 5H), 1.33 (s, 1H), 0.23 (s, 3H), 0.11 - -0.23 (m, 1H).
HRMS(B) *m*/*z* 497.3018 (M + H)⁺.

The examples in **Table 41** were prepared using a method similar to that described for the preparation of **Example 30.**

**Table 41.**

| | | |
|---|---|---|
| 31 | 32 | 33 |
| 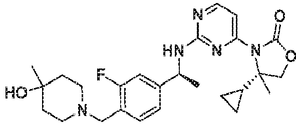 | 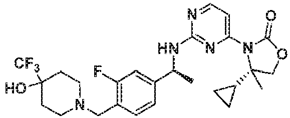 | 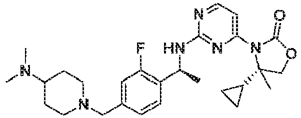 |
| 34 | 35 | 36 |
| 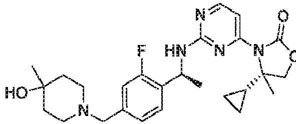 | 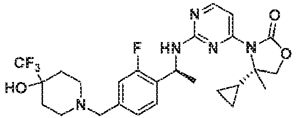 | 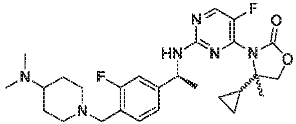 |
| 37 | 38 | 39 |
| 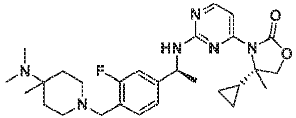 | 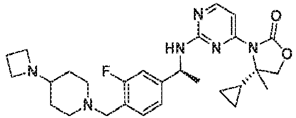 | 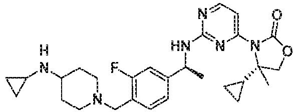 |
| 67 | 68 | 69 |
| 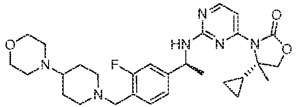 | 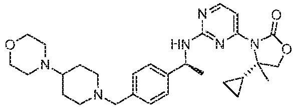 | 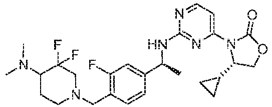 |

**Table 42. Chemical name, NMR chemical shifts and MS signal for each compound listed in Table 41.**

| Example: Name | 1NMR (400 MHz) ppm | HRMS Method |
|---|---|---|
| 31: (S)-4-cyclopropyl-3-(2-(((S)-1-(3-fluoro-4-((4-hydroxy-4-methylpiperidin-1-yl)methyl)phenyl)ethyl)amino)p yrimidin-4-yl)-4-methyloxazolidin-2-one | (MeOD) 8.15 (d, *J* = 5.9 Hz, 1H), 7.33 (t, *J* = 7.7 Hz, 1H), 7.21 (d, *J* = 5.8 Hz, 1H), 7.15 (dd, *J* = 7.8, 1.7 Hz, 1H), 7.07 (dd, *J* = 11.0, 1.7 Hz, 1H), 5.04 (q, *J* = 7.0 Hz, 1H), 3.78 (d, *J* = 9.0 Hz, 1H), 3.68 (d, *J* = 9.4 Hz, 1H), 3.55 (s, 2H), 2.54 (br s, 4H), 1.77 (s, 3H), 1.70 - 1.56 (m, 4H), 1.54 (d, *J* = 7.0 Hz, 3H), 1.32 (br s, 1H), 1.19 (s, 3H), 0.22 (br s, 3H), 0.06 - -0.24 (m, 1H). | (B) *m*/*z* 484.2708 (M + H)⁺ |
| 32: (S)-4-cyclopropyl-3-(2-(((S)-1-(3-fluoro-4-((4-hydroxy-4-(trifluoromethyl)piperidin-1-yl)methyl)phenyl)ethyl)amino)pyrimidin-4-yl)-4-methyloxazolidin-2-one | (MeOD) 8.15 (d, *J* = 5.8 Hz, 1H), 7.33 (t, *J* = 7.7 Hz, 1H), 7.21 (d, *J* = 5.8 Hz, 1H), 7.15 (dd, *J* = 7.8, 1.7 Hz, 1H), 7.07 (dd, *J* = 11.1, 1.8 Hz, 1H), 5.04 (q, *J* = 7.0 Hz, 1H), 3.78 (d, *J* = 9.1 Hz, 1H), 3.68 (d, *J* = 9.3 Hz, 1H), 3.56 (d, *J* = 1.4 Hz, 2H), 2.83 - 2.69 (m, 2H), 2.39 (td, *J* = 12.1, 2.6 Hz, 2H), 1.84 (ddt, *J* = 13.6, 11.9, 2.5 Hz, 2H), 1.77 (s, 3H), 1.70 (d, *J* = 13.3 Hz, 2H), 1.54 (d, *J* = 7.0 Hz, 3H), 1.32 (br s, 1H), 0.22 (s, 3H), 0.03 - -0.27 (m, 1H). | (B) *m*/*z* 520.2517 (M + H)⁺ |
| 33: (S)-4-cyclopropyl-3-(2-(((S)-1-(4-((4-(dimethylamino)piperidin-1-yl)methyl)-2-fluorophenyl)ethyl)amino)pyrim idin-4-yl)-4-methyloxazolidin-2-one | (MeOD) 8.17 (d, *J* = 5.8 Hz, 1H), 7.33 (t, *J* = 8.0 Hz, 1H), 7.23 (d, *J* = 5.8 Hz, 1H), 7.11 - 7.01 (m, 2H), 5.31 (q, *J* = 7.0 Hz, 1H), 3.80 (d, *J* = 9.1 Hz, 1H), 3.68 (d, *J* = 9.2 Hz, 1H), 3.46 (s, 2H), 2.94 (d, *J* = 11.0 Hz, 2H), 2.33 (s, 6H), 2.27 (d, *J* = 12.3 Hz, 1H), 2.03 (ddd, *J* = 12.0, 9.9, 2.5 Hz, 2H), 1.88 (d, *J* = 12.6 Hz, 2H), 1.79 (s, 3H), 1.56 (m, 5H), 1.31 (s, 1H), 0.22 (s, 3H), -0.18 (s, 1H) | (B) *m*/*z* 497.3026 (M + H)⁺ |
| 34: (S)-4-cyclopropyl-3-(2-(((S)-1-(2-fluoro-4-((4-hydroxy-4-methylpiperidin-1-yl)methyl)phenyl)ethyl)amino)p yrimidin-4-yl)-4-methyloxazolidin-2-one | (MeOD) 8.15 (d, *J* = 5.8 Hz, 1H), 7.32 (t, *J* = 8.0 Hz, 1H), 7.21 (d, *J* = 5.8 Hz, 1H), 7.13 - 7.00 (m, 2H), 5.30 (q, *J* = 6.9 Hz, 1H), 3.78 (d, *J* = 9.1 Hz, 1H), 3.66 (d, *J* = 8.9 Hz, 1H), 3.48 (s, 2H), 2.57 - 2.35 (m, 4H), 1.77 (s, 3H), 1.61 (q, *J* = 4.7 Hz, 4H), 1.53 (d, *J* = 7.0 Hz, 3H), 1.32 (br s, 1H), 1.21 (s, 3H), 0.20 (s, 3H), -0.20 (s, 1H) | (B) *m*/*z* 484.2710 (M + H)⁺ |
| 35: (S)-4-cyclopropyl-3-(2-(((S)-1-(2-fluoro-4-((4-hydroxy-4-(trifluoromethyl)piperidin-1-yl)methyl)phenyl)ethyl)amino)p yrimidin-4-yl)-4-methyloxazolidin-2-one | (MeOD) 8.15 (d, *J* = 5.8 Hz, 1H), 7.32 (t, *J* = 8.0 Hz, 1H), 7.21 (d, *J* = 5.8 Hz, 1H), 7.14 - 7.00 (m, 2H), 5.30 (q, *J* = 6.9 Hz, 1H), 3.78 (d, *J* = 9.1 Hz, 1H), 3.66 (d, *J* = 9.2 Hz, 1H), 3.49 (s, 2H), 2.80 - 2.65 (m, 2H), 2.41 - 2.26 (m, 2H), 1.84 (tdd, *J* = 12.7, 4.6, 2.5 Hz, 2H), 1.78 (s, 3H), 1.75 - 1.65 (m, 2H), 1.53 (d, *J* = 6.9 Hz, 3H), 1.29 (br s, 1H), 0.20 (s, 3H), - 0.20 (s, 1H) | (B) *m*/*z* 538.2437 (M + H)⁺ |
| 36: 4-cyclopropyl-3-(2-(((S)-1-(4-((4-(dimethylamino)piperidin-1-yl)methyl)-3-fluorophenyl)ethyl)amino)-5-fluoropyrimidin-4-yl)-4-methyloxazolidin-2-one | (MeOD) 8.25 (dd, *J* = 4.5, 2.8 Hz, 1H), 7.33 (td, *J* = 7.7, 3.1 Hz, 1H), 7.15 (dt, *J* = 7.8, 1.7 Hz, 1H), 7.07 (dd, *J* = 11.1, 1.7 Hz, 1H), 4.94 (q, *J* = 7.7 Hz, 1H), 4.03 - 3.90 (m, 2H), 3.62 - 3.52 (m, 2H), 2.96 (t, *J* = 9.1 Hz, 2H), 2.29 (s, 6H), 2.19 (s, 1H), 2.06 (t, *J* = 11.9 Hz, 2H), 1.85 (d, *J* = 12.3 Hz, 2H), 1.52 (t, *J* = 5.0 Hz, 5H), 1.45 (s, 1.5H), 1.25 (br s, 1.5H) 0.59 - 0.42 m, 2H), 0.36 (br s, 0.5H), 0.23 (s, 1H), 0.19--0.03 (m, 0.5H) | (G) *mlz* 515.2956 and 515.2952 (M + H)⁺ mixture of diastereom ers |
| 37: (S)-4-cyclopropyl-3-(2-(((S)-1-(4-((4-(dimethylamino)-4-methylpiperidin-1-yl)methyl)-3-fluorophenyl)ethyl)amino)pyrim idin-4-yl)-4-methyloxazolidin-2-one | (MeOD) δ 8.15 (d, *J* = 5.8 Hz, 1H), 7.33 (t, *J* = 7.7 Hz, 1H), 7.21 (d, *J* = 5.8 Hz, 1H), 7.14 (dd, *J* = 7.8, 1.7 Hz, 1H), 7.06 (dd, *J* = 11.1, 1.8 Hz, 1H), 5.03 (q, *J* = 7.0 Hz, 1H), 3.78 (d, *J* = 9.0 Hz, 1H), 3.68 (d, *J* = 9.3 Hz, 1H), 3.53 (d, *J* = 1.6 Hz, 2H), 2.74 (tt, *J* = 9.2, 4.2 Hz, 2H), 2.25 (m, 8H), 1.77 (s, 3H), 1.71 (dd, *J* = 10.5, 3.9 Hz, 2H), 1.61 (dt, *J* = 12.3, 3.0 Hz, 2H), 1.54 (d, *J* = 7.0 Hz, 3H), 1.40-1.15 (br s, 1H), 0.98 (s, 3H), 0.23 (br s, 3H), 0.04 - -0.28 (br s, 1H) | (B) *m*/*z* 511.3201 (M + H)⁺ |
| 38: (S)-3-(2-(((S)-1-(4-((4-(azetidin-1-yl)piperidin-1-yl)methyl)-3-fluorophenyl)ethyl)amino)pyrim idin-4-yl)-4-cyclopropyl-4-methyloxazolidin-2-one | (MeOD) δ 8.16 (d, *J* = 5.8 Hz, 1H), 7.31 (t, *J* = 7.7 Hz, 1H), 7.21 (d, *J* = 5.8 Hz, 1H), 7.14 (dd, *J =* 8.0, 1.7 Hz, 1H), 7.06 (d, *J* = 11.0 Hz, 1H), 5.03 (q, *J* = 7.0 Hz, 1H), 3.78 (d, *J* = 9.1 Hz, 1H), 3.69 (s, 1H), 3.52 (d, *J* = 1.5 Hz, 2H), 3.27 (t, *J* = 7.2 Hz, 4H), 2.87 (d, *J* = 10.7 Hz, 2H), 2.08 (qd, *J* = 13.4, 4.6 Hz, 5H), 1.77 (s, 3H), 1.73 (d, *J* = 12.4 Hz, 2H), 1.54 (d, *J =* 7.0 Hz, 3H), 1.36 - 1.18 (m, 2H + br s 1H), 0.22 (s, 3H), 0.05 - -0.24 (m, 1H) | (B) *m*/*z* 509.3029 (M + H)⁺ |
| 39: (S)-4-cyclopropyl-3-(2-(((S)-1-(4-((4-(cyclopropylamino)piperidin-1-yl)methyl)-3-fluorophenyl)ethyl)amino)pyrim idin-4-yl)-4-methyloxazolidin-2-one | (MeOD) δ 8.16 (d, *J* = 5.8 Hz, 1H), 7.32 (t, *J* = 7.7 Hz, 1H), 7.21 (d, *J* = 5.8 Hz, 1H), 7.15 (dd, *J* = 7.8, 1.7 Hz, 1H), 7.07 (d, *J* = 11.1 Hz, 1H), 5.04 (q, *J* = 6.9 Hz, 1H), 3.79 (d, *J* = 9.0 Hz, 1H), 3.69 (s, 1H), 3.56 - 3.50 (m, 2H), 2.89 (d, *J* = 11.4 Hz, 2H), 2.58 (ddt, *J* = 10.9, 7.1, 4.2 Hz, 1H), 2.21 - 2.05 (m, 3H), 1.94 (d, *J* = 12.3 Hz, 2H), 1.77 (s, 3H), 1.54 (d, *J* = 7.0 Hz, 3H), 1.52 - 1.35 (m, 2H + br s, 1H), 0.49 (td, *J* = 6.6, 4.6 Hz, 2H), 0.39 - 0.32 (m, 2H), 0.23 (br s, 3H), 0.06 - -0.24 (br s, 1H) | (B) *m*/*z* 509.3022 (M + H)⁺ |
| 67: (S)-4-cyclopropyl-3-(2-(((S)-1-(3-fluoro-4-((4-morpholinopiperidin-1-yl)methyl)phenyl)ethyl)amino)p yrimidin-4-yl)-4-methyloxazolidin-2-one | (MeOD) δ 8.16 (d, *J* = 5.8 Hz, 1H), 7.32 (t, *J* = 7.7 Hz, 1H), 7.21 (d, *J* = 5.8 Hz, 1H), 7.15 (dd, *J* = 7.8, 1.7 Hz, 1H), 7.07 (d, *J* = 10.9 Hz, 1H), 5.03 (t, *J* = 7.1 Hz, 1H), 3.79 (d, *J* = 9.0 Hz, 1H), 3.70 (m, *J* = 4.7 Hz, 5H), 3.53 (s, 2H), 2.96 (d, *J* = 11.4 Hz, 2H), 2.57 (t, *J* = 4.7 Hz, 4H), 2.24 - 2.11 (m, 1H), 2.07 (t, *J* = 11.7 Hz, 2H), 1.89 (d, *J* = 12.1 Hz, 2H), 1.77 (s, 3H), 1.60 - 1.45 (m, 5H), 1.31 (br s, 1H), 0.23 (br s, 3H), 0.04 - -0.27 br s, 1H) | (B) *m*/*z* 539.3180 (M + H)⁺ |
| 68: (S)-4-cyclopropyl-4-methyl-3-(2-(((S)-1-(4-((4-morpholinopiperidin-1-yl)methyl)phenyl)ethyl)amino)p yrimidin-4-yl)oxazolidin-2-one | (MeOD) 8.14 (d, *J* = 5.8 Hz, 1H), 7.30 (d, *J* = 8.3 Hz, 2H), 7.25 (d, *J* = 8.1 Hz, 2H), 7.19 (d, *J* = 5.8 Hz, 1H), 5.04 (q, *J* = 7.0 Hz, 1H), 3.77 (d, *J* = 9.1 Hz, 1H), 3.69 (q, *J* = 7.8, 6.2 Hz, 5H), 3.47 (s, 2H), 2.95 (d, *J* = 11.5 Hz, 2H), 2.65 - 2.50 (m, 4H), 2.19 (ddt, *J* = 11.5, 8.0, 3.9 Hz, 1H), 2.03 (t, *J* = 11.5 Hz, 2H), 1.89 (d, *J* = 12.4 Hz, 2H), 1.77 (s, 3H), 1.62 - 1.44 (m, 5H), 1.44 - 1.17 (br s, 1H), 0.19 (br s, 3H), -0.06 - -0.34 (br s, 1H) | (B) *m*/*z* 521.3236 (M + H)⁺ |
| 69: (4S)-4-cyclopropyl-3-(2-(((1S)-1-(4-((4-(dimethylamino)-3,3-difluoropiperidin-1-yl)methyl)-3-fluorophenyl)ethyl)amino)pyrim idin-4-yl)oxazolidin-2-one | (MeOD) 8.15 (d, *J* = 5.8 Hz, 1H), 7.42 - 7.30 (m, 2H), 7.20 (dd, *J* = 7.9, 1.7 Hz, 1H), 7.12 (dd, *J* = 11.1, 1.6 Hz, 1H), 5.15 - 5.00 (m, 1H), 4.72 (t, *J* = 7.2 Hz, 1H), 4.39 (t, *J* = 8.4 Hz, 1H), 4.09 (s, 1H), 3.63 (t, *J* = 6.3 Hz, 2H), 3.00 (dq, *J* = 12.2, 7.1, 5.1 Hz, 2H), 2.78 - 2.60 (m, 1H), 2.42 (s, 6H), 2.36 - 2.13 (m, 2H), 1.86 (q, *J* = 8.5, 6.1 Hz, 2H), 1.52 (d, *J* = 7.0 Hz, 3H), 0.96 (br s, 1H), 0.18 (br m, 4H) | (B) *m*/*z* 519.2673 (M + H)⁺ |

### Example 40: (S)-4-cyclopropyl-3-(2-(((S)-1-(3-fluoro-4-((4-(methylamino)piperidin-1-yl)methyl)phenyl)ethyl)amino)pyrimidin-4-yl)-4-methyloxazolidin-2-one

### Step 1: Preparation of tert-butyl (1-(4-((S)-1-((4-((S)-4-cyclopropyl-4-methyl-2-oxooxazolidin-3-yl)pyrimidin-2-yl)amino)ethyl)-2-fluorobenzyl)piperidin-4-yl)(methyl)carbamate

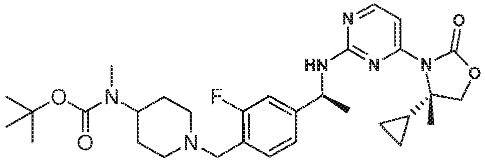

Title compound was prepared from 4-((S)-1-((4-((S)-4-cyclopropyl-4-methyl-2-oxooxazolidin-3-yl)pyrimidin-2-yl)amino)ethyl)-2-fluorobenzaldehyde and tert-butyl methyl(piperidin-4-yl)carbamate as a white solid (52 mg, 57% yield) following the procedure of example 32. ¹H NMR (400 MHz, CDCI3) δ 8.19 (d, *J* = 5.8 Hz, 1H), 7.34 - 7.22 (m, 2H), 7.08 (d, *J* = 7.8 Hz, 1H), 7.00 (d, *J* = 10.8 Hz, 1H), 5.76 (br s, 1H), 5.05 - 4.93 (m, 1H), 3.99 (br s, 1H), 3.70 (d, *J* = 8.7 Hz, 1H), 3.56 (d, *J* = 8.8 Hz, 1H), 3.49 (s, 2H), 2.92 (d, *J* = 11.0 Hz, 2H), 2.73 (s, 3H), 2.12 - 2.06 (m, 2H), 1.77 - 1.67 (m, 5H), 1.63 - 1.52 (m, 5H), 1.47 (s, 9H), 1.28 (t, *J* = 7.2 Hz, 1H), 0.20 (br s, 2H), 0.10 (br s, 1H), -0.05 (br s, 1H); MS m/z 583.3 (M + H).

### Step 2: Preparation of (S)-4-cyclopropyl-3-(2-(((S)-1-(3-fluoro-4-((4-(methylamino)piperidin-1-yl)methyl)phenyl)ethyl)amino)pyrimidin-4-yl)-4-methyloxazolidin-2-one

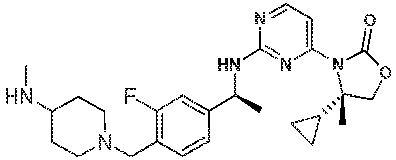

To a solution of tert-butyl (1-(4-((S)-1-((4-((S)-4-cyclopropyl-4-methyl-2-oxooxazolidin-3-yl)pyrimidin-2-yl)amino)ethyl)-2-fluorobenzyl)piperidin-4-yl)(methyl)carbamate (52 mg, 0.09 mmol) in DCM (1 mL) was added TFA (1 mL, 12 mmol) slowly at -78 °C. The reaction was stirred at room temperature for 1 h then was concentrated and diluted with DCM (10 mL). The solution was stirred with 3 eq. of MP-carbonate resin (3.28 mmol/g, Biotage) for 1 hr at room temperature. The resin was removed by filtration and washed (2 x 5 mL) with DCM. The filtrate was concentrated and purified through HPLC to give (S)-4-cyclopropyl-3-(2-(((S)-1-(3-fluoro-4-((4-(methylamino)piperidin-1-yl)methyl)phenyl)ethyl)amino)pyrimidin-4-yl)-4-methyloxazolidin-2-one as a white solid (31 mg, 72% yield).
1H NMR (400 MHz, MeOD) δ 8.14 (d, *J* = 5.8 Hz, 1H), 7.30 (t, *J* = 7.7 Hz, 1H), 7.20 (d, *J* = 5.8 Hz, 1H), 7.13 (dd, *J* = 7.9, 1.7 Hz, 1H), 7.05 (dd, *J* = 11.2, 1.8 Hz, 1H), 5.02 (q, *J* = 7.0 Hz, 1H), 3.77 (d, *J* = 9.0 Hz, 1H), 3.73 - 3.61 (m, 1H), 3.51 (s, 2H), 2.88 (d, *J* = 11.2 Hz, 2H), 2.40 - 2.34 (m, 4H), 2.14 - 2.03 (m, 2H), 1.89 (dd, *J* = 11.0, 2.2 Hz, 2H), 1.76 (s, 3H), 1.52 (d, *J* = 7.0 Hz, 3H), 1.38 (qd, *J* = 11.9, 3.4 Hz, 2H), 1.26 (br s, 1H), 0.20 (br s, 3H), -0.16 (br s, 1H); HRMS (B) m/z 483.2881 (M + H)⁺.

The examples in **Table 43** were prepared using a method similar to that described for the preparation of **Example 40.**

**Table 43.**

| | | |
|---|---|---|
| 41 | 42 | 43 |
| 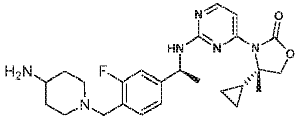 | 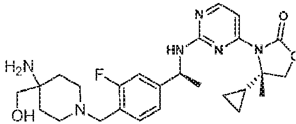 | 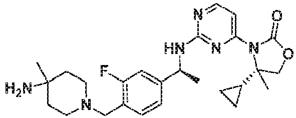 |
| 44 | | |
| 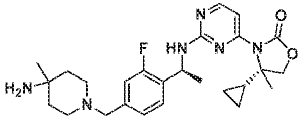 | | |

**Table 44. Chemical name, NMR chemical shifts and MS signal for each compound listed in Table 43.**

| Example: Name | 1NMR (400 MHz) ppm | HRMS Method |
|---|---|---|
| 41: (S)-3-(2-(((S)-1-(4-((4-aminopiperidin-1-yl)methyl)-3-fluorophenyl)ethyl)amino)pyrim idin-4-yl)-4-cyclopropyl-4-methyloxazolidin-2-one | (MeOD) 8.14 (d, *J* = 5.9 Hz, 1H), 7.30 (t, *J* = 7.7 Hz, 1H), 7.20 (d, *J* = 5.8 Hz, 1H), 7.13 (dd, *J* = 8.0, 1.7 Hz, 1H), 7.05 (dd, *J* = 11.2, 1.8 Hz, 1H), 5.02 (q, *J* = 7.0 Hz, 1H), 3.77 (d, *J* = 9.0 Hz, 1H), 3.71 - 3.61 (m, 1H), 3.51 (s, 2H), 2.94 - 2.79 (m, 2H), 2.63 (tt, *J* = 10.9, 4.1 Hz, 1H), 2.09 (tt, *J* = 11.9, 2.3 Hz, 2H), 1.80 (d, *J* = 12.7 Hz, 2H), 1.76 (s, 3H), 1.52 (d, *J* = 7.0 Hz, 3H), 1.48 - 1.35 (m, 2H), 1.27 (br s, 1H), 0.20 (br s, 3H), -0.17 (br s, 1H) | (B) m/z 469.2719 (M + H)⁺. |
| 42: (S)-3-(2-(((S)-1-(4-((4-amino-4-(hydroxymethyl)piperidin-1-yl)methyl)-3-fluorophenyl)ethyl)amino)pyrim | (MeOD) 8.14 (d, *J* = 5.9 Hz, 1H), 7.32 (t, *J* = 7.7 Hz, 1H), 7.19 (d, *J* = 5.8 Hz, 1H), 7.13 (dd, *J* = 7.9, 1.8 Hz, 1H), 7.05 (dd, *J* = 11.1, 1.8 Hz, 1H), 5.02 (q, *J* = 7.0 Hz, 1H), 3.77 (d, *J* = 9.0 Hz, 1H), 3.72 - 3.61 (m, 1H), 3.53 (s, | (B) m/z 499.2835 (M + H)⁺ |
| idin-4-yl)-4-cyclopropyl-4-methyloxazolidin-2-one | 2H), 3.35 (s, 2H), 2.57 - 2.44 (m, 4H, 1.75 (s, 3H), 1.70 - 1.59 (m, 2H), 1.52 (d, *J* = 7.0 Hz, 3H), 1.46 (dt, *J* = 13.4, 4.3 Hz, 4H), 1.25 (br s, 1H), 0.20 (br s, 4H), -0.17 (br s, 1H) | |
| 43: (S)-3-(2-(((S)-1-(4-((4-amino-4-methylpiperidin-1-yl)methyl)-3-fluorophenyl)ethyl)amino)pyrim idin-4-yl)-4-cyclopropyl-4-methyloxazolidin-2-one | (MeOD) 8.16 (d, *J* = 5.8 Hz, 1H), 7.33 (t, *J* = 7.8 Hz, 1H), 7.21 (d, *J* = 5.8 Hz, 1H), 7.15 (dd, *J* = 7.8, 1.7 Hz, 1H), 7.07 (dd, *J* = 11.1, 1.8 Hz, 1H), 5.04 (q, *J* = 7.0 Hz, 1H), 3.79 (d, *J* = 9.0 Hz, 1H), 3.69 (s, 1H), 3.55 (d, *J* = 1.2 Hz, 2H), 2.55 (d, *J* = 8.0 Hz, 2H), 2.46 (d, *J* = 10.2 Hz, 2H), 1.77 (s, 3H), 1.66 - 1.57 (m, 4H), 1.54 (d, *J* = 7.0 Hz, 3H), 1.14 (s, 3H), 0.23 (s, 3H), 0.04 - -0.31 (m, 1H) | (B) *m*/*z* 483.2852 (M + H)⁺. |
| 44: (S)-3-(2-(((S)-1-(4-((4-amino-4-methylpiperidin-1-yl)methyl)-2-fluorophenyl)ethyl)amino)pyrim idin-4-yl)-4-cyclopropyl-4-methyloxazolidin-2-one | (MeOD) 8.16 (d, *J* = 5.8 Hz, 1H), 7.32 (t, *J* = 8.0 Hz, 1H), 7.21 (d, *J* = 5.8 Hz, 1H), 7.12 - 7.02 (m, 2H), 5.30 (q, *J* = 7.0 Hz, 1H), 3.78 (d, *J* = 9.1 Hz, 1H), 3.66 (d, *J* = 9.0 Hz, 1H), 3.48 (s, 2H), 2.45 (d, *J* = 27.3 Hz, 4H), 1.78 (s, 3H), 1.67 - 1.56 (m, 4H), 1.53 (d, *J* = 6.9 Hz, 3H), 1.41 - 1.21 (br s, 1H), 1.15 (s, 3H), 0.20 (s, 3H), -0.21 (s, 1H) | (G) *m*/*z* 483.2885 (M + H)⁺ |

### Example 45 and 46:

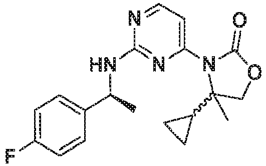

A solution of 3-(2-chloropyrimidin-4-yl)-4-cyclopropyl-4-methyloxazolidin-2-one (25 mg, 0.10 mmol) and (S)-1-(4-fluorophenyl)ethanamine (69 mg, 0.49 mmol) in DMSO was heated at 110 °C for 16 h. The reaction mixture was diluted with water (30 mL) and extrated with EtOAc (3 x 8 mL). Combined organics were dried over Na2SO4, filtered and concentrated. The residue was purified by column chromatography (InterChim 4g,10% - 50% EtOAc/Heptane) to give (S)-4-cyclopropyl-3-(2-((S)-1-(4-fluorophenyl)ethylamino)pyrimidin-4-yl)-4-methyloxazolidin-2-one and (R)-4-cyclopropyl-3-(2-((S)-1-(4-fluorophenyl)ethylamino)pyrimidin-4-yl)-4-methyloxazolidin-2-one. Example 45: first eluted product (7mg) ¹H NMR (400 MHz, MeOD) δ 8.17 - 8.08 (m, 1H), 7.38 - 7.25 (m, 2H), 7.20 - 7.13 (m, 1H), 7.02 - 6.89 (m, 2H), 5.09 - 4.94 (m, 1H), 3.81 - 3.72 (m, 1H), 3.72 - 3.62 (m, 1H), 1.72 (s, 3H), 1.51 (d, *J* = 6.9 Hz, 3H), 1.27 (br s, 1H), 0.29 - 0.16 (brs , 3H), 0 (br s 1H). HRMS(B) *m*/*z* 357.1731 (M + H)⁺.

Example 46: second eluted product (9mg) ¹H NMR (400 MHz, MeOD) δ 8.19 - 8.07 (m, 1H), 7.41 - 7.28 (m, 2H), 7.19 - 7.12 (m, 1H), 7.07 - 6.94 (m, 2H), 5.12 - 4.99 (m, 1H), 3.88 - 3.71 (m, 2H), 2.01 - 1.87 (m, 1H), 1.52 (d, *J* = 7.0 Hz, 3H), 1.33 (s, 3H), 0.68-0.55 (m, 1H), 0.55 - 0.37 (m, 3H). HRMS(B) *m*/*z* 357.1736 (M + H)⁺.

The examples in **Table 45** were prepared using a method similar to that described for the preparation of **Examples 45 and 46**

**Table 45.**

| | | |
|---|---|---|
| 47 and 48 | 49 and 50 | 51 and 52 |
| 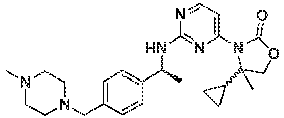 | 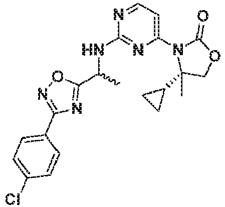 | 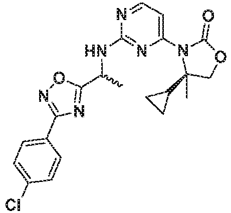 |
| 53 and 54 | 55 and 56 | 57 and 58 |
| 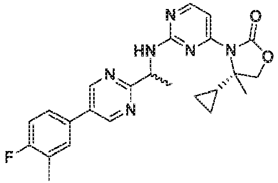 | 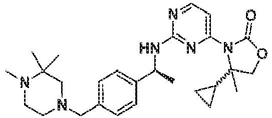 | 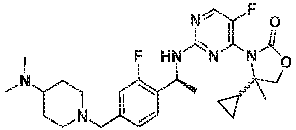 |
| 59 and 60 | 61 and 62 | 63 and 64 |
| 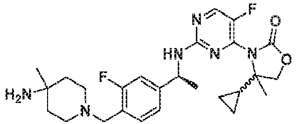 | 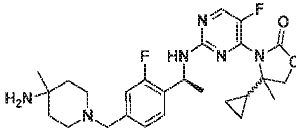 | 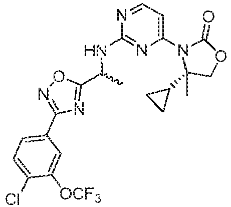 |
| 65 and 66 | | |
| 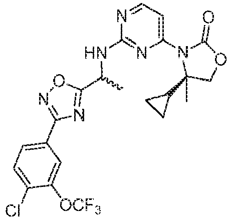 | | |

**Table 46. Chemical name, NMR chemical shifts and MS signal for each compound listed in Table 45.**

| Example: Name | Separation conditions, peal identification and analytical data |
|---|---|
| 47 & 48: (S)-4-cyclopropyl-4-methyl-3-(2-(((S)-1-(4-((4-methylpiperazin-1-yl)methyl)phenyl)ethyl)amino)p yrimidin-4-yl)oxazolidin-2-one | Separation was carried out with a column (OJ, 20x250mm) using 10%MeOH in CO₂, 75g/min to give (S)-4-cyclopropyl-4-methyl-3-(2-(((S)-1-(4-((4-methylpiperazin-1-yl)methyl)phenyl)ethyl)amino)pyrimidin-4-yl)oxazolidin-2-one and (R)-4-cyclopropyl-4-methyl-3-(2-(((S)-1-(4-((4-methylpiperazin-1-yl)methyl)phenyl)ethyl)amino)pyrimidin-4-yl)oxazolidin-2-one. |
| | Example 47: 1^{st} Peak: ¹H NMR (400 MHz, MeOD) δ 8.15 (s, 1H), 7.30 (d, *J* = 8.1 Hz, 2H), 7.25 (d, *J* = 8.3 Hz, 2H), 7.19 (d, *J* = 5.7 Hz, 1H), 5.04 (q, *J* = 7.0 Hz, 1H), 3.77 (d, *J* = 9.1 Hz, 1H), 3.66 (d, *J* = 9.2 Hz, 1H), 3.47 (s, 2H), 2.49 (s, 8H), 2.28 (s, 3H), 1.77 (s, 3H), 1.53 (d, *J* = 7.0 Hz, 3H), 0.19 (s, 3H), -0.08 - -0.34 (m, 1H). HRMS(B) *m*/*z* 451.2797 (M + H)⁺. |
| | Example 48: 2^{nd} Peak: ¹H NMR (400 MHz, MeOD) δ 8.14 (s, 1H), 7.33 (d, *J* = 8.2 Hz, 2H), 7.28 (d, *J* = 8.2 Hz, 2H), 7.15 (d, *J* = 5.8 Hz, 1H), 5.02 (d, *J* = 7.2 Hz, 1H), 3.78 (d, *J* = 4.6 Hz, 2H), 3.51 (s, 2H), 2.50 (s, 8H), 2.29 (s, 3H), 2.03 (tt, *J* = 8.5, 5.6 Hz, 1H), 1.52 (d, *J* = 7.0 Hz, 3H), 1.31 (br s, 3H), 0.63 (ddd, *J* = 8.8, 6.6, 3.2 Hz, 1H), 0.52 (t, *J* = 8.9 Hz, 1H), 0.43 (d, *J* = 5.3 Hz, 2H). HRMS(B) *m*/*z* 451.2799 (M + H)⁺. |
| 49 & 50: (4S)-3-(2-((1-(3-(4-chlorophenyl)-1,2,4-oxadiazol-5-yl)ethyl)amino)pyrimidin-4-yl)-4-cyclopropyl-4-methyloxazolidin-2-one | Separation was carried out with a column (AD-H 20x250mm) using 25% MeOH in CO₂, 70g/min) to give (S)-3-(2-(((R)-1-(3-(4-chlorophenyl)-1,2,4-oxadiazol-5-yl)ethyl)amino)pyrimidin-4-yl)-4-cyclopropyl-4-methyloxazolidin-2-one and (S)-3-(2-(((S)-1-(3-(4-chlorophenyl)-1,2,4-oxadiazol-5-yl)ethyl)amino)pyrimidin-4-yl)-4-cyclopropyl-4-methyloxazolidin-2-one. |
| | Example 49: 1^{st} Peak: ¹H NMR (400 MHz, MeOD) δ 8.20 (d, *J* = 5.9 Hz, 1H), 8.09 - 7.93 (m, 2H), 7.64 - 7.45 (m, 2H), 7.28 (d, *J* = 5.8 Hz, 1H), 5.42 (t, *J* = 7.3 Hz, 1H), 3.85 (s, 2H), 1.97 (s, 1H), 1.76 (d, *J* = 7.2 Hz, 3H), 1.40 (br s, 3H), 0.74 - 0.58 (m, 1H), 0.58 - 0.35 (m, 3H). |
| | HRMS(B) *m*/*z* 441.1423 (M + H)⁺ |
| | Example 50: 2nd Peak: ¹H NMR (400 MHz, MeOD) δ 8.21 (d, *J* = 5.9 Hz, 1H), 8.09 - 7.96 (m, 2H), 7.62 - 7.49 (m, 2H), 7.31 (d, *J* = 5.8 Hz, 1H), 5.41 (q, *J* = 7.1 Hz, 1H), 3.83 (d, *J* = 9.0 Hz, 1H), 3.75 (s, 1H), 1.78 (s, 3H), 1.77 (d, *J* = 7.5 Hz, 3H), 0.29 (s, 3H), 0.20 - -0.05 (m, 1H). HRMS(B) *m*/*z* 441.1420 (M + H)⁺ |
| 51 & 52: (4R)-3-(2-((1-(3-(4-chlorophenyl)-1,2,4-oxadiazol-5-yl)ethyl)amino)pyrimidin-4-yl)-4-cyclopropyl-4-methyloxazolidin-2-one | Separation was carried out with a column (AD-H 20x250mm) using 25% MeOH in CO₂, 70g/min) to give (R)-3-(2-(((R)-1-(3-(4-chlorophenyl)-1,2,4-oxadiazol-5-yl)ethyl)amino)pyrimidin-4-yl)-4-cyclopropyl-4-methyloxazolidin-2-one and (R)-3-(2-(((S)-1-(3-(4-chlorophenyl)-1,2,4-oxadiazol-5-yl)ethyl)amino)pyrimidin-4-yl)-4-cyclopropyl-4-methyloxazolidin-2-one. |
| | Example 51: 1st Peak: ¹H NMR (400 MHz, MeOD) δ 8.21 (d, *J* = 6.0 Hz, 1H), 8.06 - 7.96 (m, 2H), 7.60 - 7.49 (m, 2H), 7.31 (d, *J* = 5.8 Hz, 1H), 5.41 (q, *J* = 7.2 Hz, 1H), 3.83 (d, *J* = 9.0 Hz, 1H), 3.75 (s, 1H), 1.78 (s, 3H), 1.76 (d, *J* = 7.4 Hz, 3H), 0.30 (s, 3H), 0.19 - 0.02 (m, 1H). |
| | HRMS(B) *m*/*z* 441.1426 (M + H)⁺ |
| | Example 52: 2nd Peak: ¹H NMR (400 MHz, MeOD) δ 8.20 (d, *J* = 5.8 Hz, 1H), 8.08 - 7.97 (m, 2H), 7.61 - 7.47 (m, 2H), 7.28 (d, *J* = 5.8 Hz, 1H), 5.43 (q, *J* = 7.2 Hz, 1H), 3.85 (s, 2H), 1.98 (s, 1H), 1.76 (d, *J* = 7.2 Hz, 3H), 1.40 (br s, 3H), 0.71 - 0.57 (m, 1H), 0.57 - 0.36 (m, 3H). |
| | HRMS(B) *m*/*z* 441.1424 (M + H)⁺ |
| 53 & 54: (4S)-4-cyclopropyl-3-(2-((1-(5-(4-fluoro-3-methylphenyl)pyrimidin-2-yl)ethyl)amino)pyrimidin-4-yl)-4-methyloxazolidin-2-one | Separation was carried out with a column (IA-H 20x250mm) using 30% IPA in CO₂ to give (S)-4-cyclopropyl-3-(2-(((S)-1-(5-(4-fluoro-3-methylphenyl)pyrimidin-2-yl)ethyl)amino)pyrimidin-4-yl)-4-methyloxazolidin-2-one and (S)-4-cyclopropyl-3-(2-(((R)-1-(5-(4-fluoro-3-methylphenyl)pyrimidin-2-yl)ethyl)amino)pyrimidin-4-yl)-4-methyloxazolidin-2-one. |
| | Example 53: 1st Peak: ¹H NMR (400 MHz, MeOD) δ 8.97 (s, 2H), 8.16 (d, *J* = 5.9 Hz, 1H), 7.59 (dd, *J* = 7.2, 2.3 Hz, 1H), 7.52 (ddd, *J* = 7.6, 4.9, 2.5 Hz, 1H), 7.31 - 7.11 (m, 2H), 5.25 (q, *J* = 7.1 Hz, 1H), 3.82 (d, *J* = 9.1 Hz, 1H), 3.73 (s, 1H), 2.36 (d, *J* = 2.0 Hz, 3H), 1.78 (s, 3H), 1.66 (d, *J* = 7.1 Hz, 3H), 1.31 (s, 1H), 0.30 (s, 3H), 0.16 - -0.13 (m, 1H). |
| | HRMS(B) *m*/*z* 449.2081 |
| | Example 54: 2nd Peak: ¹H NMR (400 MHz, MeOD) δ 8.99 (s, 2H), 8.16 (d, *J* = 5.9 Hz, 1H), 7.66 - 7.56 (m, 1H), 7.53 (ddd, *J* = 7.5, 4.7, 2.4 Hz, 1H), 7.29 - 7.06 (m, 2H), 5.25 (q, *J* = 7.0 Hz, 1H), 3.82 (s, 2H), 2.37 (d, *J* = 2.0 Hz, 3H), 2.03 (p, *J* = 7.2 Hz, 1H), 1.65 (d, *J* = 7.0 Hz, 3H), 1.31 (br s, 3H), 0.71 - 0.62 (m, 1H), 0.59 - 0.49 (m, 2H), 0.48 - 0.39 (m, 1H). |
| | HRMS(B) *m*/*z* 449.2087 |
| 55 & 56: 4-cyclopropyl-4-methyl-3-(2-(((S)-1-(4-((3,3,4-trimethylpiperazin-1-yl)methyl)phenyl)ethyl)amino)p yrimidin-4-yl)oxazolidin-2-one | Separation was carried out with a column (OJ 20x250mm) using 10% MeOH + 5mM NH₄OH in CO₂ to give (R)-4-cyclopropyl-4-methyl-3-(2-(((S)-1-(4-((3,3,4-trimethylpiperazin-1-yl)methyl)phenyl)ethyl)amino)pyrimidin-4-yl)oxazolidin-2-one and (R)-4-cyclopropyl-4-methyl-3-(2-(((R)-1-(4-((3,3,4-trimethylpiperazin-1-yl)methyl)phenyl)ethyl)amino)pyrimidin-4-yl)oxazolidin-2-one. |
| | Example 55: 1st Peak: ¹H NMR (400 MHz, MeOD) δ 8.14 (d, *J* = 5.8 Hz, 1H), 7.26 (q, *J* = 8.3 Hz, 4H), 7.19 (d, *J* = 5.8 Hz, 1H), 5.04 (q, *J* = 7.0 Hz, 1H), 3.77 (d, *J* = 9.0 Hz, 1H), |
| | 3.65 (d, *J* = 9.0 Hz, 1H), 3.39 (s, 2H), 2.59 (t, *J* = 5.0 Hz, 2H), 2.46 (s, 2H), 2.23 (m, 5H), 1.77 (s, 3H), 1.53 (d, *J* = 7.0 Hz, 3H), 1.35 (br s, 1H), 1.06 (s, 6H), 0.18 (s, 3H), -0.19 (s, 1H). |
| | HRMS(B) *m*/*z* 511.3376 |
| | Example 56: 2nd Peak: ¹H NMR (400 MHz, MeOD) δ 8.13 (d, *J* = 5.8 Hz, 1H), 7.36 - 7.23 (m, 4H), 7.14 (d, *J* = 5.8 Hz, 1H), 5.00 (p, *J* = 6.4, 5.8 Hz, 1H), 3.78 (q, *J* = 9.4 Hz, 2H), 3.43 (s, 2H), 2.67 - 2.55 (m, 2H), 2.48 (s, 2H), 2.22 (m, 5H), 2.03 (tt, *J* = 8.6, 5.6 Hz, 1H), 1.52 (d, *J* = 7.0 Hz, 3H), 1.41 - 1.10 (br 3, 3H), 1.05 (d, *J* = 2.3 Hz, 6H), 0.69 - 0.56 (m, 1H), 0.52 (t, *J* = 8.9 Hz, 1H), 0.42 (dd, *J* = 6.6, 4.0 Hz, 2H). |
| | HRMS(B) *m*/*z* 511.3374 |
| 57 & 58: 4-cyclopropyl-3-(2-(((S)-1-(4-((4-(dimethylamino)piperidin-1-yl)methyl)-2-fluorophenyl)ethyl)amino)-5-fluoropyrimidin-4-yl)-4-methyloxazolidin-2-one | Separation was carried out with on a column (AD-H 21x250mm) using 30% MeOH + 10mM NH₄OH in CO₂ to give (S)-4-cyclopropyl-3-(2-(((S)-1-(4-((4-(dimethylamino)piperidin-1-yl)methyl)-2-fluorophenyl)ethyl)amino)-5-fluoropyrimidin-4-yl)-4-methyloxazolidin-2-one and (R)-4-cyclopropyl-3-(2-(((S)-1-(4-((4-(dimethylamino)piperidin-1-yl)methyl)-2-fluorophenyl)ethyl)amino)-5-fluoropyrimidin-4-yl)-4-methyloxazolidin-2-one. |
| | Example 57: 1st Peak: ¹H NMR (400 MHz, MeOD) δ 8.25 (d, *J* = 3.0 Hz, 1H), 7.32 (t, *J* = 7.9 Hz, 1H), 7.13 - 6.99 (m, 2H), 5.22 (q, *J* = 6.9 Hz, 1H), 4.04 - 3.86 (m, 2H), 3.46 (s, 2H), 2.94 (d, *J* = 11.4 Hz, 2H), 2.30 (s, 6H), 2.22 (dd, *J* = 13.4, 9.2 Hz, 1H), 2.01 (td, *J* = 12.1, 2.3 Hz, 2H), 1.86 (d, *J* = 12.4 Hz, 2H), 1.61 - 1.50 (m, 5H), 1.47 (s, 3H), 1.06 - 0.81 (m, 1H), 0.21 (s, 2H), 0.11 (s, 2H). |
| | HRMS(C) *m*/*z* 515.2936 (M + H)⁺ |
| | Example 58: 2nd Peak: ¹H NMR (400 MHz, MeOD) δ 8.25 (d, *J* = 3.0 Hz, 1H), 7.32 (t, *J* = 7.9 Hz, 1H), 7.14 - 7.02 (m, 2H), 5.18 (d, *J* = 7.4 Hz, 1H), 3.95 (s, 2H), 3.48 (s, 2H), 2.93 (d, *J* = 11.1 Hz, 2H), 2.31 (s, 6H), 2.22 (t, *J* = 12.2 Hz, 1H), 2.01 (ddd, *J* = 12.5, 9.5, 3.0 Hz, 2H), 1.86 (d, *J* = 12.4 |
| | Hz, 2H), 1.64 - 1.45 (m, 6H), 1.12 (s, 3H), 0.58 - 0.42 (m, 3H), 0.37 (s, 1H). |
| | HRMS(C) *m*/*z* 515.2954 (M + H)⁺ |
| 59 & 60: 3-(2-(((S)-1-(4-((4-amino-4-methylpiperidin-1-yl)methyl)-3-fluorophenyl)ethyl)amino)-5-fluoropyrimidin-4-yl)-4-cyclopropyl-4-methyloxazolidin-2-one | Separation was carried out with a column (AD-H 21x250mm) using 45% MeOH + 10mM NH₄OH in CO₂ to give (S)-3-(2-(((S)-1-(4-((4-amino-4-methylpiperidin-1-yl)methyl)-3-fluorophenyl)ethyl)amino)-5-fluoropyrimidin-4-yl)-4-cyclopropyl-4-methyloxazolidin-2-one and (R)-3-(2-(((S)-1-(4-((4-amino-4-methylpiperidin-1-yl)methyl)-3-fluorophenyl)ethyl)amino)-5-fluoropyrimidin-4-yl)-4-cyclopropyl-4-methyloxazolidin-2-one. |
| | Example 59: 1st Peak: ¹H NMR (400 MHz, MeOD) δ 8.25 (d, *J* = 2.9 Hz, 1H), 7.34 (t, *J* = 7.7 Hz, 1H), 7.15 (dd, *J* = 7.9, 1.7 Hz, 1H), 7.07 (dd, *J* = 11.3, 1.7 Hz, 1H), 4.94 (q, *J* = 6.7 Hz, 1H), 3.97 (q, *J* = 9.0 Hz, 2H), 3.63 - 3.52 (m, 2H), 2.55 (s, 2H), 2.48 (s, 2H), 1.58 (td, *J* = 7.3, 4.6 Hz, 4H), 1.52 (d, *J* = 7.0 Hz, 3H), 1.46 (s, 3H), 1.14 (s, 3H), 1.2-0.8)br s, 1H), 0.24 (m, 4H). |
| | HRMS(C) *m*/*z* 501.2777 (M + H)⁺. |
| | Example 60: 2nd Peak: ¹H NMR (400 MHz, MeOD) δ 8.24 (d, *J* = 2.9 Hz, 1H), 7.35 (t, *J* = 7.7 Hz, 1H), 7.15 (dd, *J* = 7.9, 1.7 Hz, 1H), 7.07 (dd, *J* = 11.1, 1.7 Hz, 1H), 3.97 (s, 2H), 3.58 (d, *J* = 1.5 Hz, 2H), 2.65 - 2.37 (m, 4H), 1.55 (m, 8H), 1.13 (m, 6H), 0.58 - 0.40 (m, 3H), 0.36 (s, 1H). HRMS(C) *m*/*z* 501.2775 (M + H)⁺. |
| 61 & 62: 3-(2-(((S)-1-(4-((4-amino-4-methylpiperidin-1-yl)methyl)-2-fluorophenyl)ethyl)amino)-5-fluoropyrimidin-4-yl)-4-cyclopropyl-4-methyloxazolidin-2-one | Separation was carried out with a column (AD-H 21x250mm) using 45% MeOH + 10mM NH₄OH in CO₂ to give (S)-3-(2-(((S)-1-(4-((4-amino-4-methylpiperidin-1-yl)methyl)-2-fluorophenyl)ethyl)amino)-5-fluoropyrimidin-4-yl)-4-cyclopropyl-4-methyloxazolidin-2-one and (R)-3-(2-(((S)-1-(4-((4-amino-4-methylpiperidin-1-yl)methyl)-2-fluorophenyl)ethyl)amino)-5-fluoropyrimidin-4-yl)-4-cyclopropyl-4-methyloxazolidin-2-one. |
| | Example 61: 1^{st} Peak: ¹H NMR (400 MHz, MeOD) δ 8.25 (d, *J* = 2.9 Hz, 1H), 7.32 (t, *J* = 8.0 Hz, 1H), 7.14 - 7.00 (m, 2H), 5.22 (q, *J* = 6.9 Hz, 1H), 4.06 - 3.86 (m, 2H), 3.50 (s, |
| | 2H), 2.47 (d, *J* = 23.2 Hz, 4H), 1.60 (dt, *J* = 20.9, 6.7 Hz, 4H), 1.52 (d, *J* = 7.0 Hz, 3H), 1.47 (s, 3H), 1.14 (s, 3H), 0.97 (br s, 1H), 0.20 (s, 2H), 0.15 - -0.04 (m, 2H). HRMS(C) *m*/*z* 501.2792 (M + H)⁺. |
| | Example 62: 2nd Peak: ¹H NMR (400 MHz, MeOD) δ 8.24 (d, *J* = 3.0 Hz, 1H), 7.33 (t, *J* = 7.9 Hz, 1H), 7.15 - 7.01 (m, 2H), 5.18 (d, *J* = 7.8 Hz, 1H), 3.94 (s, 2H), 3.51 (s, 2H), 2.60 - 2.32 (m, 4H), 1.57 (dtd, *J* = 10.7, 5.6, 2.7 Hz, 5H), 1.52 (d, *J* = 7.0 Hz, 3H), 1.14 (s, 3H + br s, 3H), 0.58 - 0.41 (m, 3H), 0.36 (s, 1H). |
| | HRMS(C) *m*/*z* 501.2784 (M + H)⁺. |
| 63 & 64: (4S)-3-(2-((1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one | Separation was carried out with an AD-H column (75 g/min, 120 bar, 21 x 250 mm) eluting 30% IPA/CO₂ (v/v) to give (S)-3-(2-(((R)-1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one and (S)-3-(2-(((S)-1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one |
| | Example 63: 1^{st} Peak: ¹H NMR (400 MHz, CDCl₃) δ 8.25 (d, *J* = 5.8 Hz, 5H), 8.10 - 7.91 (m, 9H), 7.61 (d, *J* = 8.4 Hz, 4H), 7.42 (d, *J* = 5.8 Hz, 4H), 5.54 (d, *J* = 39.6 Hz, 9H), 4.05 (dt, *J* = 12.3, 6.2 Hz, 1H), 3.80 (s, 10H), 3.52 (s, 6H), 1.91 (tt, *J* = 8.6, 5.6 Hz, 5H), 1.78 (d, *J* = 7.0 Hz, 15H), 1.71 - 1.43 (m, 4H), 1.23 (d, *J* = 6.2 Hz, 7H), 1.03 (s, 2H), 0.70 (ddt, *J* = 8.9, 5.2, 3.6 Hz, 5H), 0.61 - 0.50 (m, 5H), 0.50 - 0.35 (m, 10H). HRMS (B) m/z 524.1187. Chiral RT = 1.95 min |
| | Example 64: 2^{nd} Peak: ¹H NMR (400 MHz, CDCl₃) δ 8.25 (d, *J* = 5.8 Hz, 1H), 8.11 - 8.02 (m, 1H), 7.97 (dd, *J* = 8.4, 1.9 Hz, 1H), 7.61 (d, *J* = 8.3 Hz, 1H), 7.44 (d, *J* = 5.8 Hz, 1H), 5.57 (d, *J* = 71.0 Hz, 2H), 4.05 (p, *J* = 6.1 Hz, 1H), 1.87 - 1.71 (m, 7H), 3.79 (d, *J* = 8.8 Hz, 1H), 3.70 (d, *J* = 9.2 Hz, 1H), 3.52 (s, 2H), 1.68 - 1.55 (m, 2H), 1.32 (s, 2H), 1.23 (d, *J* = 6.1 Hz, 6H), 0.39 (d, *J* = 65.0 Hz, 4H). HRMS (B) m/z 524.1187. Chiral RT = 3.15 min |
| 65 & 66: (4R)-3-(2-((1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one | Separation was carried out with an AD-H column (75 g/min, 120 bar, 21 x 250 mm) eluting 25% IPA/CO₂ (v/v) to give (R)-3-(2-(((R)-1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one and (R)-3-(2-(((S)-1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one |
| | |
| | Example 65: 1^{st} Peak: ¹H NMR (400 MHz, CDCl₃) δ 8.25 (d, *J* = 5.8 Hz, 1H), 8.07 - 8.01 (m, 1H), 7.96 (dd, *J* = 8.3, 2.0 Hz, 1H), 7.61 (d, *J* = 8.3 Hz, 1H), 7.43 (d, *J* = 5.8 Hz, 1H), 5.80 - 5.39 (m, 2H), 4.05 (p, *J* = 6.1 Hz, 1H), 3.79 (d, *J* = 8.6 Hz, 1H), 3.75 - 3.64 (m, 1H), 1.76 (s, 1H), 1.71 - 1.57 (m, 2H), 1.23 (d, *J* = 6.1 Hz, 2H), 0.46 (q, *J* = 10.0, 7.2 Hz, 1H), 0.40 - 0.04 (m, 3H). HRMS (B) m/z 524.1187. Chiral RT = 2.15 min |
| | Example 66: 2^{nd} Peak: ¹H NMR (400 MHz, CDCl₃) δ 8.25 (d, *J* = 5.8 Hz, 1H), 8.04 (p, *J* = 1.4 Hz, 1H), 7.98 (dd, *J* = 8.4, 1.9 Hz, 1H), 7.61 (d, *J* = 8.4 Hz, 1H), 7.41 (d, *J* = 5.8 Hz, 1H), 5.73 (s, 1H), 5.60 - 5.39 (m, 1H), 4.05 (p, *J* = 6.1 Hz, 0H), 3.79 (s, 2H), 1.91 (tt, *J* = 8.5, 5.6 Hz, 1H), 1.78 (d, *J* = 7.0 Hz, 3H), 1.68 (s, 0H), 1.23 (d, *J* = 6.1 Hz, 1H), 0.70 (ddt, *J* = 8.8, 5.1, 3.5 Hz, 1H), 0.64 - 0.52 (m, 1H), 0.51 - 0.39 (m, 2H). HRMS (B) m/z 524.1187. Chiral RT = 3.60 min |

The compounds listed in Table 47 are made using methods similar to those described for Examples 1-69 and as outlined in the general synthetic procedures.

**Table 47.**

| Example | Name |
|---|---|
| 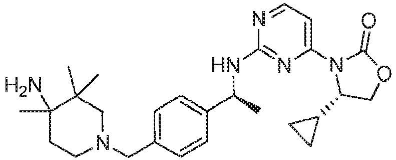 | (4S)-3-(2-(((1S)-1-(4-((4-amino-3,3,4-trimethylpiperidin-1-yl)methyl)phenyl)ethyl)amino)pyrimidin-4-yl)-4-cyclopropyloxazolidin-2-one |
| 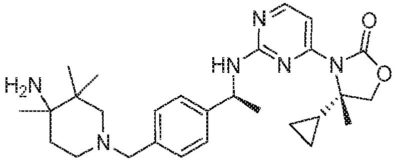 | (4S)-3-(2-(((1S)-1-(4-((4-amino-3,3,4-trimethylpiperidin-1-yl)methyl)phenyl)ethyl)amino)pyrimidin-4-yl)-4-cyclopropyl-4-methyloxazolidin-2-one |
| 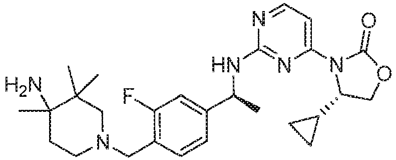 | (4S)-3-(2-(((1S)-1-(4-((4-amino-3,3,4-trimethylpiperidin-1-yl)methyl)-3-fluorophenyl)ethyl)amino)pyrimidin-4-yl)-4-cyclopropyloxazolidin-2-one |
| 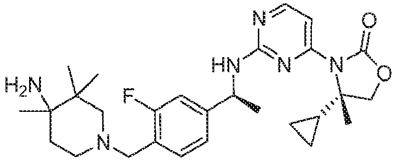 | (4S)-3-(2-(((1S)-1-(4-((4-amino-3,3,4-trimethylpiperidin-1-yl)methyl)-3-fluorophenyl)ethyl)amino)pyrimidin-4-yl)-4-cyclopropyl-4-methyloxazolidin-2-one |
| 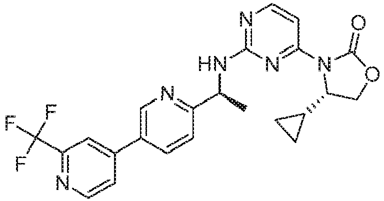 | (S)-4-cyclopropyl-3-(2-(((S)-1-(2'-(trifluoromethyl)-[3,4'-bipyridin]-6-yl)ethyl)amino)pyrimidin-4-yl)oxazolidin-2-one |
| 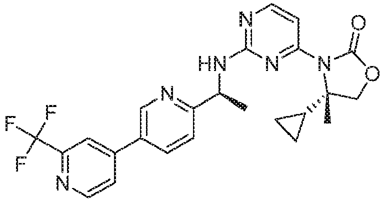 | (S)-4-cyclopropyl-4-methyl-3-(2-(((S)-1-(2'-(trifluoromethyl)-[3,4'-bipyridin]-6-yl)ethyl)amino)pyrimidin-4-yl)oxazolidin-2-one |
| 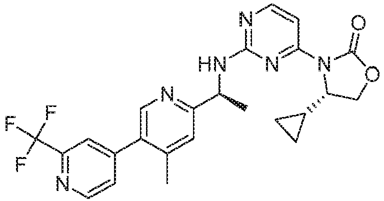 | (S)-4-cyclopropyl-3-(2-(((S)-1-(4-methyl-2'-(trifluoromethyl)-[3,4'-bipyridin]-6-yl)ethyl)amino)pyrimidin-4-yl)oxazolidin-2-one |
| 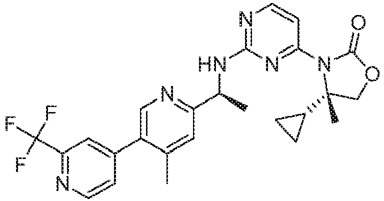 | (S)-4-cyclopropyl-4-methyl-3-(2-(((S)-1-(4-methyl-2'-(trifluoromethyl)-[3,4'-bipyridin]-6-yl)ethyl)amino)pyrimidin-4-yl)oxazolidin-2-one |
| 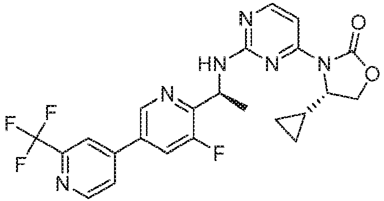 | (S)-4-cyclopropyl-3-(2-(((S)-1-(5-fluoro-2'-(trifluoromethyl)-[3,4'-bipyridin]-6-yl)ethyl)amino)pyrimidin-4-yl)oxazolidin-2-one |
| 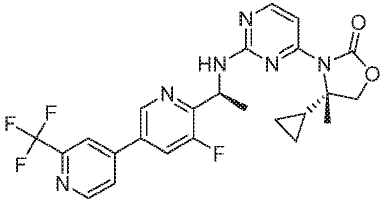 | (S)-4-cyclopropyl-3-(2-(((S)-1-(5-fluoro-2'-(trifluoromethyl)-[3,4'-bipyridin]-6-yl)ethyl)amino)pyrimidin-4-yl)-4-methyloxazolidin-2-one |
| 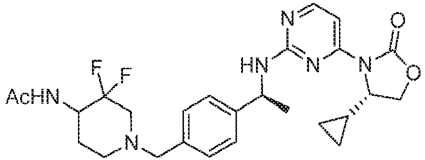 | N-(1-(4-((S)-1-((4-((S)-4-cyclopropyl-2-oxooxazolidin-3-yl)pyrimidin-2-yl)amino)ethyl)benzyl)-3,3-difluoropiperidin-4-yl)acetamide |
| 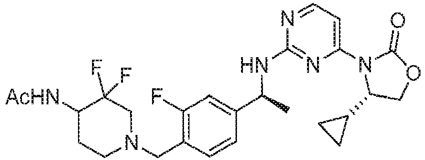 | N-(1-(4-((S)-1-((4-((S)-4-cyclopropyl-2-oxooxazolidin-3-yl)pyrimidin-2-yl)amino)ethyl)-2-fluorobenzyl)-3,3-difluoropiperidin-4-yl)acetamide |
| 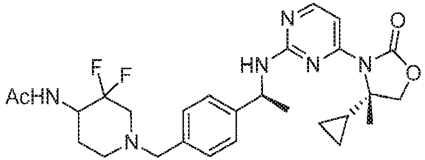 | N-(1-(4-((S)-1-((4-((S)-4-cyclopropyl-4-methyl-2-oxooxazolidin-3-yl)pyrimidin-2-yl)amino)ethyl)benzyl)-3,3-difluoropiperidin-4-yl)acetamide |
| 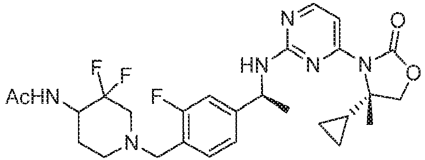 | N-(1-(4-((S)-1-((4-((S)-4-cyclopropyl-4-methyl-2-oxooxazolidin-3-yl)pyrimidin-2-yl)amino)ethyl)-2-fluorobenzyl)-3,3-difluoropiperidin-4-yl)acetamide |
| 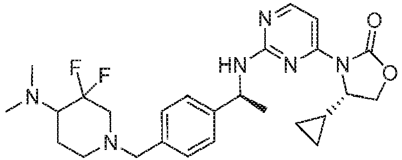 | (4S)-4-cyclopropyl-3-(2-(((1S)-1-(4-((4-(dimethylamino)-3,3-difluoropiperidin-1-yl)methyl)phenyl)ethyl)amino)pyrimidin-4-yl)oxazolidin-2-one |
| 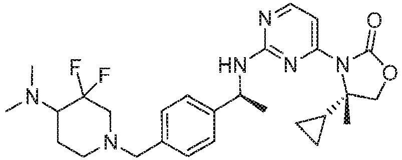 | (4S)-4-cyclopropyl-3-(2-(((1S)-1-(4-((4-(dimethylamino)-3,3-difluoropiperidin-1-yl)methyl)phenyl)ethyl)amino)pyrimidin-4-yl)-4-methyloxazolidin-2-one |
| 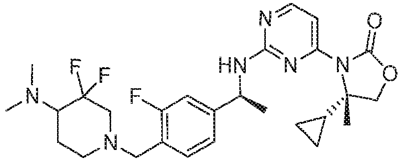 | (4S)-4-cyclopropyl-3-(2-(((1S)-1-(4-((4-(dimethylamino)-3,3-difluoropiperidin-1-yl)methyl)-3-fluorophenyl)ethyl)amino)pyrimidin-4-yl)-4-methyloxazolidin-2-one |
| 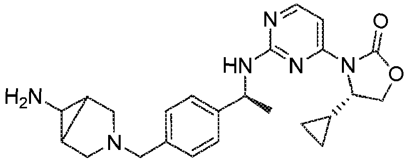 | (4S)-3-(2-(((1S)-1-(4-((6-amino-3-azabicyclo[3.1.0]hexan-3-yl)methyl)phenyl)ethyl)amino)pyrimidin-4-yl)-4-cyclopropyloxazolidin-2-one |
| 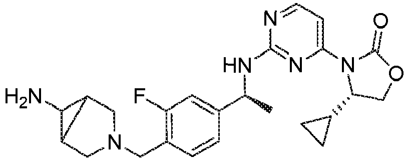 | (4S)-3-(2-(((1S)-1-(4-((6-amino-3-azabicyclo[3.1.0]hexan-3-yl)methyl)-3-fluorophenyl)ethyl)amino)pyrimidin-4-yl)-4-cyclopropyloxazolidin-2-one |
| 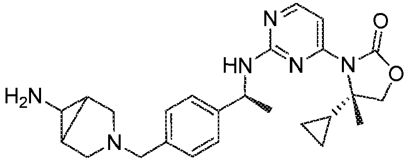 | (4S)-3-(2-(((1S)-1-(4-((6-amino-3-azabicyclo[3.1.0]hexan-3-yl)methyl)phenyl)ethyl)amino)pyrimidin-4-yl)-4-cyclopropyl-4-methyloxazolidin-2-one |
| 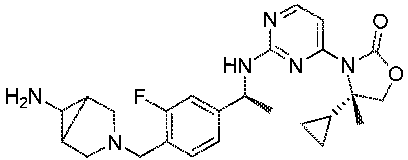 | (4S)-3-(2-(((1S)-1-(4-((6-amino-3-azabicyclo[3.1.0]hexan-3-yl)methyl)-3-fluorophenyl)ethyl)amino)pyrimidin-4-yl)-4-cyclopropyl-4-methyloxazolidin-2-one |
| 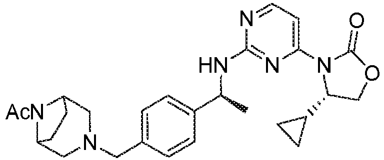 | (4S)-3-(2-(((1S)-1-(4-((8-acetyl-3,8-diazabicyclo[3.2.1]octan-3-yl)methyl)phenyl)ethyl)amino)pyrimidin-4-yl)-4-cyclopropyloxazolidin-2-one |
| 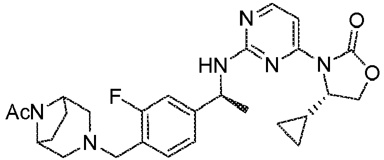 | (4S)-3-(2-(((1S)-1-(4-((8-acetyl-3,8-diazabicyclo[3.2.1]octan-3-yl)methyl)-3-fluorophenyl)ethyl)amino)pyrimidin-4-yl)-4-cyclopropyloxazolidin-2-one |
| 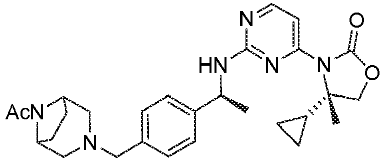 | (4S)-3-(2-(((1S)-1-(4-((8-acetyl-3,8-diazabicyclo[3.2.1]octan-3-yl)methyl)phenyl)ethyl)amino)pyrimidin-4-yl)-4-cyclopropyl-4-methyloxazolidin-2-one |
| 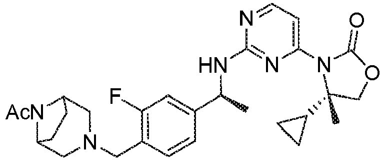 | (4S)-3-(2-(((1S)-1-(4-((8-acetyl-3,8-diazabicyclo[3.2.1]octan-3-yl)methyl)-3-fluorophenyl)ethyl)amino)pyrimidin-4-yl)-4-cyclopropyl-4-methyloxazolidin-2-one |
| 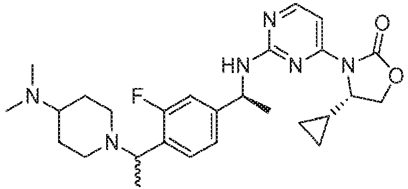 | (4S)-4-cyclopropyl-3-(2-(((1S)-1-(4-(1-(4-(dimethylamino)piperidin-1-yl)ethyl)-3-fluorophenyl)ethyl)amino)pyrimidin-4-yl)oxazolidin-2-one |
| 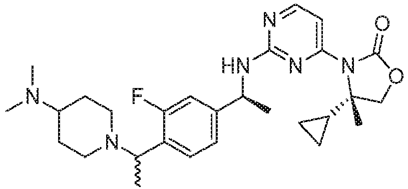 | (4S)-4-cyclopropyl-3-(2-(((1S)-1-(4-(1-(4-(dimethylamino)piperidin-1-yl)ethyl)-3-fluorophenyl)ethyl)amino)pyrimidin-4-yl)-4-methyloxazolidin-2-one |
| 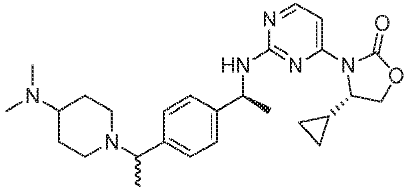 | (4S)-4-cyclopropyl-3-(2-(((1S)-1-(4-(1-(4-(dimethylamino)piperidin-1-yl)ethyl)phenyl)ethyl)amino)pyrimidin-4-yl)oxazolidin-2-one |
| 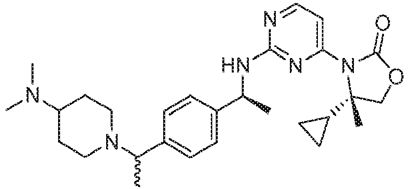 | (4S)-4-cyclopropyl-3-(2-(((1S)-1-(4-(1-(4-(dimethylamino)piperidin-1-yl)ethyl)phenyl)ethyl)amino)pyrimidin-4-yl)-4-methyloxazolidin-2-one |
| 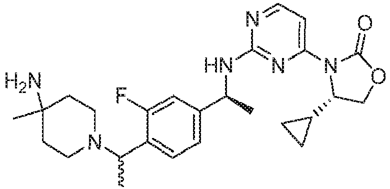 | (4S)-3-(2-(((1S)-1-(4-(1-(4-amino-4-methylpiperidin-1-yl)ethyl)-3-fluorophenyl)ethyl)amino)pyrimidin-4-yl)-4-cyclopropyloxazolidin-2-one |
| 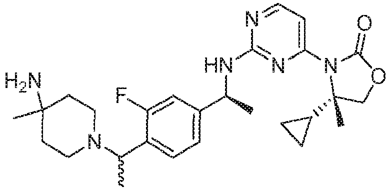 | (4S)-3-(2-(((1S)-1-(4-(1-(4-amino-4-methylpiperidin-1-yl)ethyl)-3-fluorophenyl)ethyl)amino)pyrimidin-4-yl)-4-cyclopropyl-4-methyloxazolidin-2-one |
| 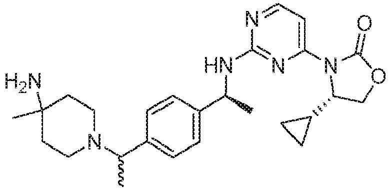 | (4S)-3-(2-(((1S)-1-(4-(1-(4-amino-4-methylpiperidin-1-yl)ethyl)phenyl)ethyl)amino)pyrimidin-4-yl)-4-cyclopropyloxazolidin-2-one |
| 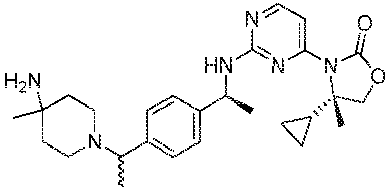 | (4S)-3-(2-(((1S)-1-(4-(1-(4-amino-4-methylpiperidin-1-yl)ethyl)phenyl)ethyl)amino)pyrimidin-4-yl)-4-cyclopropyl-4-methyloxazolidin-2-one |

### Biological Data

### Mutant IDH1 biochemical assay: LC-MS detection of 2-HG.

Mutant IDH1 R132H catalytic activity was monitored using the quantitative liquid chromatography/mass spectrometry (LC-MS) detection of 2-HG, a product of the NADPH-dependent alpha-KG reduction reaction.

More specifically, the biochemical reactions were performed at room temperature in 384-well Greiner flat-bottom plates (Costar, Cat. No. 781201) using a final reaction volume of 30 µL and the following assay buffer conditions: 50 mM HEPES pH 7.4, 10 mM MgCl₂, 50 mM KCI, 1 mM DTT, 0.02% BSA, 5 uM NADPH and 100 uM alpha-KG.

The final reaction mixture contained 3.3% DMSO and inhibitors with concentrations ranging 0.02 - 50 µM. The IDH1 enzyme was used at a final concentration of 0.25 nM. Following 45 minutes incubation, the reaction mixtures were quenched by the addition of 10 µL of 16% formic acid containing 800 nM of 5-carbon labeled ¹³C-2-HG). The protein was then precipitated by the addition of 2.5 volumes of acetonitrile followed by centrifugation (3000 x g, 20 minutes). The concentration of 2-HG in the resulting supernatants was measured by LC-MS (see below).

LC-MS method. Reaction mixture supernatants were submitted to chromatographic separation on a BiobasicAX column (2.1 mm x 20 mm, 5 µm particle, Thermo Scientific Inc.). The chromatographic mobile phases were A) 25 mM ammonium biocarbonate and B) acetonitrile (0.1% ammonium hydroxide). Nicotinamide was eluted at 1 ml/min using a 85-5% B gradient over 0.9 minutes (Agilent 1200SL LC system, Thermofisher LX-4 autosampler) and analyzed by multiple reaction monitoring (MRM) on a API4000 QTrap mass spectrometer (ABSciex, Framingham, MA) in the positive electrospray ionization (ESI+) mode. The mass transition for 2-HG and ¹³C-2-HG were 147→129 and 152→134, respectively. The relative responses (2-HG/¹³C-2-HG) were measured at varied inhibitor concentrations and used to calculate inhibitory IC50 values (normalized IC50 regression curves).

### R132 protein expression and purification.

IDH1 R132H was cloned into the pET47b vector using the restriction sites XmaI/XhoI which yields an in frame, N-terminal His₆ site cleavable with Prescission protease. This plasmid was transformed into Rosetta™ 2(DE3) (Novagen) cells. In shake flasks, 8L of cells were grown in Terrific Broth (Teknova) (plus kanamycin 50µg/mL and chloramphenicol 34µg/mL) at 37°C to an OD₆₀₀ of 0.8 and protein expression was induced by addition of IPTG to a concentration of 0.20mM. The cells were subsequently grown for 18 hours at 18°C.

### His₆-IDH1 (R132H) Uncut protein

MAHHHHHHSAALEVLFQGPGMSKKISGGSVVEMQGDEMTRIIWELIKEKLIFPYVELDLHSYD LGI EN RDATN DQVTKDAAEAI KKH NVGVKCATITPDEKRVEEFKLKQM WKSPNGTI RN I LGGTV FREAIICKNIPRLVSGWVKPIIIGHHAYGDQYRATDFVVPGPGKVEITYTPSDGTQKVTYLVHNF EEGGGVAMGMYNQDKSIEDFAHSSFQMALSKGWPLYLSTKNTILKKYDGRFKDIFQEIYDKQ YKSQFEAQKIWYEHRLIDDMVAQAMKSEGGFIWACKNYDGDVQSDSVAQGYGSLGMMTSVL VCPDGKTVEAEAAHGTVTRHYRMYQKGQETSTNPIASIFAWTRGLAHRAKLDNNKELAFFAN ALEEVSIETIEAGFMTKDLAACIKGLPNVQRSDYLNTFEFMDKLGENLKIKLAQAKL (stop) (SEQ ID NO: 1)

### IDH1 (R132H) Prescission Cut Protein (N-term gpg is cloning artifact)

GPGMSKKISGGSVVEMQGDEMTRIIWELIKEKLIFPYVELDLHSYDLGIENRDATNDQVTKDAA EAIKKHNVGVKCATITPDEKRVEEFKLKQMWKSPNGTIRNILGGTVFREAIICKNIPRLVSGWVK PIIIGHHAYGDQYRATDFVVPGPGKVEITYTPSDGTQKVTYLVHNFEEGGGVAMGMYNQDKSI EDFAHSSFQMALSKGWPLYLSTKNTILKKYDGRFKDIFQEIYDKQYKSQFEAQKIWYEHRLIDD MVAQAMKSEGGFIWACKNYDGDVQSDSVAQGYGSLGMMTSVLVCPDGKTVEAEAAHGTVT RHYRMYQKGQETSTNPIASIFAWTRGLAHRAKLDNNKELAFFANALEEVSIETIEAGFMTKDLA ACIKGLPNVQRSDYLNTFEFMDKLGENLKIKLAQAKL (stop) (SEQ ID NO: 2)

### Purification

The cells were homogenized in Lysis Buffer with protease inhibitors (cOmplete EDTA-free protease inhibitor tablets (Roche), 1 tablet per 50mL of buffer), DNAse, and to 200 µM PMSF and lysed in a Microfluidizer. After lysis, Triton X-100 was added to 0.1% and stirred at 4°C for 30 minutes.

The cleared lysate was loaded onto 2 x 5mL HisTrap FF crude columns (GE), washed extensively with Lysis Buffer until the A₂₈₀ stabilized and eluted with Ni Elution Buffer. Peak eluted fractions were concentrated to 30mL, EDTA was added to 1mM and GST-Prescission protease was added to 3U/100µg of protein. The sample was dialyzed against 2L Dialysis Buffer I (MWCO 50kDa) for 6 hours at 4°C then dialyzed against 2L of Dialysis Buffer II for at least 6 more hours. GST-Prescission cleaved sample was rocked with Glutathione Agarose Beads, spun down and then the supernatant was loaded through a 5mL HisTrap HP column and the flow through was collected.

Flow through was then diluted with ice cold 20mM Tris pH 7.4 and 1 mM TCEP until the conductivity dropped to less than 5 mS/cm (a roughly three fold dilution). This sample was then flowed through a HiTrap Q column and the flow through was concentrated to 10mL and loaded onto an equilibrated 26/60 Superdex 200 column using SEC Buffer as the mobile phase. Peak fractions were collected, concentrated and aliquoted.
Lysis Buffer: 50mM Tris pH=7.4, 500mM NaCl, 20mM Imidazole, and 1mM TCEP
Ni Elution Buffer: 50mM Tris pH=7.4, 150mM NaCl, 200mM Imidazole, and 1mM TCEP
Dialysis Buffer I: 20mM Tris pH=7.4, 150mM NaCl, 1mM TCEP, and 50mM Imidazole
Dialysis Buffer II:20mM Tris pH=7.4, 150mM NaCl, and 1mM TCEP
SEC Buffer: 20mM Tris pH=7.4, 150mM NaCl, and 1mM TCEP

The results of the mutant IDH1 biochemical assay (mIDH R132H) are given in Table 48. Some of the examples were run in the assay multiple times and therefore the IC₅₀ values are expressed as a range of activity.

### Fluorescence biochemical assay

The IDH1 (R132H) mutant catalyzes the reduced form of NADP+ (NADPH) and α-ketoglutarate (α-KG) to form nicotinamide adenine dinucleotide phosphate (NADP+) and R (-)-2-hydroxyglutarate (2HG). The reaction can be monitored kinetically by following the oxidation of NADPH to NADP+ which is measured using fluorescence, excitation at 355 nm and emission at 530 nm. Reactions were monitored using the Perkin-Elmer Envision, Model 2101. More specifically, the biochemical reactions were performed at room temperature in 384-well Greiner flat-bottom plates (Cat. No. 781076) using a final reaction volume of 20 µL and the following assay buffer conditions: 50 mM HEPES pH 7.5, 10 mM MgCl₂, 1 mM DTT, 0.02% BSA, 0.02% Tween-20, 10 µM NADPH and 100 µM α-KG. The final reaction mixture contained 2.5% DMSO and test compounds with concentrations ranging 0.0000008 - 25 µM. The IDH1 (R132H) enzyme was used at a final concentration of 10 nM. Curve fitting for dose response IC50 determinations was done in the Helios module of the software package DAVID. The 4-parameter logistic model was used: y = min + ((max - min) / 1 + (x / IC₅₀)^{slope})

The results of the fluorescense biochemical assay (mIDH R132H) are given in Table 48. Some of the examples were run in the assay multiple times and therefore the IC₅₀ values are expressed as a range of activity.

**Table 48. Results of the LC-MS and fluorescence biochemical assays.**

| **Example Number** | **LC-MS biochemical assay IC50 (µM)** | **Fluorescence biochemical assay IC50 (µM)** |
|---|---|---|
| 1 | --- | 0.365 |
| 2 | --- | 0.316 |
| 3 | <0.0229 | 0.0269 |
| 4 | --- | 0.0248 |
| 5 | --- | 0.0332 |
| 6 | --- | 0.934 |
| 7 | 0.00845 | 0.0174 |
| 8 | 0.0471 | 0.0713 |
| 9 | 0.0437 | 0.105 |
| 10 | 0.0228 | 0.0716 |
| 11 | 0.0767 | 0.0359 |
| 12 | --- | 0.323 |
| 13 | --- | 0.191 |
| 14 | --- | 0.0491 |
| 15 | --- | 0.108 |
| 16 | --- | 0.0292 |
| 17 | --- | 0.169 |
| 18 | 0.0158 | 0.0238 |
| 19 | 0.0572 | 0.0443 |
| 20 | 0.0839 | 0.218 |
| 21 | --- | 1.2 |
| 22 | --- | 1.14 |
| 23 | --- | 1.37 |
| 24 | --- | 1.19 |
| 25 | --- | 0.372 |
| 26 | --- | 0.349 |
| 27 | 0.0200 | 0.00727 |
| 28 | --- | 0.0627 |
| 29 | --- | 0.124 |
| 30 | 0.211 - 0.271 | 0.122 - 0.172 |
| 31 | 0.267 | --- |
| 32 | 0.0133 | --- |
| 33 | 0.175 | 0.109 - 0.115 |
| 34 | 0.192 | 0.0418 - 0.0531 |
| 35 | 0.0109 | <0.0159 |
| 36 | 0.963 | --- |
| 37 | 0.0286 | --- |
| 38 | --- | --- |
| 39 | --- | --- |
| 40 | 0.117 | 0.105 - 0.111 |
| 41 | 0.376 | 0.194 - 0.473 |
| 42 | 0.217 | --- |
| 43 | 0.151 | --- |
| 45 | 0.189 | 0.235 - 0.253 |
| 46 | 0.791 | 0.793 - 1.21 |
| 47 | 0.703 | --- |
| 48 | 2.66 | --- |
| 49 | 2.52 | --- |
| 50 | 0.0202 - 0.0260 | 0.0629 |
| 51 | 3.16 - 3.26 | --- |
| 52 | 0.0326 - 0.0540 | --- |
| 53 | 0.0227 - 0.0354 | 0.0362 |
| 54 | >5 | --- |
| 55 | 0.0779 | --- |
| 56 | 0.527 | --- |
| 57 | 0.868 | 0.29 |
| 58 | 2.72 | 0.971 |
| 59 | 0.922 | 0.183 |
| 60 | 1.52 | 0.584 |
| 61 | 0.696 | 0.236 |
| 62 | 1.62 | 0.748 |
| 63 | 2.26 | 1.89 - 2.12 |
| 64 | 0.0252 | 0.0103 - 0.0424 |
| 65 | >5 | >25 |
| 66 | 0.0850 | 0.0343 - 0.0725 |
| 67 | --- | 0.033 |
| 68 | --- | 0.096 |
| 69 | --- | 0.025 |

### Enumerated Embodiments

Embodiment 1. The compound according to formula (I): 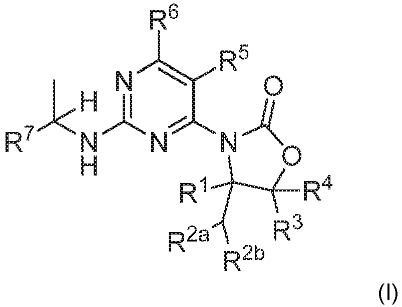 wherein:
R¹ is hydrogen, methyl or ethyl;
R^{2a} and R^{2b} are joined together forming a cyclopropyl ring;
R³ and R⁴ are each independently hydrogen, methyl or ethyl or R³ and R⁴ are joined together forming cyclopropyl, cyclobutyl or oxetanyl;
R⁵ and R⁶ are each independently hydrogen, deuterium, halo, -C(O)OCH₃, C₁₋₃ alkyl or C₁₋₃ haloalkyl;
R⁷ is 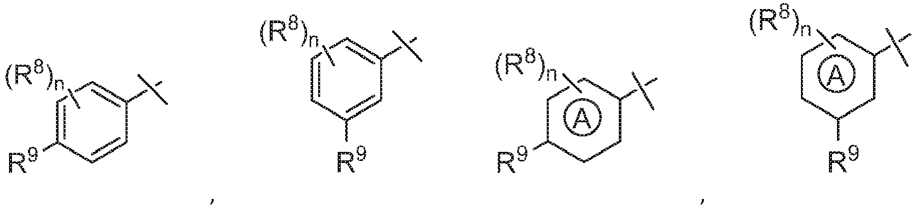 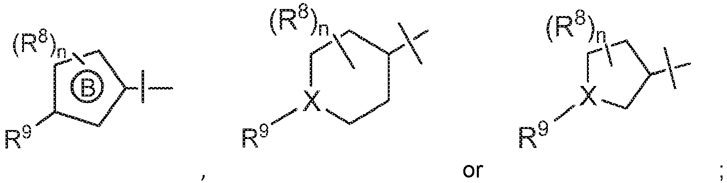
   ring A is a 6 membered heteroaryl ring having one to three nitrogen atoms;
   ring B is a 5 membered heteroaryl ring having one to four heteroatoms each independently selected from the group consisting of N, O and S;
   X is N or CH;
   each R⁸ is independently hydrogen, halo, C₁₋₃ alkyl, C₁₋₃ haloalkyl, C₁₋₃ alkoxy or C₁₋₃ haloalkoxy;
   n is 1 or 2;
   R⁹ is hydrogen, halo, C₁₋₃ haloalkyl, optionally substituted C₁₋₆ alkyl, optionally substituted C₃₋₆ cycloalkyl, optionally substituted 5 or 6 membered heterocyclic, optionally substituted aryl, optionally substituted heteroaryl, -OR^{9a}, -SO₂R^{9a}, -C(O)NHR^{9a}, CH₂R^{9b} or CHCH₃R^{9b} provided that when X is N, R⁹ is hydrogen, C₁₋₃ haloalkyl, optionally substituted C₁₋₆ alkyl, optionally substituted C₃₋₆ cycloalkyl, optionally substituted aryl, optionally substituted heteroaryl, -SO₂R^{9a} or -C(O)NHR^{9a}, wherein:
      said C₁₋₆ alkyl is optionally substituted with one to three substituents each independently selected from the group consisting of: OH and phenoxy, and
      said C₃₋₆ cycloalkyl, 5 or 6 membered heterocyclic, aryl and heteroaryl are each optionally substituted with one to three substituents each independently selected from the group consisting of: halo, hydroxyl, cyano, -NRR, C₁₋₆ alkyl, C₁₋₆ haloalkyl, C₁₋₃ alkoxy and C₁₋₃ haloalkoxy;
   R^{9a} is optionally substituted C₁₋₆ alkyl, C₁₋₆ haloalkyl, optionally substituted C₃₋₆ cycloalkyl, optionally substituted phenyl or optionally substituted heterocyclic, wherein:
      said C₁₋₆ alkyl is optionally substituted with one C₃₋₆ cycloalkyl,
      said C₃₋₆ cycloalkyl and heterocyclic are optionally substituted with one to three substituents each independently selected from the group consisting of: hydroxyl, CH₂OH, -NRR, cyano, C₁₋₃ alkyl, C₁₋₃ haloalkyl and C₁₋₃ alkoxy, and
      said phenyl is optionally substituted with one to three substituents each independently selected from the group consisting of: halo, hydroxyl, cyano, -NRR, C₁₋₆ alkyl, C₁₋₆ haloalkyl, C₁₋₃ alkoxy and C₁₋₃ haloalkoxy;
   R^{9b} is optionally substituted C₃₋₆ cycloalkyl, optionally substituted phenyl, or optionally substituted heterocyclic, wherein said C₃₋₆ cycloalkyl and heterocyclic are optionally substituted with one to four substituents each independently selected from the group consisting of: hydroxyl, CH₂OH, -NRR, -NRC(O)CH₃, 4 to 6 membered heterocyclic, cyano, halo, C₁₋₃ alkyl, C₁₋₃ haloalkyl and C₁₋₃ alkoxy, and
      said phenyl is optionally substituted with one to three substituents each independently selected from the group consisting of: halo, hydroxyl, cyano, C₁₋₆ alkyl, C₁₋₆ haloalkyl, C₁₋₃ alkoxy and C₁₋₃ haloalkoxy; and
   each R is independently selected from the group consisting of H, C₁₋₃ alkyl and C₃₋₆ cycloalkyl; or a pharmaceutically acceptable salt thereof.

Embodiment 2. The compound according to embodiment 1 wherein R³ and R⁴ are both hydrogen; or a pharmaceutically acceptable salt thereof.

Embodiment 3. The compound according to embodiments 1 or 2 wherein R¹ is hydrogen or methyl; or a pharmaceutically acceptable salt thereof.

Embodiment 4. The compound according to any one of embodiments 1-3 of formula (II): 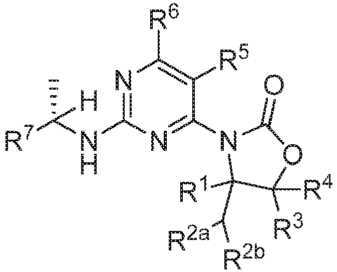 ;(II) or a pharmaceutically acceptable salt thereof.

Embodiment 5. The compound according to any one of embodiments 1-4 of formula (III): 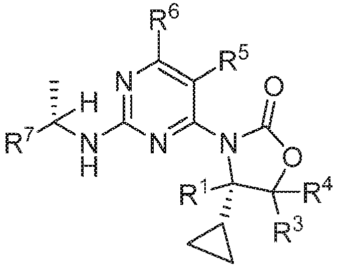 (III); or a pharmaceutically acceptable salt thereof.

Embodiment 6. The compound according to any one of embodiments 1-5 wherein R⁵ is hydrogen and R⁶ is hydrogen, fluoro, chloro or methyl; or a pharmaceutically acceptable salt thereof.

Embodiment 7. The compound according to any one of embodiments 1-6 wherein R⁶ is hydrogen and R⁵ is hydrogen or fluoro.

Embodiment 8. The compound according to any one of embodiments 1-7 wherein R⁵ and R⁶ are both hydrogen; or a pharmaceutically acceptable salt thereof.

Embodiment 9. The compound according to any one of embodiments 1-8 wherein R⁷ is 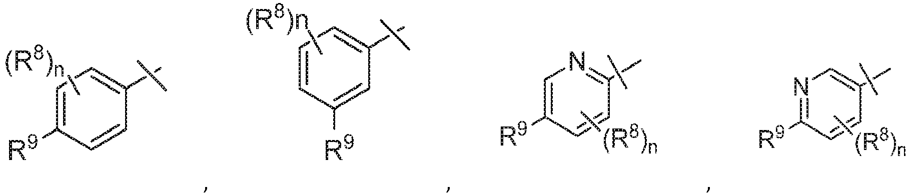 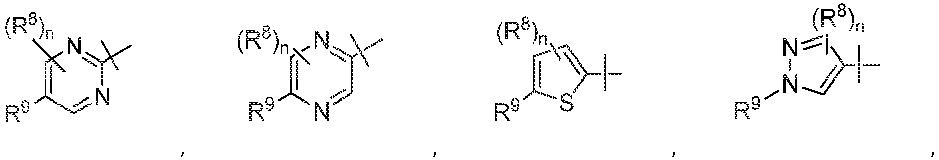 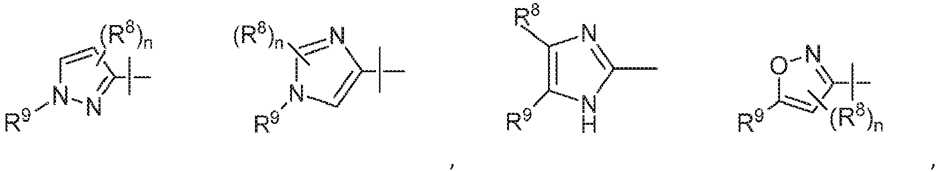 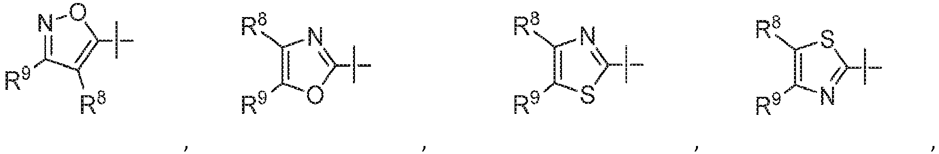 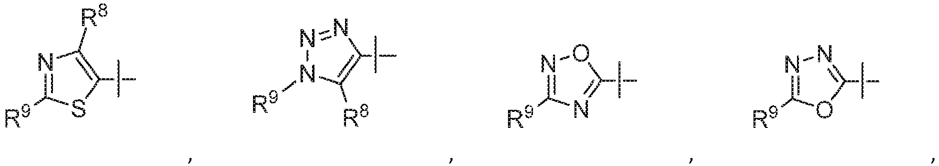 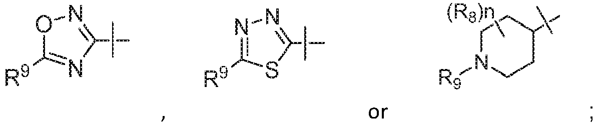 or a pharmaceutically acceptable salt thereof.

Embodiment 10. The compound according to any one of embodiments 1-9 wherein R⁷ is 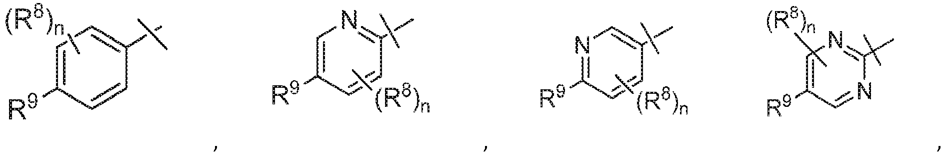 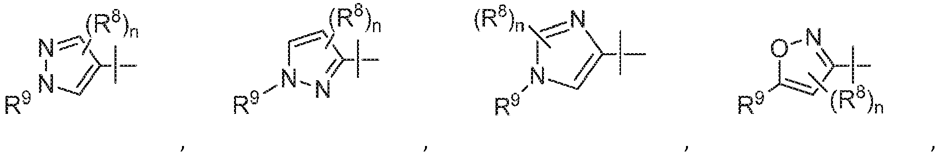 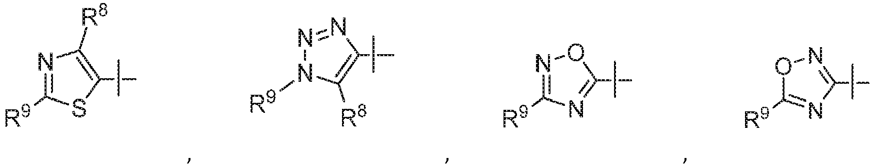 or 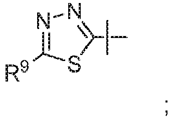 or a pharmaceutically acceptable salt thereof.

Embodiment 11. The compound according to any one of embodiments 1-10 wherein R⁹ is hydrogen, -OCF₃, halo, C₁₋₃ haloalkyl, optionally substituted 5 or 6 membered heterocyclic, optionally substituted optionally substituted aryl, optionally substituted heteroaryl, CH₂R^{9b} or CHCH₃R^{9b}, wherein said aryl and heteroaryl are optionally substituted with one to three substituents each independently selected from the group consisting of: halo, C₁₋₆ alkyl, and C₁₋₆ haloalkyl; or a pharmaceutically acceptable salt thereof.

Embodiment 12. The compound according to any one of embodiments 1-11 wherein R⁹ is hydrogen, halo, -OCF₃, or C₁₋₃ haloalkyl; or a pharmaceutically acceptable salt thereof.

Embodiment 13. The compound according to any one of embodiments 1-11 wherein R⁹ is phenyl optionally substituted with one or two substituents each independently selected from the group consisting of: fluoro, chloro, methyl, OCF₃ and C₁₋₄ haloalkyl; or a pharmaceutically acceptable salt thereof.

Embodiment 14. The compound according to any one of embodiments 1-11 wherein R⁹ is pyrazolyl or pyridinyl optionally substituted with one or two substituents each independently selected from the group consisting of C₁₋₆ alkyl and C₁₋₆ haloalkyl; or a pharmaceutically acceptable salt thereof.

Embodiment 15. The compound according to any one of embodiments 1-11 wherein R⁹ is CH₂R^{9b} or CHCH₃R^{9b} wherein R^{9b} is optionally substituted heterocyclic; or a pharmaceutically acceptable salt thereof.

Embodiment 16. The compound according to embodiment 15 wherein R^{9b} is piperidinyl, piperazinyl, morpholinyl, 3,8-diazabicyclo[3.2.1]octanyl or 3-azabicyclo[3.1.0]hexanyl each of which is optionally substituted with one to four substituents each independently selected from the group consisting of: hydroxyl, CH₂OH, -NRR, -NRC(O)CH₃, 4 to 6 membered heterocyclic, cyano, halo, C₁₋₃ alkyl, C₁₋₃ haloalkyl, and C₁₋₃ alkoxy; or a pharmaceutically acceptable salt thereof.

Embodiment 17. The compound according to embodiment 1 having the following formula 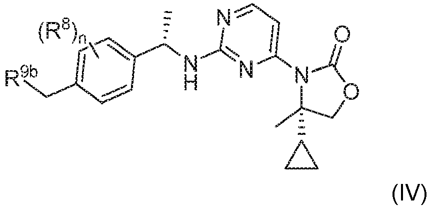 wherein R^{9b} is optionally substituted heterocyclic; or a pharmaceutically acceptable salt thereof.

Embodiment 18. The compound according to embodiment 17 wherein R^{9b} is piperidinyl, piperazinyl, morpholinyl, 3,8-diazabicyclo[3.2.1]octanyl or 3-azabicyclo[3.1.0]hexanyl each of which is optionally substituted with one to four substituents each independently selected from the group consisting of: hydroxyl, CH₂OH, -NRR, -NRC(O)CH₃, 4 to 6 membered heterocyclic, cyano, halo, C₁₋₃ alkyl, C₁₋₃ haloalkyl, and C₁₋₃ alkoxy; or a pharmaceutically acceptable salt thereof.

Embodiment 19. The compound according to embodiment 1 having the following formula 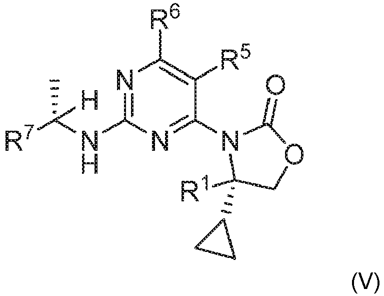 wherein:
R¹ is hydrogen or methyl;
R⁵ and R⁶ are each independently hydrogen, halo or methyl;
R⁷ is 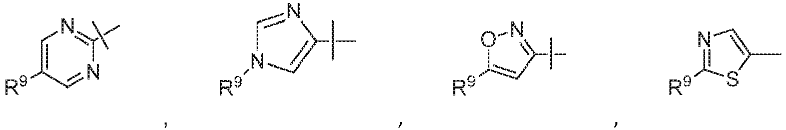 or 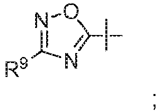 and
R⁹ is phenyl or pyridinyl optionally substituted with one or two substituents each independently selected from the group consisting of: fluoro, chloro, methyl, OCF₃, CF₂H, and CF₃; or a pharmaceutically acceptable salt thereof.

Embodiment 20. The compound according to embodiment 1 selected from the group consisting of: (S)-3-(2-(((S)-1(S)-3-(2-(((S)-1-(1-(4-chlorophenyl)-1H-imidazol-4-yl)ethyl)amino)-6-methylpyrimidin-4-yl)-4-cyclopropyloxazolidin-2-one;
(S)-3-(2-(((S)-1-(5-(4-chlorophenyl)isoxazol-3-yl)ethyl)amino)-6-methylpyrimidin-4-yl)-4-cyclopropyloxazolidin-2-one;
(S)-4-cyclopropyl-3-(2-(((S)-1-(2-(2-(trifluoromethyl)pyridin-4-yl)thiazol-5-yl)ethyl)amino)pyrimidin-4-yl)oxazolidin-2-one;
(S)-4-cyclopropyl-3-(2-(((S)-1-(2-(4-(difluoromethyl)phenyl)thiazol-5-yl)ethyl)amino)pyrimidin-4-yl)oxazolidin-2-one;
(S)-4-cyclopropyl-3-(2-(((S)-1-(4-((4-(dimethylamino)piperidin-1-yl)methyl)-3-fluorophenyl)ethyl)amino)pyrimidin-4-yl)-4-methyloxazolidin-2-one;
(S)-4-cyclopropyl-3-(2-(((S)-1-(3-fluoro-4-((4-hydroxy-4-(trifluoromethyl)piperidin-1-yl)methyl)phenyl)ethyl)amino)pyrimidin-4-yl)-4-methyloxazolidin-2-one ;
(S)-4-cyclopropyl-3-(2-(((S)-1-(4-((4-(dimethylamino)piperidin-1-yl)methyl)-2-fluorophenyl)ethyl)amino)pyrimidin-4-yl)-4-methyloxazolidin-2-one;
(S)-4-cyclopropyl-3-(2-(((S)-1-(2-fluoro-4-((4-hydroxy-4-methylpiperidin-1-yl)methyl)phenyl)ethyl)amino)pyrimidin-4-yl)-4-methyloxazolidin-2-one;
(S)-4-cyclopropyl-3-(2-(((S)-1-(2-fluoro-4-((4-hydroxy-4-(trifluoromethyl)piperidin-1-yl)methyl)phenyl)ethyl)amino)pyrimidin-4-yl)-4-methyloxazolidin-2-one;
(S)-3-(2-(((S)-1-(4-((4-(azetidin-1-yl)piperidin-1-yl)methyl)-3-fluorophenyl)ethyl)amino)pyrimidin-4-yl)-4-cyclopropyl-4-methyloxazolidin-2-one ;
(S)-4-cyclopropyl-3-(2-(((S)-1-(4-((4-(cyclopropylamino)piperidin-1-yl)methyl)-3-fluorophenyl)ethyl)amino)pyrimidin-4-yl)-4-methyloxazolidin-2-one ;
(S)-3-(2-(((S)-1-(4-((4-amino-4-(hydroxymethyl)piperidin-1-yl)methyl)-3-fluorophenyl)ethyl)amino)pyrimidin-4-yl)-4-cyclopropyl-4-methyloxazolidin-2-one;
(S)-3-(2-(((S)-1-(4-((4-amino-4-methylpiperidin-1-yl)methyl)-3-fluorophenyl)ethyl)amino)pyrimidin-4-yl)-4-cyclopropyl-4-methyloxazolidin-2-one;
(R)-3-(2-(((S)-1-(3-(4-chlorophenyl)-1,2,4-oxadiazol-5-yl)ethyl)amino)pyrimidin-4-yl)-4-cyclopropyl-4-methyloxazolidin-2-one;
(S)-4-cyclopropyl-3-(2-(((S)-1-(5-(4-fluoro-3-methylphenyl)pyrimidin-2-yl)ethyl)amino)pyrimidin-4-yl)-4-methyloxazolidin-2-one;
(R)-4-cyclopropyl-4-methyl-3-(2-(((S)-1-(4-((3,3,4-trimethylpiperazin-1-yl)methyl)phenyl)ethyl)amino)pyrimidin-4-yl)oxazolidin-2-one; and
(S)-3-(2-(((S)-1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one; or a pharmaceutically acceptable salt thereof.

Embodiment 21. A pharmaceutical composition comprising a compound according to any one of embodiments 1-20, or a pharmaceutically acceptable salt thereof, and a pharmaceutically acceptable carrier or excipient.

Embodiment 22. A method for the treatment of a disease or disorder associated with a mutant IDH protein having a neomorphic activity comprising administration of a therapeutically effective amount of a compound according to any of one of embodiments 1-20, or a pharmaceutically acceptable salt thereof, to subject in need of thereof.

Embodiment 23. A method for the treatment of a disease or disorder associated with a mutant IDH protein having a neomorphic activity comprising administration of a therapeutically effective amount of a compound according to any one of embodiments 1-20, or a pharmaceutically acceptable salt thereof, and another therapeutic agent to subject in need of thereof.

## Claims

1. The compound according to formula (I): 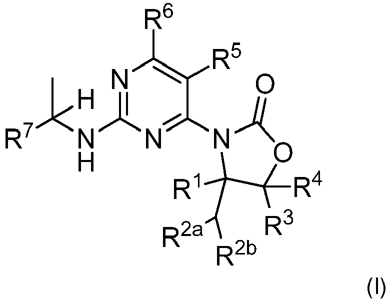 wherein:
R¹ is hydrogen, methyl or ethyl;
R^{2a} and R^{2b} are joined together forming a cyclopropyl ring;
R³ and R⁴ are each independently hydrogen, methyl or ethyl or R³ and R⁴ are joined together forming cyclopropyl, cyclobutyl or oxetanyl;
R⁵ and R⁶ are each independently hydrogen, deuterium, halo, -C(O)OCH₃, C₁₋₃ alkyl or C₁₋₃ haloalkyl;
R⁷ is 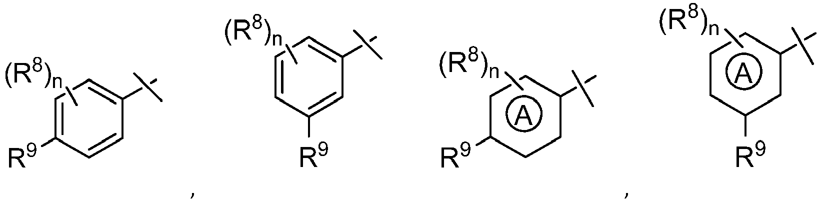 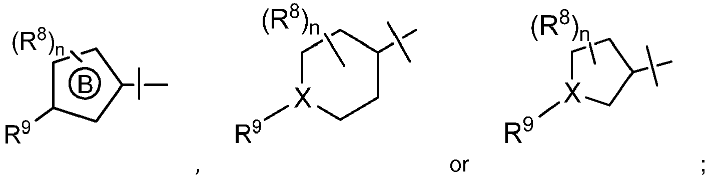
ring A is a 6 membered heteroaryl ring having one to three nitrogen atoms;
ring B is a 5 membered heteroaryl ring having one to four heteroatoms each independently selected from the group consisting of N, O and S;
X is N or CH;
each R⁸ is independently hydrogen, halo, C₁₋₃ alkyl, C₁₋₃ haloalkyl, C₁₋₃ alkoxy or C₁₋₃ haloalkoxy;
n is 1 or 2;
R⁹ is hydrogen, halo, C₁₋₃ haloalkyl, optionally substituted C₁₋₆ alkyl, optionally substituted C₃₋₆ cycloalkyl, optionally substituted 5 or 6 membered heterocyclic, optionally substituted aryl, optionally substituted heteroaryl, -OR^{9a}, -SO₂R^{9a}, -C(O)NHR^{9a}, CH₂R^{9b} or CHCH₃R^{9b} provided that when X is N, R⁹ is hydrogen, C₁₋₃ haloalkyl, optionally substituted C₁₋₆ alkyl, optionally substituted C₃₋₆ cycloalkyl, optionally substituted aryl, optionally substituted heteroaryl, -SO₂R^{9a} or -C(O)NHR^{9a}, wherein:
said C₁₋₆ alkyl is optionally substituted with one to three substituents each independently selected from the group consisting of: OH and phenoxy, and
said C₃₋₆ cycloalkyl, 5 or 6 membered heterocyclic, aryl and heteroaryl are each optionally substituted with one to three substituents each independently selected from the group consisting of: halo, hydroxyl, cyano, -NRR, C₁₋₆ alkyl, C₁₋₆ haloalkyl, C₁₋₃ alkoxy and C₁₋₃ haloalkoxy;
R^{9a} is optionally substituted C₁₋₆ alkyl, C₁₋₆ haloalkyl, optionally substituted C₃₋₆ cycloalkyl, optionally substituted phenyl or optionally substituted heterocyclic, wherein:
said C₁₋₆ alkyl is optionally substituted with one C₃₋₆ cycloalkyl,
said C₃₋₆ cycloalkyl and heterocyclic are optionally substituted with one to three substituents each independently selected from the group consisting of: hydroxyl, CH₂OH, -NRR, cyano, C₁₋₃ alkyl, C₁₋₃ haloalkyl and C₁₋₃ alkoxy, and
said phenyl is optionally substituted with one to three substituents each independently selected from the group consisting of: halo, hydroxyl, cyano, -NRR, C₁₋₆ alkyl, C₁₋₆ haloalkyl, C₁₋₃ alkoxy and C₁₋₃ haloalkoxy;
R^{9b} is optionally substituted C₃₋₆ cycloalkyl, optionally substituted phenyl, or optionally substituted heterocyclic, wherein
said C₃₋₆ cycloalkyl and heterocyclic are optionally substituted with one to four substituents each independently selected from the group consisting of: hydroxyl, CH₂OH, -NRR, -NRC(O)CH₃, 4 to 6 membered heterocyclic, cyano, halo, C₁₋₃ alkyl, C₁₋₃ haloalkyl and C₁₋₃ alkoxy, and said phenyl is optionally substituted with one to three substituents each independently selected from the group consisting of: halo, hydroxyl, cyano, C₁₋₆ alkyl, C₁₋₆ haloalkyl, C₁₋₃ alkoxy and C₁₋₃ haloalkoxy; and
each R is independently selected from the group consisting of H, C₁₋₃ alkyl and C₃₋₆ cycloalkyl; or a pharmaceutically acceptable salt thereof.

2. The compound according to claim 1 wherein R³ and R⁴ are both hydrogen; or a pharmaceutically acceptable salt thereof.

3. The compound according to claim 1 or 2 wherein R¹ is hydrogen or methyl; or a pharmaceutically acceptable salt thereof.

4. The compound according to any one of claims 1-3 of formula (II): 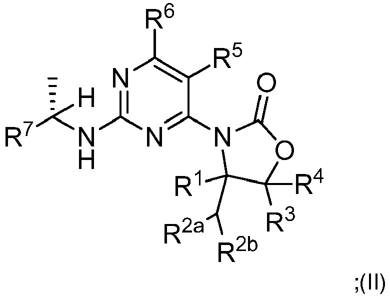 or a pharmaceutically acceptable salt thereof.

5. The compound according to any one of claims 1-4 of formula (III): 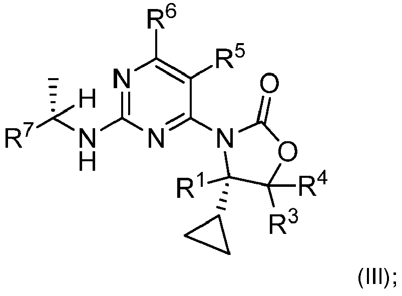 or a pharmaceutically acceptable salt thereof.

6. The compound according to any one of claims 1-5 wherein R⁵ is hydrogen or fluoro and R⁶ is hydrogen, fluoro, chloro or methyl; or a pharmaceutically acceptable salt thereof.

7. The compound according to any one of claims 1-6 wherein R⁷ is 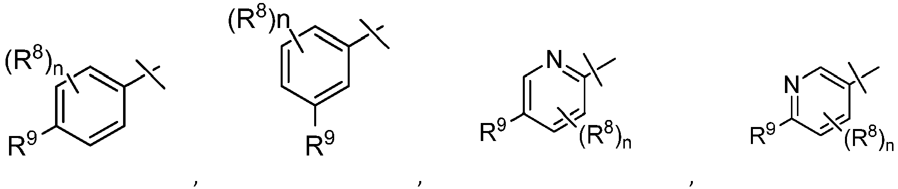 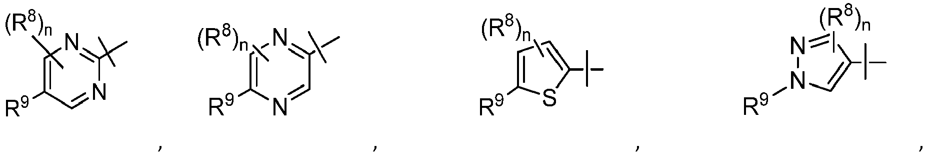 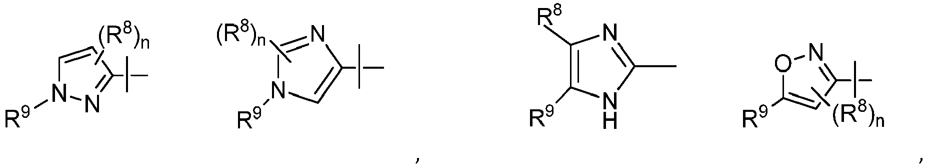 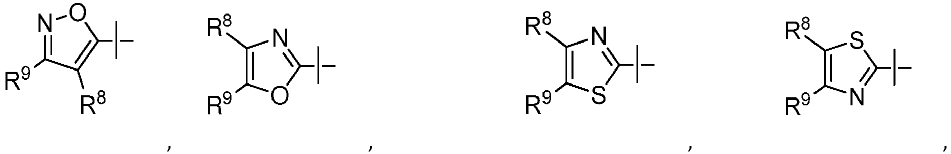 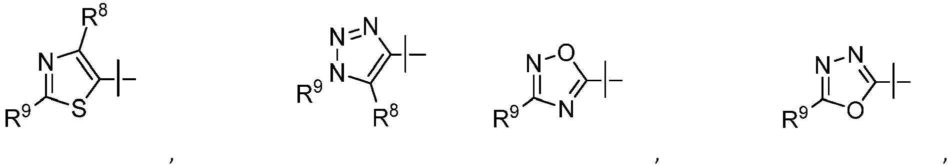 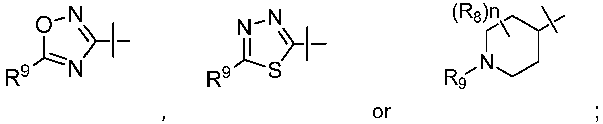 or a pharmaceutically acceptable salt thereof.

8. The compound according to any one of claims 1-7 wherein R⁹ is hydrogen, -OCF₃, halo, C₁₋₃ haloalkyl, optionally substituted 5 or 6 membered heterocyclic, optionally substituted aryl, optionally substituted heteroaryl, CH₂R^{9b} or CHCH₃R^{9b}, wherein said aryl and heteroaryl are optionally substituted with one to three substituents each independently selected from the group consisting of: halo, C₁₋₆ alkyl, and C₁₋₆ haloalkyl; or a pharmaceutically acceptable salt thereof.

9. The compound according to any one of claims 1-8 wherein R⁹ is CH₂R^{9b} or CHCH₃R^{9b} wherein R^{9b} is optionally substituted heterocyclic; or a pharmaceutically acceptable salt thereof.

10. The compound according to claim 9 wherein R^{9b} is piperidinyl, piperazinyl, morpholinyl, 3,8-diazabicyclo[3.2.1]octanyl or 3-azabicyclo[3.1.0]hexanyl each of which is optionally substituted with one to four substituents each independently selected from the group consisting of: hydroxyl, CH₂OH, -NRR, -NRC(O)CH₃, 4 to 6 membered heterocyclic, cyano, halo, C₁₋₃ alkyl, C₁₋₃ haloalkyl, and C₁₋₃ alkoxy; or a pharmaceutically acceptable salt thereof.

11. The compound according to claim 1 having the following formula 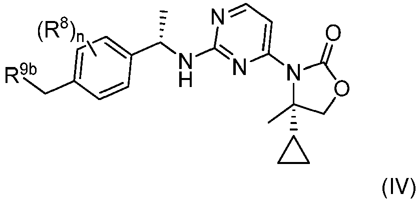 wherein R^{9b} is optionally substituted heterocyclic; or a pharmaceutically acceptable salt thereof.

12. The compound according to claim 1 having the following formula 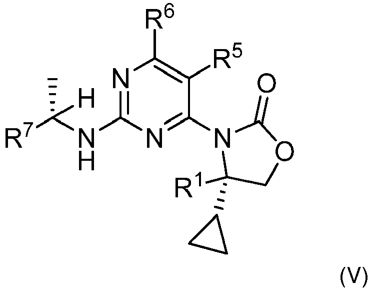 wherein:
R¹ is hydrogen or methyl;
R⁵ and R⁶ are each independently hydrogen, halo or methyl;
R⁷ is 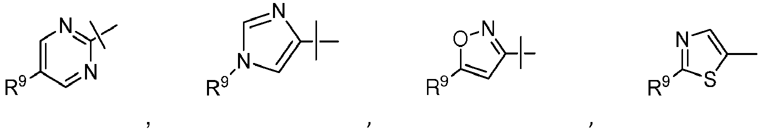 or 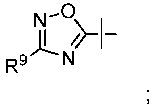 and
R⁹ is phenyl or pyridinyl optionally substituted with one or two substituents each independently selected from the group consisting of: fluoro, chloro, methyl, OCF₃, CF₂H, and CF₃; or a pharmaceutically acceptable salt thereof.

13. The compound according to claim 1 selected from the group consisting of: (S)-3-(2-(((S)-1(S)-3-(2-(((S)-1-(1-(4-chlorophenyl)-1H-imidazol-4-yl)ethyl)amino)-6-methylpyrimidin-4-yl)-4-cyclopropyloxazolidin-2-one;
(S)-3-(2-(((S)-1-(5-(4-chlorophenyl)isoxazol-3-yl)ethyl)amino)-6-methylpyrimidin-4-yl)-4-cyclopropyloxazolidin-2-one;
(S)-4-cyclopropyl-3-(2-(((S)-1-(2-(2-(trifluoromethyl)pyridin-4-yl)thiazol-5-yl)ethyl)amino)pyrimidin-4-yl)oxazolidin-2-one;
(S)-4-cyclopropyl-3-(2-(((S)-1-(2-(4-(difluoromethyl)phenyl)thiazol-5-yl)ethyl)amino)pyrimidin-4-yl)oxazolidin-2-one;
(S)-4-cyclopropyl-3-(2-(((S)-1-(4-((4-(dimethylamino)piperidin-1-yl)methyl)-3-fluorophenyl)ethyl)amino)pyrimidin-4-yl)-4-methyloxazolidin-2-one; (S)-4-cyclopropyl-3-(2-(((S)-1-(3-fluoro-4-((4-hydroxy-4-(trifluoromethyl)piperidin-1-yl)methyl)phenyl)ethyl)amino)pyrimidin-4-yl)-4-methyloxazolidin-2-one; (S)-4-cyclopropyl-3-(2-(((S)-1-(4-((4-(dimethylamino)piperidin-1-yl)methyl)-2-fluorophenyl)ethyl)amino)pyrimidin-4-yl)-4-methyloxazolidin-2-one; (S)-4-cyclopropyl-3-(2-(((S)-1-(2-fluoro-4-((4-hydroxy-4-methylpiperidin-1-yl)methyl)phenyl)ethyl)amino)pyrimidin-4-yl)-4-methyloxazolidin-2-one; (S)-4-cyclopropyl-3-(2-(((S)-1-(2-fluoro-4-((4-hydroxy-4-(trifluoromethyl)piperidin-1-yl)methyl)phenyl)ethyl)amino)pyrimidin-4-yl)-4-methyloxazolidin-2-one; (S)-3-(2-(((S)-1-(4-((4-(azetidin-1-yl)piperidin-1-yl)methyl)-3-fluorophenyl)ethyl)amino)pyrimidin-4-yl)-4-cyclopropyl-4-methyloxazolidin-2-one;
(S)-4-cyclopropyl-3-(2-(((S)-1-(4-((4-(cyclopropylamino)piperidin-1-yl)methyl)-3-fluorophenyl)ethyl)amino)pyrimidin-4-yl)-4-methyloxazolidin-2-one ;
(S)-3-(2-(((S)-1-(4-((4-amino-4-(hydroxymethyl)piperidin-1-yl)methyl)-3-fluorophenyl)ethyl)amino)pyrimidin-4-yl)-4-cyclopropyl-4-methyloxazolidin-2-one; (S)-3-(2-(((S)-1-(4-((4-amino-4-methylpiperidin-1-yl)methyl)-3-fluorophenyl)ethyl)amino)pyrimidin-4-yl)-4-cyclopropyl-4-methyloxazolidin-2-one; (R)-3-(2-(((S)-1-(3-(4-chlorophenyl)-1,2,4-oxadiazol-5-yl)ethyl)amino)pyrimidin-4-yl)-4-cyclopropyl-4-methyloxazolidin-2-one;
(S)-4-cyclopropyl-3-(2-(((S)-1-(5-(4-fluoro-3-methylphenyl)pyrimidin-2-yl)ethyl)amino)pyrimidin-4-yl)-4-methyloxazolidin-2-one;
(R)-4-cyclopropyl-4-methyl-3-(2-(((S)-1-(4-((3,3,4-trimethylpiperazin-1-yl)methyl)phenyl)ethyl)amino)pyrimidin-4-yl)oxazolidin-2-one; and (S)-3-(2-(((S)-1-(3-(4-chloro-3-(trifluoromethoxy)phenyl)-1,2,4-oxadiazol-5-yl)ethyl)amino)pyrimidin-4-yl)-4-cyclopropyl-4-methyloxazolidin-2-one; or a pharmaceutically acceptable salt thereof.

14. A pharmaceutical composition comprising a compound according to any one of claims 1-13, or a pharmaceutically acceptable salt thereof, and a pharmaceutically acceptable carrier or excipient.

15. A compound according to any of one of claims 1-13, or a pharmaceutically acceptable salt thereof for use in the treatment of a cancer selected from glioma, glioblastoma multiforme, paraganglioma, and supratentorial primordial neuroectodermal tumors (pNET), leukemia, skin cancer, prostate cancer; thyroid cancer; colon cancer; lung cancer; sarcoma, and fibrosarcoma.

16. A compound or a pharmaceutically acceptable salt thereof for use according to claim 15 in combination with another therapeutic agent.

## Patentansprüche

1. Verbindung gemäß Formel (I): 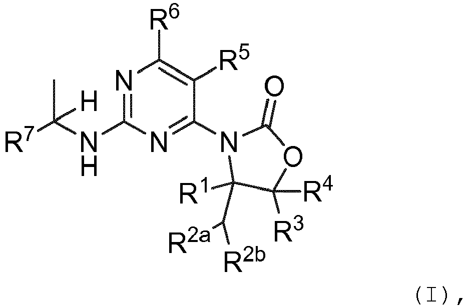 wobei:
R¹ für Wasserstoff, Methyl oder Ethyl steht,
R^{2a} und R^{2b} unter Bildung eines Cyclopropylrings miteinander verbunden sind,
R³ und R⁴ jeweils unabhängig voneinander für Wasserstoff, Methyl oder Ethyl stehen oder R³ und R⁴ unter Bildung von Cyclopropyl, Cyclobutyl oder Oxetanyl miteinander verbunden sind,
R⁵ und R⁶ jeweils unabhängig voneinander für Wasserstoff, Deuterium, Halogen, -C(O)OCH₃, C₁₋₃-Alkyl oder C₁₋₃-Halogenalkyl stehen,
R⁷ für 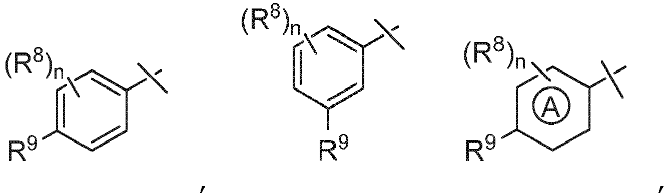 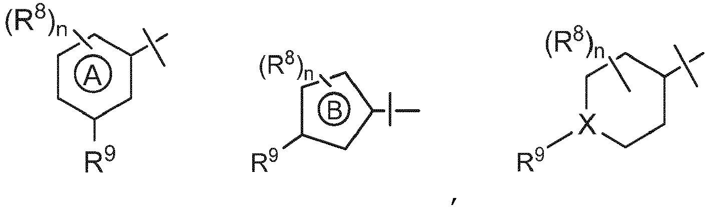 oder 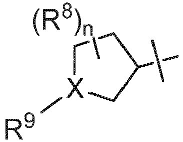 steht,
Ring A für einen 6-gliedrigen Heteroarylring mit einem bis drei Stickstoffatomen steht,
Ring B für einen 5-gliedrigen Heteroarylring mit einem bis vier jeweils unabhängig voneinander aus der aus N, O und S bestehenden Gruppe ausgewählten Heteroatomen steht,
X für N oder CH steht,
R⁸ jeweils unabhängig für Wasserstoff, Halogen, C₁₋₃-Alkyl, C₁₋₃-Halogenalkyl, C₁₋₃-Alkoxy oder C₁₋₃-Halogenalkoxy steht,
n für 1 oder 2 steht,
R⁹ für Wasserstoff, Halogen, C₁₋₃-Halogenalkyl, gegebenenfalls substituiertes C₁₋₆-Alkyl, gegebenenfalls substituiertes C₃₋₆-Cycloalkyl, gegebenenfalls substituiertes 5- oder 6-gliedriges Heterocyclyl, gegebenenfalls substituiertes Aryl, gegebenenfalls substituiertes Heteroaryl, -OR^{9a}, -SO₂R^{9a}, -C(O)NHR^{9a}, CH₂R^{9b} oder CHCH₃R^{9b} steht, mit der Maßgabe, dass, wenn X für N steht, R⁹ für Wasserstoff, C₁₋₃-Halogenalkyl, gegebenenfalls substituiertes C₁₋₆-Alkyl, gegebenenfalls substituiertes C₃₋₆-Cycloalkyl, gegebenenfalls substituiertes Aryl, gegebenenfalls substituiertes Heteroaryl, -SO₂R^{9a} oder -C(O)NHR^{9a} steht, wobei:
C₁₋₆-Alkyl gegebenenfalls durch einen bis drei jeweils unabhängig voneinander aus der aus OH und Phenoxy bestehenden Gruppe ausgewählte Substituenten substituiert ist und
C₃₋₆-Cycloalkyl, 5- oder 6-gliedriges Heterocyclyl, Aryl und Heteroaryl jeweils gegebenenfalls durch einen bis drei jeweils unabhängig voneinander aus der aus Halogen, Hydroxy, Cyano, -NRR, C₁₋₆-Alkyl, C₁₋₆-Halogenalkyl, C₁₋₃-Alkoxy und C₁₋₃-Halogenalkoxy bestehenden Gruppe ausgewählte Substituenten substituiert sind,
R^{9a} für gegebenenfalls substituiertes C₁₋₆-Alkyl, C₁₋₆-Halogenalkyl, gegebenenfalls substituiertes C₃₋₆-Cycloalkyl, gegebenenfalls substituiertes Phenyl oder gegebenenfalls substituiertes Heterocyclyl steht, wobei:
C₁₋₆-Alkyl gegebenenfalls durch ein C₃₋₆-Cycloalkyl substituiert ist,
C₃₋₆-Cycloalkyl und Heterocyclyl gegebenenfalls durch einen bis drei jeweils unabhängig voneinander aus der aus Hydroxy, CH₂OH, -NRR, Cyano, C₁₋₃-Alkyl, C₁₋₃-Halogenalkyl und C₁₋₃-Alkoxy bestehenden Gruppe ausgewählte Substituenten substituiert sind und
Phenyl gegebenenfalls durch einen bis drei jeweils unabhängig voneinander aus der aus Halogen, Hydroxy, Cyano, -NRR, C₁₋₆-Alkyl, C₁₋₆-Halogenalkyl, C₁₋₃-Alkoxy und C₁₋₃-Halogenalkoxy bestehenden Gruppe ausgewählte Substituenten substituiert ist,
R^{9b} für gegebenenfalls substituiertes C₃₋₆-Cycloalkyl, gegebenenfalls substituiertes Phenyl oder gegebenenfalls substituiertes Heterocyclyl steht, wobei
C₃₋₆-Cycloalkyl und Heterocyclyl gegebenenfalls durch einen bis vier jeweils unabhängig voneinander aus der aus Hydroxy, CH₂OH, -NRR, -NRC(O)CH₃, 4- bis 6-gliedrigem Heterocyclyl, Cyano, Halogen, C₁₋₃-Alkyl, C₁₋₃-Halogenalkyl und C₁₋₃-Alkoxy bestehenden Gruppe ausgewählte Substituenten substituiert sind und
Phenyl gegebenenfalls durch einen bis drei jeweils unabhängig voneinander aus der aus Halogen, Hydroxy, Cyano, C₁₋₆-Alkyl, C₁₋₆-Halogenalkyl, C₁₋₃-Alkoxy und C₁₋₃-Halogenalkoxy bestehenden Gruppe ausgewählte Substituenten substituiert ist, und
R jeweils unabhängig voneinander aus der aus H, C₁₋₃-Alkyl und C₃₋₆-Cycloalkyl bestehenden Gruppe ausgewählt ist, oder ein pharmazeutisch unbedenkliches Salz davon.

2. Verbindung nach Anspruch 1, wobei R³ und R⁴ beide für Wasserstoff stehen, oder ein pharmazeutisch unbedenkliches Salz davon.

3. Verbindung nach Anspruch 1 oder 2, wobei R¹ für Wasserstoff oder Methyl steht, oder ein pharmazeutisch unbedenkliches Salz davon.

4. Verbindung nach einem der Ansprüche 1-3 der Formel (II) : 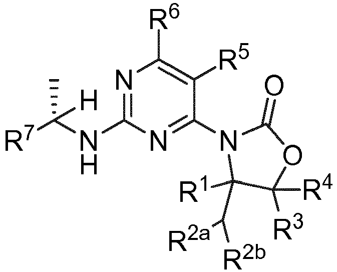 (II), oder ein pharmazeutisch unbedenkliches Salz davon.

5. Verbindung nach einem der Ansprüche 1-4 der Formel (III) : 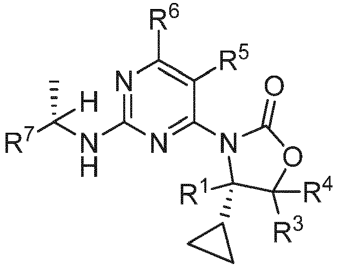 (III); oder ein pharmazeutisch unbedenkliches Salz davon.

6. Verbindung nach einem der Ansprüche 1-5, wobei R⁵ für Wasserstoff oder Fluor steht und R⁶ für Wasserstoff, Fluor, Chlor oder Methyl steht, oder ein pharmazeutisch unbedenkliches Salz davon.

7. Verbindung nach einem der Ansprüche 1-6, wobei R⁷ für 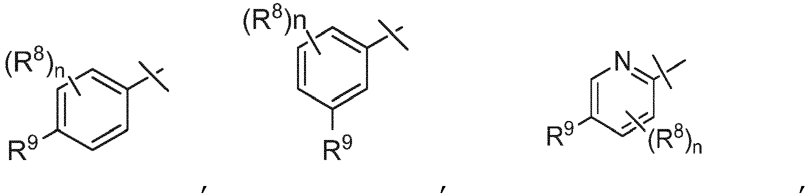 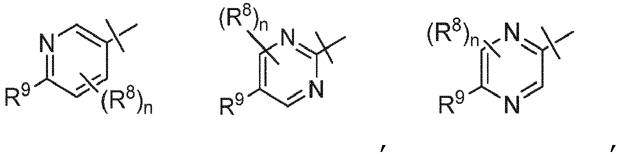 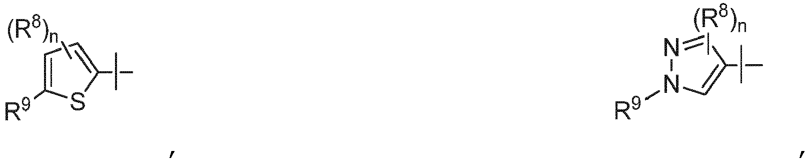 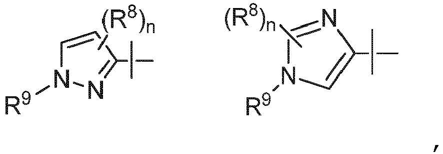 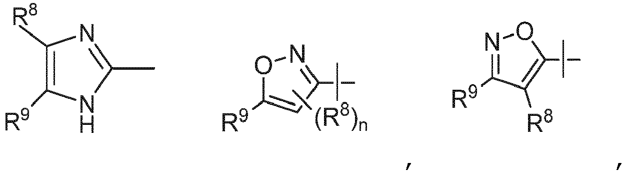 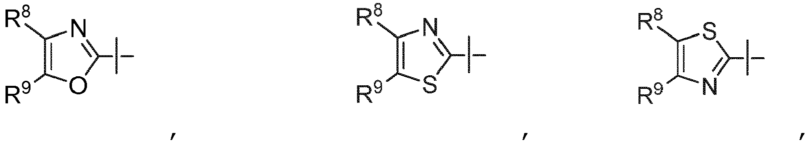 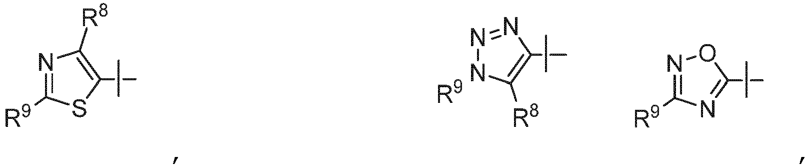 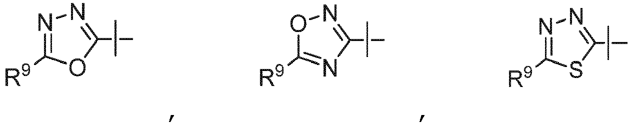 oder 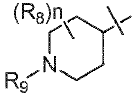 steht, oder ein pharmazeutisch unbedenkliches Salz davon.

8. Verbindung nach einem der Ansprüche 1-7, wobei R⁹ für Wasserstoff, -OCF₃, Halogen, C₁₋₃-Halogenalkyl, gegebenenfalls substituiertes 5- oder 6-gliedriges Heterocyclyl, gegebenenfalls substituiertes Aryl, gegebenenfalls substituiertes Heteroaryl, CH₂R^{9b} oder CHCH₃R^{9b} steht, wobei Aryl und Heteroaryl gegebenenfalls durch einen bis drei jeweils unabhängig voneinander aus der aus Halogen, C₁₋₆-Alkyl und C₁₋₆-Halogenalkyl bestehenden Gruppe ausgewählte Substituenten substituiert sind, oder ein pharmazeutisch unbedenkliches Salz davon.

9. Verbindung nach einem der Ansprüche 1-8, wobei R⁹ für CH₂R^{9b} oder CHCH₃R^{9b} steht, wobei R^{9b} für gegebenenfalls substituiertes Heterocyclyl steht, oder ein pharmazeutisch unbedenkliches Salz davon.

10. Verbindung nach Anspruch 9, wobei R^{9b} für Piperidinyl, Piperazinyl, Morpholinyl, 3,8-Diazabicyclo[3.2.1]octanyl oder 3-Azabicyclo[3.1.0]hexanyl steht, die jeweils gegebenenfalls durch einen bis vier unabhängig voneinander aus der aus Hydroxy, CH₂OH, -NRR, -NRC(O)CH₃, 4- bis 6-gliedrigem Heterocyclyl, Cyano, Halogen, C₁₋₃-Alkyl, C₁₋₃-Halogenalkyl und C₁₋₃-Alkoxy bestehenden Gruppe ausgewählte Substituenten substituiert sind, oder ein pharmazeutisch unbedenkliches Salz davon.

11. Verbindung nach Anspruch 1 mit der folgenden Formel 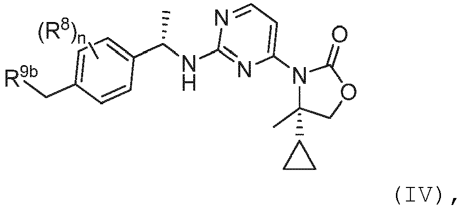 wobei R^{9b} für gegebenenfalls substituiertes Heterocyclyl steht, oder ein pharmazeutisch unbedenkliches Salz davon.

12. Verbindung nach Anspruch 1 mit der folgenden Formel 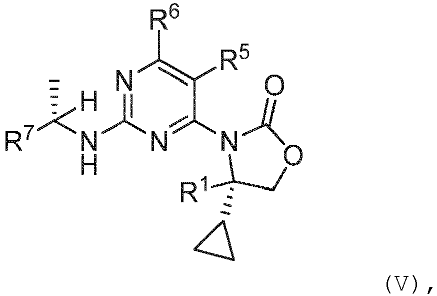 wobei:
R¹ für Wasserstoff oder Methyl steht,
R⁵ und R⁶ jeweils unabhängig voneinander für Wasserstoff, Halogen oder Methyl stehen,
R⁷ für 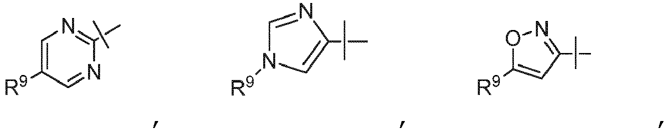 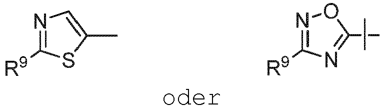 steht und
R⁹ für Phenyl oder Pyridinyl, gegebenenfalls substituiert durch einen oder zwei jeweils unabhängig voneinander aus der aus Fluor, Chlor, Methyl, OCF₃, CF₂H und CF₃ bestehenden Gruppe ausgewählte Substituenten, steht, oder ein pharmazeutisch unbedenkliches Salz davon.

13. Verbindung nach Anspruch 1, ausgewählt aus der Gruppe bestehend aus:
(S)-3-(2-(((S)-1(S)-3-(2-(((S)-1-(1-(4-Chlorphenyl)-1H-imidazol-4-yl)ethyl)amino)-6-methylpyrimidin-4-yl)-4-cyclopropyloxazolidin-2-on,
(S)-3-(2-(((S)-1-(5-(4-Chlorphenyl)isoxazol-3-yl)ethyl)amino)-6-methylpyrimidin-4-yl)-4-cyclopropyloxazolidin-2-on,
(S)-4-Cyclopropyl-3-(2-(((S)-1-(2-(2-(trifluormethyl)-pyridin-4-yl)thiazol-5-yl)ethyl)amino)pyrimidin-4-yl)oxazolidin-2-on,
(S)-4-Cyclopropyl-3-(2-(((S)-1-(2-(4-(difluormethyl)-phenyl)thiazol-5-yl)ethyl)amino)pyrimidin-4-yl)oxazolidin-2-on,
(S)-4-Cyclopropyl-3-(2-(((S)-1-(4-((4-(dimethylamino)-piperidin-1-yl)methyl)-3-fluorphenyl)ethyl)amino)-pyrimidin-4-yl)-4-methyloxazolidin-2-on,
(S)-4-Cyclopropyl-3-(2-(((S)-1-(3-fluor-4-((4-hydroxy-4-(trifluormethyl)piperidin-1-yl)methyl)phenyl)ethyl)-amino)pyrimidin-4-yl)-4-methyloxazolidin-2-on,
(S)-4-Cyclopropyl-3-(2-(((S)-1-(4-((4-(dimethylamino)-piperidin-1-yl)methyl)-2-fluorphenyl)ethyl)amino)-pyrimidin-4-yl)-4-methyloxazolidin-2-on,
(S)-4-Cyclopropyl-3-(2-(((S)-1-(2-fluor-4-((4-hydroxy-4-methylpiperidin-1-yl)methyl)phenyl)ethyl)amino)-pyrimidin-4-yl)-4-methyloxazolidin-2-on,
(S)-4-Cyclopropyl-3-(2-(((S)-1-(2-fluor-4-((4-hydroxy-4-(trifluormethyl)piperidin-1-yl)methyl)phenyl)ethyl)-amino)pyrimidin-4-yl)-4-methyloxazolidin-2-on,
(S)-3-(2-(((S)-1-(4-((4-(Azetidin-1-yl)piperidin-1-yl)methyl)-3-fluorphenyl)ethyl)amino)pyrimidin-4-yl)-4-cyclopropyl-4-methyloxazolidin-2-on,
(S)-4-Cyclopropyl-3-(2-(((S)-1-(4-((4-(cyclopropylamino)piperidin-1-yl)methyl)-3-fluorphenyl)ethyl)amino)pyrimidin-4-yl)-4-methyloxazolidin-2-on,
(S)-3-(2-(((S)-1-(4-((4-Amino-4-(hydroxymethyl)-piperidin-1-yl)methyl)-3-fluorphenyl)ethyl)amino)-pyrimidin-4-yl)-4-cyclopropyl-4-methyloxazolidin-2-on,
(S)-3-(2-(((S)-1-(4-((4-Amino-4-methylpiperidin-1-yl)methyl)-3-fluorphenyl)ethyl)amino)pyrimidin-4-yl)-4-cyclopropyl-4-methyloxazolidin-2-on,
(R)-3-(2-(((S)-1-(3-(4-Chlorphenyl)-1,2,4-oxadiazol-5-yl)ethyl)amino)pyrimidin-4-yl)-4-cyclopropyl-4-methyloxazolidin-2-on,
(S)-4-Cyclopropyl-3-(2-(((S)-1-(5-(4-fluor-3-methylphenyl)pyrimidin-2-yl)ethyl)amino)pyrimidin-4-yl)-4-methyloxazolidin-2-on,
(R)-4-Cyclopropyl-4-methyl-3-(2-(((S)-1-(4-((3,3,4-trimethylpiperazin-1-yl)methyl)phenyl)ethyl)amino)-pyrimidin-4-yl)oxazolidin-2-on und
(S)-3-(2-(((S)-1-(3-(4-Chlor-3-(trifluormethoxy)-phenyl)-1,2,4-oxadiazol-5-yl)ethyl)amino)pyrimidin-4-yl)-4-cyclopropyl-4-methyloxazolidin-2-on, oder ein pharmazeutisch unbedenkliches Salz davon.

14. Pharmazeutische Zusammensetzung, umfassend eine Verbindung nach einem der Ansprüche 1-13 oder ein pharmazeutisch unbedenkliches Salz davon und einen pharmazeutisch unbedenklichen Träger oder Exzipienten.

15. Verbindung nach einem der Ansprüche 1-13 oder ein pharmazeutisch unbedenkliches Salz davon zur Verwendung bei der Behandlung einer Krebserkrankung ausgewählt aus Gliom, Glioblastoma multiforme, Paragangliom und supratentorialen primitiven neuroektodermalen Tumoren (pNET), Leukämie, Hautkrebs, Prostatakrebs, Schilddrüsenkrebs, Kolonkrebs, Lungenkrebs, Sarkom und Fibrosarkom.

16. Verbindung oder pharmazeutisch unbedenkliches Salz davon zur Verwendung nach Anspruch 15 in Kombination mit einem anderen Therapeutikum.

## Revendications

1. Composé selon la formule (I) : 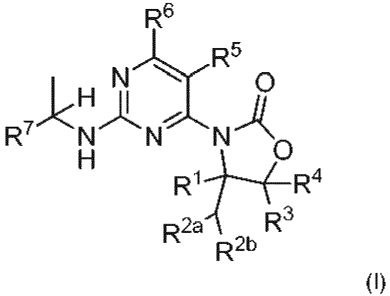 dans lequel :
R¹ est hydrogène, méthyle ou éthyle ;
R^{2a} et R^{2b} sont assemblés conjointement de manière à former un cycle cyclopropyle ;
R³ et R⁴ sont chacun indépendamment hydrogène, méthyle ou éthyle ou R³ et R⁴ sont assemblés conjointement de manière à former cyclopropyle, cyclobutyle ou oxétanyle ;
R⁵ et R⁶ sont chacun indépendamment hydrogène, deutérium, halogéno, -C(O)OCH₃, alkyle en C₁₋₃ ou halogénoalkyle en C₁₋₃ ;
R⁷ est 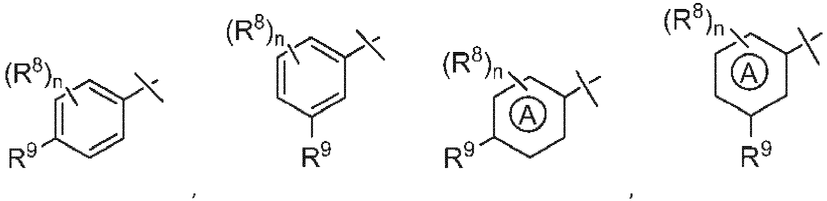 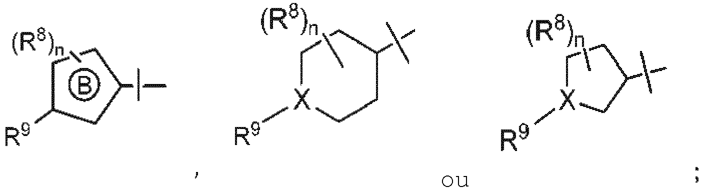
le cycle A est un cycle hétéroaryle à 6 chaînons ayant un à trois atomes d'azote ;
le cycle B est un cycle hétéroaryle à 5 chaînons ayant un à quatre hétéroatomes chacun indépendamment choisis dans le groupe constitué de N, O et S;
X est N ou CH ;
chaque R⁸ est indépendamment hydrogène, halogéno, alkyle en C₁₋₃, halogénoalkyle en C₁₋₃, alcoxy en C₁₋₃ ou halogénoalcoxy en C₁₋₃ ;
n est 1 ou 2 ;
R⁹ est hydrogène, halogéno, halogénoalkyle en C₁₋₃, alkyle en C₁₋₆ facultativement substitué, cycloalkyle en C₃₋₆ facultativement substitué, hétérocyclique de 5 ou 6 chaînons facultativement substitué, aryle facultativement substitué, hétéroaryle facultativement substitué, -OR^{9a}, -SO₂R^{9a}, -C(O)NHR9^{a}, CH₂R^{9b} ou CHCH₃R^{9b}, à condition que lorsque X est N, R⁹ soit hydrogène, halogénoalkyle en C₁₋₃, alkyle en C₁₋₆ facultativement substitué, cycloalkyle en C₃₋₆ facultativement substitué, cycloalkyle en C₃₋₆ facultativement substitué, aryle facultativement substitué, hétéroaryle facultativement substitué, -SO₂R^{9a} ou -C(O)NHR9^{a}, dans lequel :
ledit alkyle en C₁₋₆ est facultativement substitué par un à trois substituants chacun indépendamment choisis dans le groupe constitué de : OH et phénoxy, et lesdits cycloalkyle en C₃₋₆, hétérocyclique de 5 ou 6 chaînons, aryle et hétéroaryle sont chacun facultativement substitués par un à trois substituants chacun indépendamment choisis dans le groupe constitué de : halogéno, hydroxyle, cyano, -NRR, alkyle en C₁₋₆, halogénoalkyle en C₁₋₆, alcoxy en C₁₋₃ et halogénoalcoxy en C₁₋₃ ;
R^{9a} est alkyle en C₁₋₆ facultativement substitué, halogénoalkyle en C₁₋₆, cycloalkyle en C₃₋₆ facultativement substitué, phényle facultativement substitué ou hétérocyclique facultativement substitué, où :
ledit alkyle en C₁₋₆ est facultativement substitué par un cycloalkyle en C₃₋₆,
lesdits cycloalkyle en C₃₋₆ et hétérocyclique sont facultativement substitués par un à trois substituants chacun indépendamment choisis dans le groupe constitué de : hydroxyle, CH₂OH, -NRR, cyano, alkyle en C₁₋₃, halogénoalkyle en C₁₋₃ et alcoxy en C₁₋₃, et
ledit phényle est facultativement substitué par un à trois substituants chacun indépendamment choisis dans le groupe constitué de : halogéno, hydroxyle, cyano, -NRR, alkyle en C₁₋₆, halogénoalkyle en C₁₋₆, alcoxy en C₁₋₃ et halogénoalcoxy en C₁₋₃ ;
R^{9b} est cycloalkyle en C₃₋₆ facultativement substitué, phényle facultativement substitué, ou hétérocyclique facultativement substitué, où
lesdits cycloalkyle en C₃₋₆ et hétérocyclique sont facultativement substitués par un à quatre substituants chacun indépendamment choisis dans le groupe constitué de : hydroxyle, CH₂OH, -NRR, -NRC(O)CH₃, hétérocyclique de 4 à 6 chaînons, cyano, halogéno, alkyle en C₁₋₃, halogénoalkyle en C₁₋₃ et alcoxy en C₁₋₃, et ledit phényle est facultativement substitué par un à trois substituants chacun indépendamment choisis dans le groupe constitué de : halogéno, hydroxyle, cyano, alkyle en C₁₋₆, halogénoalkyle en C₁₋₆, alcoxy en C₁₋₃ et halogénoalcoxy en C₁₋₃ ; et
chaque R est indépendamment choisi dans le groupe constitué de H, alkyle en C₁₋₃ et cycloalkyle en C₃₋₆ ; ou sel pharmaceutiquement acceptable de celui-ci.

2. Composé selon la revendication 1 dans lequel R³ et R⁴ sont tous deux hydrogène ; ou sel pharmaceutiquement acceptable de celui-ci.

3. Composé selon la revendication 1 ou 2 dans lequel R^{?} est hydrogène ou méthyle ; ou sel pharmaceutiquement acceptable de celui-ci.

4. Composé selon l'une quelconque des revendications 1 à 3 de formule (II) : 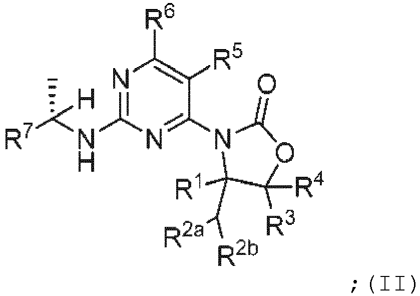 ou sel pharmaceutiquement acceptable de celui-ci.

5. Composé selon l'une quelconque des revendications 1 à 4 de formule (III) : 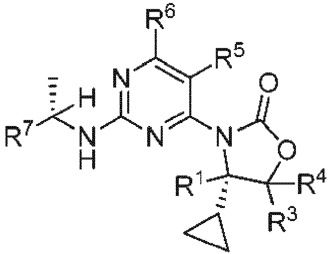 (III) ; ou sel pharmaceutiquement acceptable de celui-ci.

6. Composé selon l'une quelconque des revendications 1 à 5 dans lequel R⁵ est hydrogène ou fluoro et R⁶ est hydrogène, fluoro, chloro ou méthyle ; ou sel pharmaceutiquement acceptable de celui-ci.

7. Composé selon l'une quelconque des revendications 1 à 6 dans lequel R⁷ est 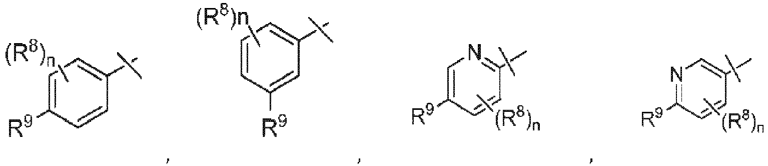 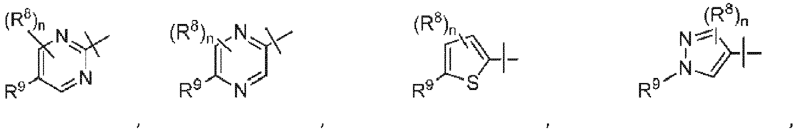 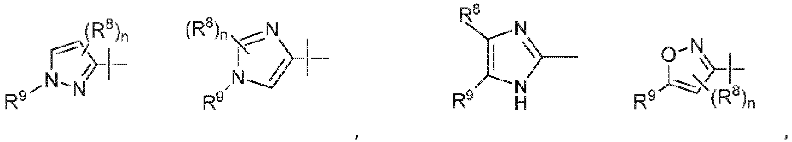 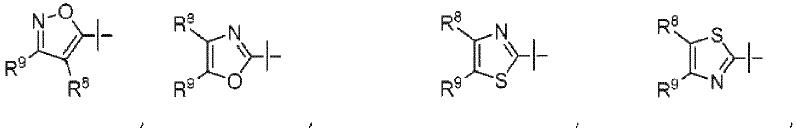 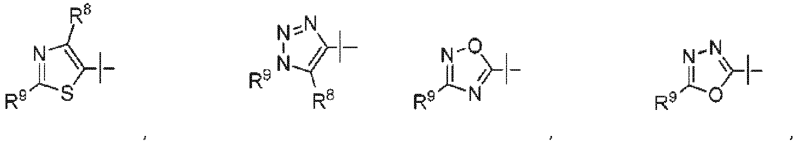 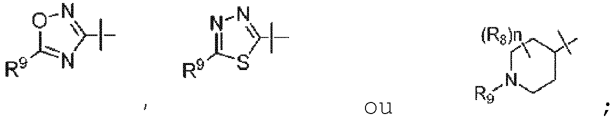 ou sel pharmaceutiquement acceptable de celui-ci.

8. Composé selon l'une quelconque des revendications 1 à 7 dans lequel R⁹ est hydrogène, -OCF₃, halogéno, halogénoalkyle en C₁₋₃, hétérocyclique de 5 ou 6 chaînons facultativement substitué, aryle facultativement substitué, hétéroaryle facultativement substitué, CH₂R^{9b} ou CHCH₃R^{9b}, dans lequel lesdits aryle et hétéroaryle sont facultativement substitués par un à trois substituants chacun indépendamment choisis dans le groupe constitué de : halogéno, alkyle en C₁₋₆, et halogénoalkyle en C₁₋₆ ; ou sel pharmaceutiquement acceptable de celui-ci.

9. Composé selon l'une quelconque des revendications 1 à 8 dans lequel R⁹ est CH₂R^{9b} ou CHCH₃R^{9b} où R^{9b} est hétérocyclique facultativement substitué ; ou sel pharmaceutiquement acceptable de celui-ci.

10. Composé selon la revendication 9 dans lequel R^{9b} est pipéridinyle, pipérazinyle, morpholinyle, 3,8-diazabicyclo[3.2.1]octanyle ou 3-azabicyclo[3.1.0]hexanyle, dont chacun est facultativement substitué par un à quatre substituants chacun indépendamment choisis dans le groupe constitué de : hydroxyle, CH₂OH, -NRR, -NRC(O)CH₃, hétérocyclique de 4 à 6 chaînons, cyano, halogéno, alkyle en C₁₋₃, halogénoalkyle en C₁₋₃, et alcoxy en C₁₋₃ ; ou un sel pharmaceutiquement acceptable de celui-ci.

11. Composé selon la revendication 1 ayant la formule suivante 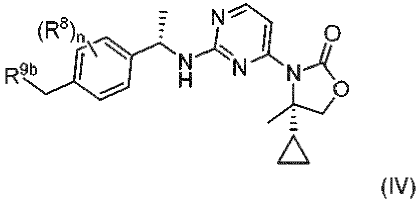 dans lequel R^{9b} est hétérocyclique facultativement substitué ; ou sel pharmaceutiquement acceptable de celui-ci.

12. Composé selon la revendication 1 ayant la formule suivante 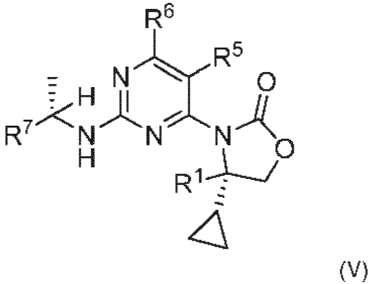 dans lequel :
R¹ est hydrogène ou méthyle ;
R⁵ et R⁶ sont chacun indépendamment hydrogène, halogéno ou méthyle ;
R⁷ est 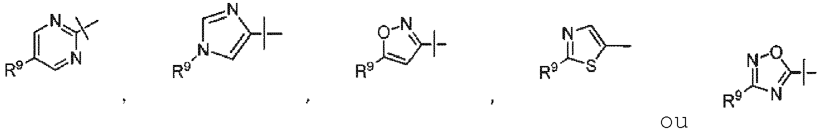 ; et
R⁹ est phényle ou pyridinyle facultativement substitué par un ou deux substituants chacun indépendamment choisis dans le groupe constitué de: fluoro, chloro, méthyle, OCF₃, CF₂H et CF₃ ; ou sel pharmaceutiquement acceptable de celui-ci.

13. Composé selon la revendication 1 choisi dans le groupe constitué de :
(S)-3-(2-(((S)-1(S)-3-(2-(((S)-1-(1-(4-chlorophényl)-1H-imidazol-4-yl)éthyl)amino)-6-méthylpyrimidin-4-yl)-4-cyclopropyloxazolidin-2-one;
(S)-3-(2-(((S)-1-(5-(4-chlorophényl)isoxazol-3-yl)éthyl)amino)-6-méthylpyrimidin-4-yl)-4-cyclopropyloxazolidin-2-one;
(S)-4-cyclopropyl-3-(2-(((S)-1-(2-(2-(trifluorométhyl)pyridin-4-yl)thiazol-5-yl)éthyl)amino)pyrimidin-4-yl)oxazolidin-2-one;
(S)-4-cyclopropyl-3-(2-(((S)-1-(2-(4-(difluorométhyl)phényl)thiazol-5-yl)éthyl)amino)pyrimidin-4-yl)oxazolidin-2-one;
(S)-4-cyclopropyl-3-(2-(((S)-1-(4-((4-(diméthylamino)pipéridin-1-yl)méthyl)-3-fluorophényl)éthyl)amino)pyrimidin-4-yl)-4-méthyloxazolidin-2-one;
(S)-4-cyclopropyl-3-(2-(((S)-1-(3-fluoro-4-((4-hydroxy-4-(trifluorométhyl)pipéridin-1-yl)méthyl)phényl)éthyl)amino)pyrimidin-4-yl)-4-méthyloxazolidin-2-one;
(S)-4-cyclopropyl-3-(2-(((S)-1-(4-((4-(diméthylamino)pipéridin-1-yl)méthyl)-2-fluorophényl)éthyl)amino)pyrimidin-4-yl)-4-méthyloxazolidin-2-one;
(S)-4-cyclopropyl-3-(2-(((S)-1-(2-fluoro-4-((4-hydroxy-4-méthylpipéridin-1-yl)méthyl)phényl)éthyl)amino)pyrimidin-4-yl)-4-méthyloxazolidin-2-one;
(S)-4-cyclopropyl-3-(2-(((S)-1-(2-fluoro-4-((4-hydroxy-4-(trifluorométhyl)pipéridin-1-yl)méthyl)phényl)éthyl)amino)pyrimidin-4-yl)-4-méthyloxazolidin-2-one;
(S)-3-(2-(((S)-1-(4-((4-(azetidin-1-yl)pipéridin-1-yl)méthyl)-3-fluorophényl)éthyl)amino)pyrimidin-4-yl)-4-cyclopropyl-4-méthyloxazolidin-2-one;
(S)-4-cyclopropyl-3-(2-(((S)-1-(4-((4-(cyclopropylamino)pipéridin-1-yl)méthyl)-3-fluorophényl)éthyl)amino)pyrimidin-4-yl)-4-méthyloxazolidin-2-one;
(S)-3-(2-(((S)-1-(4-((4-amino-4-(hydroxyméthyl)pipéridin-1-yl)méthyl)-3-fluorophényl)éthyl)amino)pyrimidin-4-yl)-4-cyclopropyl-4-méthyloxazolidin-2-one;
(S)-3-(2-(((S)-1-(4-((4-amino-4-méthylpipéridin-1-yl)méthyl)-3-fluorophényl)éthyl)amino)pyrimidin-4-yl)-4-cyclopropyl-4-méthyloxazolidin-2-one;
(R)-3-(2-(((S)-1-(3-(4-chlorophényl)-1,2,4-oxadiazol-5-yl)éthyl)amino)pyrimidin-4-yl)-4-cyclopropyl-4-méthyloxazolidin-2-one;
(S)-4-cyclopropyl-3-(2-(((S)-1-(5-(4-fluoro-3-méthylphényl)pyrimidin-2-yl)éthyl)amino)pyrimidin-4-yl)-4-méthyloxazolidin-2-one;
(R)-4-cyclopropyl-4-méthyl-3-(2-(((S)-1-(4-((3,3,4-triméthylpipérazin-1-yl)méthyl)phényl)éthyl)amino)pyrimidin-4-yl)oxazolidin-2-one ; et
(S)-3-(2-(((S)-1-(3-(4-chloro-3-(trifluorométhoxy)phényl)-1,2,4-oxadiazol-5-yl)éthyl)amino)pyrimidin-4-yl)-4-cyclopropyl-4-méthyloxazolidin-2-one ; ou sel pharmaceutiquement acceptable de celui-ci.

14. Composition pharmaceutique comprenant un composé selon l'une quelconque des revendications 1 à 13, ou un sel pharmaceutiquement acceptable de celui-ci, et un véhicule ou excipient pharmaceutiquement acceptable.

15. Composé selon l'une quelconque des revendications 1 à 13, ou sel pharmaceutiquement acceptable de celui-ci pour utilisation dans le traitement d'un cancer choisi parmi un gliome, un glioblastome multiforme, un paragangliome et des tumeurs neuro-ectodermiques primordiales sustentorielles (pNET), une leucémie, un cancer de la peau, un cancer de la prostate ; un cancer de la thyroïde ; un cancer du côlon ; un cancer du poumon ; un sarcome et un fibrosarcome.

16. Composé ou sel pharmaceutiquement acceptable de celui-ci pour utilisation selon la revendication 15 en combinaison avec un autre agent thérapeutique.
